(19) Europäisches Patentamt / European Patent Office / Office européen des brevets

(11) **EP 4 092 132 A1**

(12) **EUROPEAN PATENT APPLICATION**
published in accordance with Art. 153(4) EPC

(43) Date of publication:
**23.11.2022 Bulletin 2022/47**

(21) Application number: **20913540.9**

(22) Date of filing: **18.12.2020**

(51) International Patent Classification (IPC):
*C12Q 1/6806* (2018.01)   *C12M 1/00* (2006.01)
*C12M 1/34* (2006.01)   *C12N 15/10* (2006.01)
*C12N 15/12* (2006.01)   *C12Q 1/6851* (2018.01)
*C12Q 1/686* (2018.01)   *C12Q 1/6869* (2018.01)

(52) Cooperative Patent Classification (CPC):
**C07K 14/435; C12M 1/00; C12M 1/34; C12N 15/10;
C12Q 1/6806; C12Q 1/6851; C12Q 1/686;
C12Q 1/6869**

(86) International application number:
**PCT/JP2020/047471**

(87) International publication number:
**WO 2021/145140 (22.07.2021 Gazette 2021/29)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **16.01.2020   JP 2020005315
17.08.2020   JP 2020137596**

(71) Applicant: **Ricoh Company, Ltd.
Tokyo 143-8555 (JP)**

(72) Inventors:
• **OSAKI Yusuke**
  **Tokyo 143-8555 (JP)**

• **NAKAZAWA Satoshi**
  **Tokyo 143-8555 (JP)**
• **YONEKAWA Yuuki**
  **Tokyo 143-8555 (JP)**
• **UNNO Hirotaka**
  **Tokyo 143-8555 (JP)**
• **HASHIMOTO Michie**
  **Tokyo 143-8555 (JP)**
• **KAJIKAWA Masunori**
  **Tokyo 143-8555 (JP)**

(74) Representative: **Marks & Clerk LLP
15 Fetter Lane
London EC4A 1BW (GB)**

Remarks:
The complete document including Reference
Table(s) and the Sequence Listing(s) can be
downloaded from the EPO website

(54) **SAMPLE FOR EVALUATING PERFORMANCE OF GENETIC TESTING APPARATUS, METHOD FOR PREPARING SAID SAMPLE, AND DEVICE FOR EVALUATING PERFORMANCE, METHOD FOR EVALUATING PERFORMANCE, PROGRAM FOR EVALUATING PERFORMANCE, AND APPARATUS FOR EVALUATING PERFORMANCE OF GENETIC TESTING APPARATUS**

(57)   A sample for evaluating performance of a genetic testing apparatus includes: a nucleic acid A; and a nucleic acid B, in which the nucleic acid A and the nucleic acid B have mutually different sequences, the nucleic acid A with a specific number of molecules is contained, more nucleic acid B than the number of molecules of the nucleic acid A is contained, and a ratio A/B of the number of molecules of the nucleic acid A with respect to the number of molecules of the nucleic acid B is specified.

EP 4 092 132 A1

**Description**

TECHNICAL FIELD

The present invention relates to a sample for evaluating performance of a genetic testing apparatus, a method for preparing the sample, and a device for evaluating performance, a method for evaluating performance, a program for evaluating performance, and an apparatus for evaluating performance of a genetic testing apparatus.

Priority is claimed on Japanese Patent Application No. 2020-005315 filed January 16, 2020 and Japanese Patent Application No. 2020-137596 filed August 17, 2020, the contents of which are incorporated herein by reference.

BACKGROUND ART

In recent years, development of techniques for early detection of cancers and organ abnormalities and testing of fetal conditions and maternal health targeted at blood circulation DNAs (circulating cell-free DNA: cfDNA) has been advanced. Testing using a next generation sequencer (NGS), in particular, can enable not only simultaneous testing of multiple regions but also detection of a target with higher sensitivity as compared with other methods and thus has attracted attention. Also, testing using the NGS is used not only at medical sites but also for environmental DNA analysis for inspecting biota in water or soil and inspection of microbiota in gut and oral cavities.

However, in testing using the NGS in the related art, it is possible to detect mutation of an ultra-low concentration of an allele ratio of about 0.05% in an example of cancer, while there is no nucleic acid sample as a standard to accurately evaluate the performance thereof. Specifically, there is a problem in that in a case in which a standard sample of an allele ratio of about 0.05% is produced by a dilution method, for example, alleles with a lower content ratio (hereinafter, referred to as "minor alleles" in some cases), in particular, are always affected by Poisson distribution at the time of dispensing, and variations occur in the final concentration. Although it is possible to avoid this problem by producing a large quantity of standard samples, the volume of nucleic acid samples used in actual NGS sequence detection testing is about 50 μL at most, Poisson distribution has influences thereon even if standard samples with ultra-low concentration can be accurately prepared at the time of dispensing for the NGS sequence detection testing, and such influences cannot be avoided. Although a method of preparing more minor alleles than the number of copies that are not affected by the Poisson distribution is also conceivable as another method, the number of copies for the alleles of a higher content ratio (hereinafter, also referred to as "major alleles" in some cases) becomes significantly large in this case. A nucleic acid sample with ultra-high concentration obtained by this method has high viscosity, the concentration of magnesium ions in a reaction solution decreases, and various reactions that are important in testing using NGS including a PCR reaction are inhibited.

Although samples prepared by diluting commercially available human genomic DNAs by operators in each facility through manual techniques have been used as standard samples until now, it is not possible to secure preparation accuracy as positive standard samples (positive control) of a low allele ratio as described above. Therefore, even if performance of testing is evaluated using the standard sample, it is not possible to determine whether the variations in the standard sample are the reason or whether there are any deficiencies in performance of testing. For these reasons, a standard sample for accurately evaluating performance of testing has been desired. Also, in order to provide more accurate and precise information in a method using a liquid biopsy (liquid biopsy), a standard sample with guaranteed accuracy has been desired.

Patent Document 1 discloses a method for managing the quality of genetic testing with higher accuracy in a case in which a plurality of genes are analysis targets, as in panel testing or the like. Specifically, a method is disclosed for evaluating the quality of genetic testing for testing genes in samples collected from a subject for a plurality of types of genetic mutation including a first type of genetic mutation and a second type of genetic mutation that is different from the first type in which a quality management sample containing a first standard gene with the first type of genetic mutation and a second standard gene with the second type of genetic mutation is prepared, sequence information of the genes contained in the quality management sample is acquired, and an indicator for evaluating quality of the genetic testing is output based on the acquired sequence information.

However, for the quality management sample described in Patent Document 1, the content ratio of the two types of genes with different mutation is not specified, the preparation method therefor is not specifically examined, preparation accuracy is thus not secured, and there is room for improvement.

DISCLOSURE OF INVENTION

Problems to be Solved by the Invention

The present invention provides a sample for evaluating performance of a genetic testing apparatus capable of reducing the total amount of nucleic acid in a reaction space and accurately evaluating the performance of the genetic testing apparatus.

Means for Solving the Problems

A sample for evaluating performance of a genetic testing apparatus includes: a nucleic acid A; and a nucleic acid B, in which the nucleic acid A and the nucleic acid B have mutually different sequences, the nucleic acid A with a specific number of molecules is contained, more nucleic acid B than the number of molecules of the nucleic acid A is contained, and a ratio A/B of the number of molecules of the nucleic acid A with respect to the number of molecules of the nucleic acid B is specified.

Effects of the Invention

According to the sample for evaluating performance of a genetic testing apparatus of the present invention, it is possible to provide a sample for evaluating performance of a genetic testing apparatus capable of reducing the total amount of nucleic acid in a reaction space and accurately evaluating performance of the genetic testing apparatus.

BRIEF DESCRIPTION OF THE DRAWINGS

Fig. 1 is a graph showing a relationship between the number of molecules with variations based on Poisson distribution and a variation coefficient CV.
Fig. 2 is a graph showing a relationship between a frequency of cells for which DNA has already been copied and fluorescence intensity.
Fig. 3 is a schematic view showing an example of an ejection head based on a solenoid valve method.
Fig. 4 is a schematic view showing an example of an ejection head based on a piezoelectric method.
Fig. 5 is a schematic view of a modification example of the ejection head based on the piezoelectric method in Fig. 4.
Fig. 6(a) is a schematic view showing an example of a voltage applied to a piezoelectric element. Fig. 6(b) is a schematic view showing another example of the voltage applied to the piezoelectric element.
Figs. 7(a) to 7(c) are schematic views showing an example of liquid droplet states.
Fig. 8 is an outline view showing an example of a dispensing device for causing liquid droplets to sequentially land inside a well.
Fig. 9 is a schematic view showing an example of a liquid droplet-forming device.
Fig. 10 is a diagram showing, as an example, a hardware block of a control means in the liquid droplet-forming device in Fig. 9.
Fig. 11 is a diagram showing, as an example, functional blocks of the control means in the liquid droplet-forming device in Fig. 9.
Fig. 12 is a flowchart showing an example of operations of the liquid droplet-forming device.
Fig. 13 is a schematic view showing a modification example of the liquid droplet-forming device.
Fig. 14 is a schematic view showing another modification example of the liquid droplet-forming device.
Figs. 15(a) and 15(b) are diagrams showing, as an example, a case in which there are two fluorescent particles in a flying liquid droplet.
Fig. 16 is a diagram showing, as an example, a relationship between a luminance value Li in a case in which no overlapping between particles occur and an actually measured luminance value Le.
Fig. 17 is a schematic view showing another modification example of the liquid droplet-forming device.
Fig. 18 is a schematic view showing another example of the liquid droplet-forming device.
Fig. 19 is a schematic view showing an example of a method for counting cells that have passed through a microchannel.
Fig. 20 is a schematic view showing an example of a method for acquiring an image in the vicinity of a nozzle unit of an ejection head.
Fig. 21 is a graph representing a relationship between a probability P (> 2) and an average number of cells.
Figs. 22(a) and 22(b) are diagrams (a perspective view and a sectional view) showing an example of a device for evaluating performance of a genetic testing apparatus according to the present invention.
Fig. 23 is a block diagram showing an example of a hardware configuration of an apparatus for evaluating performance of the genetic testing apparatus according to the present invention.
Fig. 24 is a diagram showing an example of a functional configuration of the apparatus for evaluating performance of the genetic testing apparatus according to the present invention.

Fig. 25 is a flowchart showing an example of processing of a program for evaluating performance of the genetic testing apparatus according to the present invention.

Figs. 26(a) to 26(e) are diagrams showing PM scores calculated based on the read counts obtained from three runs of samples (IJ group) with ultra-low allele frequencies and one run of manually diluted samples (manual group) in Example 2.

Fig. 27 is a table showing PM scores calculated based on the read counts obtained from the three runs of samples (IJ group) with ultra-low allele frequencies and one run of manually diluted samples (manual group) in Example 2.

Fig. 28 is a table showing PM scores (reproducibility evaluation in the IJ group) calculated based on read counts obtained from three runs of samples (IJ group) with ultra-low allele frequencies and one run of manually diluted samples (manual group) in Example 2.

Fig. 29 is a graph showing a relationship between PM scores calculated based on read counts obtained from one run of samples (IJ group) with ultra-low allele frequencies and the number of added copies in Example 3.

Fig. 30 is a diagram showing PM scores calculated based on the read counts obtained from four runs of an operator A/equipment 1, two runs of an operator B/equipment 1, and three runs of an operator C/equipment 2 in Example 4.

Fig. 31 is a graph showing a relationship between PM scores calculated based on the read counts obtained from one run of samples with ultra-low allele frequencies and the allele frequencies in Example 5.

Fig. 32 is a graph showing a relationship between PM scores calculated based on the read counts obtained from one run of samples with ultra-low allele frequencies and the allele frequencies in Example 5.

Fig. 33 is a graph showing a regression line (left diagram) in a case in which the horizontal axis represents the number of copies and a regression line (right diagram) in a case in which the horizontal axis represents the logarithm of the number of copies in Example 6.

EMBODIMENTS FOR CARRYING OUT THE INVENTION

Hereinafter, a sample for evaluating performance of a genetic testing apparatus and a method for preparing the same, and a device for evaluating performance, a method for evaluating performance, a program for evaluating performance, and an apparatus for evaluating performance of a genetic testing apparatus according to an embodiment of the present invention (hereinafter, simply referred to as a "performance evaluation sample according to the present embodiment", a "preparation method according to the present embodiment", a "device according to the present embodiment", a "performance evaluation method according to the present embodiment", a "performance evaluation program according to the present embodiment", and a "performance evaluation apparatus according to the present embodiment" in some cases) will be described with reference to specific embodiments and drawings as needed. These embodiments and drawings are just examples for facilitating understanding of the present invention and are not intended to limit the present invention. In other words, the shapes, the dimensions, the dispositions, and the like of members described below can be changed or improved without departing from the gist of the present invention, and equivalents thereof are included in the present invention.

Also, similar reference signs will be applied to similar components in all the drawings, and repeated description will be appropriately omitted.

In the specification, all the technical terms and scientific terms used in the specification have the same meanings as those typically understood by those skilled in the art unless otherwise defined in the specification. The entire contents of all patents, applications, other publications, and information referred to in the specification will be incorporated in the specification for reference. Also, in a case in which some conflicts occur between the publications referred to in the specification and the description in the specification, the description in the specification is to be referred to with priority.

<Sample for evaluating performance of genetic testing apparatus>

A sample for evaluating performance according to the present embodiment includes a nucleic acid A and a nucleic acid B, and the nucleic acid A and the nucleic acid B have mutually different sequences. Also, the sample according to the present embodiment contains the nucleic acid A with a specific number of molecules, and contains more nucleic acid B than the number of molecules of the nucleic acid A. Moreover, in the sample according to the present embodiment, a ratio A/B of the number of molecules of the nucleic acid A with respect to the number of molecules of the nucleic acid B is specified.

Since the number of molecules of the nucleic acid A is an accurately measured specific number of molecules in the sample according to the present embodiment, it is possible to set the content thereof to be a very minute amount, such as one molecule, and it is thus possible to reduce the total amount of nucleic acids, namely the nucleic acid A and the nucleic acid B even if a large amount of nucleic acid B is present relative to the nucleic acid A. Also, it is possible to accurately evaluate a genetic testing apparatus using a standard sample with accuracy guaranteed by accuracy of the number of molecules in the nucleic acid A as will be described later in examples. Examples of the genetic testing apparatus include a quantitative PCR apparatus, a nucleic acid sequence determination apparatus (sequencer) (for example, a next generation sequencer), and the like.

Examples of the quantitative PCR apparatus include a real-time PCR apparatus and the like.

The real-time PCR apparatus is an apparatus that quantifies a template nucleic acid based on an amplification rate through measurement of amplification achieved by PCR with time (in real time). Quantification is performed using a fluorescent dye, and there are mainly an intercalation method and a hybridization method.

In the intercalation method, an amplification reaction of the template nucleic acid is performed in the presence of an intercalator that is uniquely inserted (intercalated) into a double-stranded DNA and emits fluorescence. Examples of the intercalator include SYBR Green I (CAS No.: 163795-75-3) or derivatives thereof. On the other hand, a method using a TaqMan (registered trademark) probe is the most typical hybridization method, and a probe obtained by linking a fluorescent substance and a quenching substance to complementary oligonucleotide to a target nucleic acid sequence is used.

The next generation sequencer is a group of base sequence analysis apparatuses that have been developed in recent years and has an analysis ability dramatically improved by performing a large amount of parallel processing on DNA templates amplified in a clone-like manner or a single DNA molecule inside a flow cell.

Sequencing techniques that can be used in the present embodiment can be sequencing techniques that acquire a plurality of reads through reading the same region in an overlapping manner (deep sequencing).

Specific sequencing techniques that can be used in the present embodiment are not particularly limited, and examples thereof include sequencing techniques by which it is possible to acquire a large read count per run based on a sequence principle other than Sanger's method, such as ion semiconductor sequencing, pyrosequencing, sequencing-by-synthesis using a reversible dye terminator, sequencing-by-ligation, and sequencing based on probe ligation of oligonucleotide.

Examples of performance of the genetic testing apparatus include base sequence analysis performance for each reaction space, a fluorescence measurement performance for each reaction space, and the like. High performance of the genetic testing apparatus may mean that there are no variations in base sequence analysis accuracy and fluorescent measurement accuracy for each reaction space, for example.

Note that as for the "number of molecules" in the specification, a molecule read as one nucleic acid is counted as one molecule by the genetic testing apparatus.

[Specific number of molecules]

In the specification, the specific number of molecules means that the number of molecules of the nucleic acid A contained in the sample is specified with accuracy at a specific or higher level. In other words, it is possible to state that the number of molecules of the nucleic acid A actually contained in the sample is known. In other words, the specific number of molecules in the specification is a value with higher accuracy and reliability as a number than a predetermined number of molecules (calculated estimated value) obtained through series dilution in the related art, and in particular, the value is a controlled value not depending on Poisson distribution even in a region of a low number of molecules of 1000 or less.

In regard to the controlled value, a variation coefficient CV representing uncertainty preferably falls within a size of any of values of $CV < 1/\sqrt{x}$ or $CV \leq 20\%$ with respect to the average number x of molecules.

With the sample according to the present embodiment, it is possible to more accurately evaluate performance of a genetic testing apparatus than in the related art by the number of molecules of the nucleic acid A contained in the sample being specified.

The number of molecules in the nucleic acid A may be any specific number of molecules that is smaller than that in the nucleic acid B. Specifically, the number of molecules in the nucleic acid A is preferably 1 or more and 200 or less, is more preferably 1 or more and 100 or less, and is further preferably 1 or more and 50 or less in terms of reducing the total amount of nucleic acids, namely the nucleic acid A and the nucleic acid B.

Here, the "number of molecules" and the "number of copies" of the nucleic acid A may be associated with each other. Specifically, in a case of a yeast fungus in a G1 phase in which a base sequence of the nucleic acid A is introduced into two locations on a genome, for example, the number of the same chromosomes into which the nucleic acid A has been introduced is 1, and the number of molecules in the nucleic acid A = the number of copies of the nucleic acid A = 2 if the number of yeast fungi = 1. In the specification, the specific number of molecules in the nucleic acid A may be referred to as an absolute number of the nucleic acid A.

In a case in which there are a plurality of reaction spaces (hereinafter, also referred to as "wells") containing the nucleic acid A when performance of a genetic testing apparatus is evaluated using the sample according to the present embodiment, the expression that the number of copies of the nucleic acid A contained in each well is the same means that variations in the number of nucleic acids A generated when the reaction space is filled with the sample is within an allowable range. Whether or not the variations in number of the nucleic acid A are within the allowable range can be determined based on information indicating uncertainty described below.

Examples of the information related to the specific number of molecules in the nucleic acid A include information regarding uncertainty, information of a carrier, which will be described later, information regarding the nucleic acid A, and the like.

"Uncertainty" is defined as a "parameter that characterizes variations in value that can be reasonably linked to a measurement amount accompanying a result of measurement" in ISO/IEC Guide 99: 2007 [International metrology terms-basic and general concepts and related terms (VIM)].

Here, the "value that can be reasonably linked to the measurement amount" means a candidate of a true value of the measurement amount. In other words, uncertainty means information regarding variations in measurement result caused by operations, equipment, and the like related to manufacturing of the measurement target. As the uncertainty increases, variations expected as a measurement result increase. Uncertainty may be a standard deviation obtained from the measurement result or may be a half value of a reliability level represented as a width of values in which the true value is included at a predetermined probability or more.

Uncertainty can be calculated based on a guideline related to uncertainty of measurement in Guide to the Expression of Uncertainty in Measurement (GUM: ISO/IEC Guide 98-3) and Japan Accreditation Board Note 10 tests.

As a method for calculating uncertainty, it is possible to apply two methods, namely a type A evaluation method using statistics of measurement values and the like and a type B evaluation method using information regarding uncertainty obtained from a calibration certificate, a manufacturer specification, published information, and the like, for example.

Uncertainty can be expressed at the same reliability level by converting uncertainty obtained due to factors such as operations and measurement into standard uncertainty. The standard uncertainty indicates variations in average value obtained from measurement values.

As an example of a method for calculating uncertainty, a factor causing uncertainty is extracted, and uncertainty due to each factor (standard deviation) is then calculated, for example. Then, the calculated uncertainty due to each factor is synthesized by a square sum method, thereby calculating synthetic standard uncertainty. Since the square sum method is used in the calculation for the synthetic standard uncertainty, it is possible to ignore factors with small uncertainty among the factors that cause the uncertainty.

As the information regarding uncertainty, a variation coefficient of the nucleic acid A with which the container is filled may be used. The variation coefficient means a relative value of the variations in number of nucleic acids A with which each container is filled generated when the container is filled with the nucleic acid A, for example. In other words, the variation coefficient means filling accuracy of the number of nucleic acids A with which the container is filled. The variation coefficient is a value obtained by dividing the standard deviation $\sigma$ by the average value x. Here, if the value obtained by dividing the standard deviation $\sigma$ by the average number of copies (the average number of filling copies) x is defined as a variation coefficient CV, the relational Equation 1 below is obtained.

$$CV = \sigma / x \qquad \text{(Equation 1)}$$

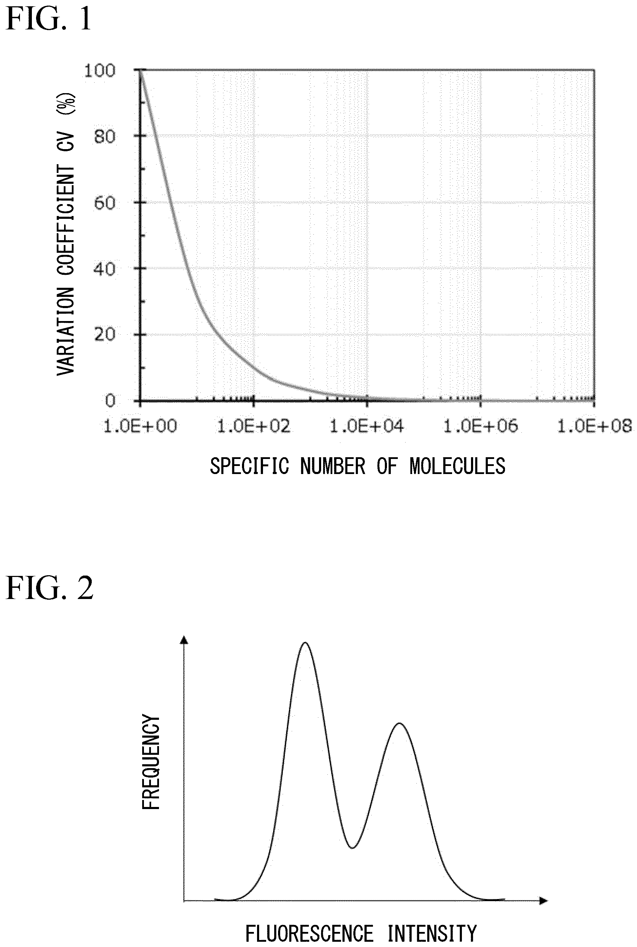

In general, the nucleic acid A has a random distribution state of Poisson distribution in the sample. Therefore, the standard deviation $\sigma$ can be regarded as satisfying the relational Equation 2 with the average number of copies x in a serial dilution method, that is, in the random distribution state in the Poisson distribution. In this manner, if the variation coefficient CV (CV value) of the average number of copies x from the standard deviation $\sigma$ and the average number of copies x is obtained using Equation 3 derived from Equation 1 above and Equation 2 below in a case in which the sample containing the nucleic acid A is diluted by the serial dilution method, the variation coefficient CV is as shown in Table 1 and Fig. 1. The CV value of the variation coefficient of the number of molecules with variations based on the Poisson distribution can be obtained from Fig. 1.

$$\sigma = \sqrt{x} \qquad \text{(Equation 2)}$$

$$CV = \frac{1}{\sqrt{x}} \qquad \text{(Equation 3)}$$

[Table 1]

| Average number of copies x | Variation coefficient CV |
|---|---|
| 1.00E+00 | 100.00% |
| 1.00E+01 | 31.62% |
| 1.00E+02 | 0.00% |
| 1.00E+03 | 3.16% |
| 1.00E+04 | 1.00% |
| 1.00E+05 | 0.32% |
| 1.00E+06 | 0.10% |
| 1.00E+07 | 0.03% |
| 1.00E+08 | 0.01% |

It is possible to ascertain from the results in Table 1 and Fig. 1 that in a case in which the well is filled with the 100 copies of the nucleic acid A by the series dilution method, for example, the number of copies of the nucleic acid A with which the well is finally filled has at least a variation coefficient (CV value) of 10% if other accuracy is ignored.

In regard to the number of molecules in the nucleic acid A, the CV value of the variation coefficient and the average specific number of copies x of the nucleic acid A preferably satisfy the following expression $CV < 1\sqrt{x}$ and further preferably satisfy $CV < 1/2\sqrt{x}$.

As the information regarding uncertainty, it is preferable to use information regarding uncertainty of all wells based on the specific number of molecules in the nucleic acid A contained in the wells in a case in which there are a plurality of wells containing the nucleic acid A.

Several factors that cause uncertainty are conceivable, and in a case in which a nucleic acid A is introduced into cells and the cells are counted and dispensed to the wells, examples thereof include the number of nucleic acids A in the cells (for example, the cell cycle or the like of the cells), means for disposing the cells in the well (including an ink jet apparatus and a result of an operation of each part of an apparatus or the like that controls a timing of an operation of the ink jet apparatus. For example, the number of cells contained in liquid droplets when the liquid droplets of a cell suspension are formed), a frequency at which the cells are disposed at appropriate positions in the well (for example, the number of cells disposed in the well), contamination caused by the nucleic acid A being included in the cell suspension due to cells destroyed in the cell suspension (inclusion of impurities; also referred to as "contamination"), and the like.

Examples of the information regarding the nucleic acid A include information regarding the number of molecules in the nucleic acid A. Examples of the information regarding the number of molecules in the nucleic acid A include information regarding uncertainty of the number of molecules in the nucleic acid A contained in the well, for example.

[Nucleic acid A]

In general, nucleic acids mean nitrogen-containing bases derived from purine or pyrimidine and organic compounds of polymers in which sugar and a phosphoric acid are regularly linked and include nucleic acid analogs. The nucleic acids are not particularly limited, can be appropriately selected in accordance with purposes, and examples thereof include a DNA, an RNA, a cDNA, and the like. The nucleic acid A may be a nucleic acid fragment, may be incorporated in a nucleus of a cell, and is preferably incorporated in a nucleus of a cell.

The nucleic acid A may be a natural product obtained from an organism, may be processed product, may be manufactured using a genetic recombination technique, or may be an artificially synthesized nucleic acid that is chemically synthesized. Among these, one kind may be used alone, or two or more kinds may be used together. In a case in which two or more kinds of nucleic acids A are used together, both the nucleic acids A contain a known smaller number of molecules than the nucleic acid B. By employing an artificially synthesized nucleic acid, it is possible to reduce impurities, to obtain lower molecules, and thereby to improve initial reaction efficiency.

The sequence of the nucleic acid A may be a sequence derived from any organisms from among eukaryotes, prokaryotes, multicellular organisms, and unicellular organisms. Examples of the eukaryotes include animals, insects, plants, fungi, algae, protozoa, and the like. Preferable examples of the animals include vertebrates such as fish, amphibians, reptiles, birds, and mammals. The sample for evaluating performance according to the present embodiment can be used as a standard sample with guaranteed accuracy when various DNA mixed samples are measured in fish environmental DNA analysis, meat type determination, halal testing, and the like by the nucleic acid A being a DNA of a vertebrate in the sample for evaluating performance according to the present embodiment.

Among the vertebrates, mammals are preferably employed. Examples of the mammals include humans, monkeys, marmosets, dogs, cows, horses, sheep, pigs, rabbits, mice, rats, guinea pigs, hamsters, and the like, and preferably examples include humans.

Among these, the nucleic acid A is preferably a human genomic DNA or a fragment thereof.

The artificially synthesized nucleic acid means a nucleic acid obtained by artificially synthesizing a nucleic acid including components (base, deoxyribose, a phosphoric acid) that are similar to those of a DNA or an RNA that is naturally present. The artificially synthesized nucleic acid may be a nucleic acid with a base sequence coding protein, for example, or may be a nucleic acid with any selected base sequence.

Examples of the analogs of the nucleic acid or a nucleic acid fragment include a nucleic acid or a nucleic acid fragment with a non-nucleic acid component linked thereto, a nucleic acid or a nucleic acid fragment labeled with a labeling agent such as a fluorescent dye or an isotope (for example, a primer or probe marked with a fluorescent dye or a radioisotope), and an artificial nucleic acid in which a partial chemical structure of nucleotides constituting the nucleic acid or the nucleic acid fragment is changed (for example, PNA, BNA, LNA, or the like).

The form of the nucleic acid A is not particularly limited, can be appropriately selected in accordance with a purpose, and examples thereof include a double-stranded nucleic acid, a single-stranded nucleic acid, and a partially double-stranded or single-stranded nucleic acid, and the nucleic acid A may be a cyclic or linear plasmid. Also, the nucleic acid may have modifications or mutations.

The nucleic acid A preferably has a specific base sequence with a known base sequence. The specific base sequence is not particularly limited and can be appropriately selected in accordance with a purpose, and examples thereof include a base sequence used for genetic disease testing, an unnatural base sequence that is not present in nature, a base sequence derived from animal cells, a base sequence derived from plant cells, a base sequence derived from fungal cells, a base sequence derived from bacteria, a base sequence derived from virus, and the like. Among these, one kind may be used alone, or two or more kinds may be used together.

In a case in which a base sequence used for genetic disease testing is used as the nucleic acid A, the nucleic acid A is not particularly limited as long as the nucleic acid A includes a base sequence unique to the genetic disease and can be appropriately selected in accordance with purposes.

The nucleic acid A may be a nucleic acid derived from a used cell or may be a nucleic acid introduced through gene introduction. The number of kinds of the nucleic acid A may be one or more. In a case in which two or more kinds of the nucleic acids A are contained, all the nucleic acids A include a known number of molecules that is smaller than that of the nucleic acid B. In a case in which a nucleic acid incorporated into a nucleus of a cell through gene introduction is used as the nucleic acid A, it is preferable to check that the nucleic acid A of the specific number of molecules (one molecule (one copy), for example) has been introduced into one cell. A method for checking that the nucleic acid A of the specific number of molecules has been introduced is not particularly limited and can be appropriately selected in accordance with purposes, and examples thereof include a sequence, a PCR method, a southern blotting method, and the like.

In a case in which the nucleic acid A is introduced into a nucleus of a cell, a gene-introducing method is not particularly limited as long as it is possible to introduce a target number of copies of the specific nucleic acid sequence into a target location, and examples thereof include homologous recombination, CRISPR/Cas9, CRISPR/Cpfl, TALEN, Zinc finger nuclease, Flip-in, Jump-in, and the like. Alternatively, the nucleic acid A may be introduced into a nucleus of a cell in the form of plasmid, an artificial chromosome, or the like. In a case in which a yeast fungus (yeast cell) is used as a cell, for example, it is preferable to employ homologous recombination among these in terms of high efficiency and ease of control.

The nucleic acid A may be micropartitioned in the sample using a microregion or a carrier. At this time, the nucleic acid A micropartitioned by a microregion or a carrier may be one or more molecules. In a case in which the nucleic acid A of two or more molecules is micropartitioned, the plurality of present nucleic acids A may have the same sequence or different sequences. In a case in which the nucleic acid A is micropartitioned by a carrier in the sample, the nucleic acid A is present with the nucleic acid A linked directly to the carrier or with the nucleic acid A indirectly linked thereto via a linker or the like.

Examples of forms of the microregion include cells, liposomes, microcapsules, viruses, droplets, emulsions, and the like. Examples of forms of the carrier include metal particles, magnetic particles, ceramic particles, polymer particles, protein particles, and the like.

(Cell)

A cell is a structural and functional unit forming an organism, and a specific sequence in a nucleus can be used as the nucleic acid A. The nucleic acid A may have a base sequence that is originally present in a nucleus or may be introduced from the outside.

The cell is not particularly limited and can be appropriately selected in accordance with purposes, and examples thereof include a eukaryotic cell, a prokaryotic cell, a multicellular organism cell, a unicellular organism cell, and the like. One kind of cell may be used alone, or two or more kinds of cells may be used together.

The eukaryotic cell is not particularly limited and can be appropriately selected in accordance with purposes, and examples thereof include an animal cell, an insect cell, a plant cell, a fungal cell, algae, protozoa, and the like. Among these, one kind may be used alone, or two or more kinds may be used together. In particular, the animal cell or the fungal cell is preferably used.

Examples of animals from which the animal cells can be derived include similar examples to those shown above for the "nucleic acid A". In particular, mammals are preferably employed.

The animal cells may be adhesive cells or may be floating cells. Adhesive cells may be primary cells collected directly from a tissue or an organ, may be cells obtained by passages through primary cells collected directly from a tissue or an organ, may be differentiated cells, or may be undifferentiated cells.

The differentiated cells are not particularly limited and can be appropriately selected in accordance with purposes, and examples thereof include endothelial cells such as liver cells that are parenchymal liver cells, stellate cells, Kupffer cells, vascular endothelial cells, sinusoidal endothelial cells, and corneal endothelial cells; epidermal cells such as fibroblasts, osteoblasts, osteoclasts, periodontal-derived cells, and epidermal keratinocytes; epithelial cells such as tracheal epithelial cells, intestinal epithelial cells, cervical epithelial cells, and corneal epithelial cells; mammary glandular cells and pericytes; muscle cells such as smooth muscle cells and cardiac muscle cells, nephrocytes, and islet of langerhans cells; nerve cells such as peripheral nerve cells and optic nerve cells; chondrocytes, osteocytes, and the like.

The undifferentiated cells are not particularly limited and can be appropriately selected in accordance with purposes, and examples thereof include totipotent stem cells such as embryonic stem cells (ES cells), and induced pluripotent stem cells (iPS cells); pluripotent stem cells such as mesenchymal stem cells; unipotent stem cells such as endothelial progenitor cells, and the like.

Fungal cells are not particularly limited and can be appropriately selected in accordance with purposes, and examples thereof include molds and yeast fungi. Among these, one kind may be used alone, or two or more kinds may be used together. In particular, yeast fungi are preferably used since it is possible to adjust the cell cycle and to use monoploid cells. The cell cycle means a process in which cell division occurs when the cells increase in number, and cells (daughter cells) generated in the cell division become cells (mother cells) that perform cell division again and produce new daughter cells.

The yeast fungi are not particularly limited and can be appropriately selected in accordance with purposes, and preferable examples thereof include yeast fungi synchronously cultivated in synchronization with the G0/G1 phase and immobilized in the G1 phase. Moreover, preferable examples of yeast fungi include Bar1 gene-deficient yeast with increased sensitivity of pheromone (sex hormones) that controls the cell cycle in the G1 phase. If the yeast fungi are Bar1 gene-deficient yeast, it is possible to lower the present ratio of yeast fungi that the cell cycle cannot be controlled and thereby prevent the number of molecules in the nucleic acid A in the cells contained in the sample from increasing or the like.

The prokaryotic cells are not particularly limited and can be appropriately selected in accordance with purposes, and examples thereof include eubacteria such as coliform bacteria, ancient bacteria, and the like. Among these, one kind may be used alone, or two or more kinds may be used together.

The cells are preferably dead cells. In the case of dead cells, it is possible to prevent cell division occurs after sorting and the amount of intracellular nucleic acid from changing. It is preferable that the cells be capable of emitting light when they receive light. If the cells are capable of emitting light when they receive light, it is possible to cause the cells to land inside the wells with the number of cells highly accurately controlled.

It is preferable that the cells be capable of emitting light when they receive light. Light receiving means the cells receiving light. Light emission of the cells is detected by an optical sensor. An optical sensor means a passive sensor that collects visible light beams that can be viewed by the human eye and any of near infrared rays and short-wavelength infrared rays with longer wavelengths than visible light beams, and lights up to a thermal infrared region and acquires the shape and the like of cells that are targets as image data.

The cells that are capable of emitting light when they receive light are not particularly limited and can be appropriately selected in accordance with purposes, and examples thereof include cells stained with a fluorescent dye, cells with expression of fluorescent protein, and cells labeled with fluorescently labeled antibody. The site in cells stained with the fluorescent dye, the expression site of the fluorescent protein, and the site labeled with the fluorescently labeled antibody are not particularly limited, and examples thereof include entire cells, cell nuclei, cell membranes, and the like.

Examples of the fluorescent dye include fluoresceins, azos, rhodamines, coumarins, pyrenes, cyanines, and the like. Among these, one kind may be used alone, or two or more kinds may be used together. Among these, fluoresceins, azos, rhodamines, or cyanines are particularly used, and eosin, Evans blue, trypan blue, rhodamine 6G, rhodamine B, rhodamine 123, or Cy3 is more preferably used.

Commercially available fluorescent dyes can be used, and examples of the commercially available product include product name: EosinY (manufactured by Wako Pure Chemical Corporation), product name: Evans Blue (manufactured by Wako Pure Chemical Corporation), product name: Trypan Blue (manufactured by Wako Pure Chemical Corporation), product name: Rhodamine 6G (manufactured by Wako Pure Chemical Corporation), product name: Rhodamine B (manufactured by Wako Pure Chemical Corporation), product name: Rhodamine 123 (manufactured by Wako Pure Chemical Corporation), and the like.

Examples of the fluorescent protein include Sirius, EBFP, ECFP, mTurquoise, TagCFP, AmCyan, mTFP1, MidoriishiCyan, CFP, TurboGFP, AcGFP, TagGFP, Azami-Green, ZsGreen, EmGFP, EGFP, GFP2, HyPer, TagYFP, EYFP, Venus, YFP, PhiYFP, PhiYFP-m, TurboYFP, ZsYellow, mBanana, KusabiraOrange, mOrange, TurboRFP, DsRed-Express, DsRed2, TagRFP, DsRed-Monomer, AsRed2, mStrawberry, TurboFP602, mRFP1, JRed, KillerRed, mCherry, mPlum, PS-CFP, Dendra2, Kaede, EosFP, KikumeGR and the like. Among these, one kind may be used alone, or two or more kinds may be used together.

The fluorescently labeled antibodies are not particularly limited as long as they can be linked to target cells and have been fluorescently labeled and can be appropriately selected in accordance with purposes, and examples thereof include an FITC labeled anti-CD4 antibody, a PE labeled anti-CD8 antibody, and the like. Among these, one kind may be used alone, or two or more kinds may be used together.

The volume-average particle diameter of the cells is preferably 30 $\mu$m or less, is more preferably 10 $\mu$m or less, and is particularly preferably 7 $\mu$m or less in the free state. If the volume-average particle diameter is 30 $\mu$m or less, the cells can be suitably used in a liquid droplet ejection means in an ink jet method, a cell sorter, or the like.

The volume-average particle diameter of the cells can be measured by the following measuring method, for example. In a case in which yeast is used as cells, it is possible to measure the volume-average particle diameter by extracting 10 $\mu$L of produced stained yeast dispersion, placing it on a plastic slide manufactured by PMMA, using an automated cell counter (product name: Countess Automated Cell Counter; manufactured by Invitrogen), and the like. Note that the number of cells can also be obtained by a similar measurement method.

The density of the cells in the cell suspension is not particularly limited, can be appropriately selected in accordance with purposes, is preferably $5 \times 10^4$/mL or more and $5 \times 10^8$/mL or less, and is more preferably $5 \times 10^4$/mL or more and $5 \times 10^7$/mL or less. If the cell density falls within the aforementioned range, it is possible to reliably include the cells in the ejected liquid droplets. The cell density can be measured using an automated cell counter (product name: Countess Automated Cell Counter manufactured by Invitrogen) similarly to the measurement method for the volume-average particle diameter.

(Liposomes)

Liposomes are lipid vesicles formed by a lipid bilayer including lipid molecules and specifically means vesicles including closed lipid with a space isolated from the outside by the generated lipid bilayer based on the polarity of a hydrophobic group and a hydrophilic group of the lipid molecules.

(Microcapsules)

Liposomes are closed vesicles formed by a lipid bilayer membrane using lipids and have an aqueous phase (internal aqueous phase) in the space of the closed vesicles. The internal aqueous phase includes water and the like. The liposomes may be single lamellas (single-layer lamellas, unilamellar, one layer of double-layer membrane) or may be multilayer lamellas (multi lamellas or multiple double-layer membranes with onion-shaped structures, in which the individual layers are sectioned by water-like layers).

Liposomes may be able to encapsulate the nucleic acid A, and the form thereof is not particularly limited. "Encapsulating" means that a form in which a nucleic acid is included in the internal aqueous phase or the membrane itself is employed for the liposome. Examples thereof include a form in which the nucleic acid A is sealed in the closed space formed by the membrane, a form in which the nucleic acid A is encapsulated in the membrane itself, and the like, and combinations thereof may also be employed.

The size (average particle diameter) of the liposome is not particularly limited as long as it can encapsulate the nucleic acid A. Further, a spherical form or a form that is close thereto is preferably used.

The component (membrane component) constituting the lipid bilayer of the liposome is selected from lipids. Any lipid can be used as long as it is dissolved in a mixed solvent of a water-soluble organic solvent and an ester-based organic solvent. Specific examples of lipids include phospholipids, lipids other than phospholipids, cholesterols, and derivatives thereof. Among these components, one kind may be used alone, or two or more kinds may be used together.

(Microcapsules)

The microcapsules mean minute granules having wall materials and hollow structures and can encapsulate the nucleic acid A in the hollow structures. The microcapsules are not particularly limited, and the wall material, the size, and the like can be appropriately selected in accordance with the purpose.

Examples of the wall material for microcapsules include a polyurethane resin, polyurea, a polyurea-polyurethane resin, a urea-formaldehyde resin, a melamine-formaldehyde resin, polyamide, polyester, polysulfone amide, polycarbonate, polysulfinate, epoxy resin, acrylic acid ester, methacrylic acid ester, vinyl acetate, gelatin, and the like. Among these, one kind may be used alone, or two or more kinds may be used together.

The size of the microcapsules is not particularly limited as long as it can encapsulate the nucleic acid A, and can be appropriately selected in accordance with purposes. A method for manufacturing microcapsules is not particularly limited and can be appropriately selected in accordance with the purposes, and examples thereof include an in-situ method, an interfacial polymerization method, a core salvation method, and the like.

[Nucleic acid B]

Examples of the nucleic acid B include the same as those exemplified in the aforementioned nucleic acid A. Among these, the nucleic acid B is preferably a human genomic DNA or a fragment thereof.

The sample in the present embodiment may contain one kind of such a nucleic acid B alone or two or more kinds thereof in combination. In a case in which two or more kinds of nucleic acids B are contained, both the nucleic acids B are contained by larger amounts than that of the nucleic acid A.

Although the number of molecules of the nucleic acid B may be any number as long as the number of molecules in the nucleic acid B is larger than the number of molecules in the nucleic acid A, the number of molecules in the nucleic acid B is preferably 200 or more, is more preferably 1000 or more, and is further preferably 5000 or more, and is particularly preferably 10000 or more in terms of obtaining an excessive amount as compared with the number of molecules in the nucleic acid A. On the other hand, the upper limit of the nucleic acid B can be about $10^8$ or less, for example, in terms of obtaining such an amount with which the total amount of nucleic acids, namely the nucleic acid A and the nucleic acid B does not prevent various kinds of processing (including a PCR reaction) in the genetic testing apparatus.

In a case in which two or more kinds of nucleic acids B are contained, it is preferable that the number of molecules in each nucleic acid B be equal to or greater than the aforementioned lower limit value and the total number of molecules in the nucleic acids B be equal to or less than the aforementioned upper limit value.

The ratio A/B of the number of molecules in the nucleic acid A with respect to the number of molecules in the nucleic acid B may be any specific known value, is not particularly limited, is preferably such that the number of molecules in the nucleic acid B is excessively larger than the number of molecules in the nucleic acid A, is preferably 10% or less, is more preferably 1% or less, and is further preferably less than 1%.

Also, the aforementioned ratio A/B preferably corresponds to the occurrence frequency of a specific genetic disease. For example, in a case in which the specific genetic disease is a homozygous mutation, and the occurrence frequency is 1 %, the nucleic acid A is a gene with a known sequence with mutation, the nucleic acid B is a gene with a known sequence with no mutation, and the aforementioned ratio A/B can be specified as 1/99. Also, in a case in which the specific genetic disease is a heterozygous mutation, and the occurrence frequency is 1 %, the nucleic acid A is a gene with a known sequence with mutation, the nucleic acid B is a gene with a known sequence with no mutation, and the aforementioned ratio A/B can be specified as 1/199. Moreover, in a case in which the nucleic acid A is an EGFR gene with mutation, and the nucleic acid B is an EGFR gene with no mutation, for example, as will be described in the examples below, the ratio A/B can be specified so as to correspond to the value of the occurrence frequency of the EGFR gene mutation. At this time, the nucleic acid A preferably includes a base sequence containing a sequence in which exons 18, 19, 20, and 21 of the EGFR gene are tandemly linked. Sequences such as restriction enzyme sites or the like and sequences that are homological to primers used for amplification of the nucleic acid A may further be added to the 5' terminal of the exon 18, between the exons, and the 3' terminal of the exon 21 as will be described in the examples below, for example. Specific examples of such a nucleic acid A include a nucleic acid having a base sequence represented by a sequence number 1 and the like.

In general, "genetic diseases" include single genetic diseases, multifactorial genetic diseases, chromosomal abnormalities, and the like. Among these, diseases that are developed by mutations are preferable. Mutations described here include substitutions, deletions, or insertions of nucleic acids in genes, gene fusions, and copy number variations. "Substitutions" mean states in which at least one base in a sequence of the gene is a different base. "Substitutions" include point mutations and one-base variations. "Deletions" and "insertions" mean states in which at least one base in the sequence in the gene is inserted and/or deleted. "Gene fusions" mean states in which a sequence on a 5' side of a certain gene and a sequence on a 3' terminal side of another gene are linked to each other due to chromosomal translocation or the like. "Copy number variations" mean that the number of copies on a genome per cell are different between individuals. Specific examples include variable nucleotide of tandem repeat (VNTR; repeated sequence variations), short tandem repeat polymorphism (STRP; microsatellite variations), gene amplification, and the like.

The total amount of nucleic acids included in the sample according to the present embodiment, that is, the total number of molecules in the nucleic acid A and the nucleic acid B may be any amount as long as it does not prevent various kinds of processing (including a PCR reaction) in the genetic testing apparatus and can be $10^8$ or less, for example. Moreover, in a case in which the sample according to the present embodiment is used as a standard sample in liquid biopsy, it is desirable that the total amount of the nucleic acids be preferably equivalent to the amount of nucleic acid (about 30 to 90 ng of human genome) in the sample used in the testing, and the total number of molecules in the nucleic acid A and the nucleic acid B is preferably 30000 or less and is more preferably 25000 or less.

On the other hand, although the lower limit of the total amount of nucleic acid is not particularly limited, the lower limit can be 3 or more and can be more than 200.

[Other components]

The performance evaluation sample according to the present embodiment can include a solvent as another component. Preferable examples of the solvent include water-soluble solvents such as water, ethanol, dimethyl sulfoxide (DMSO), acetone, N,N-dimethylformamide (DMF), and the like.

The sample according to the present embodiment can be widely used in the bio-related industry, the life science industry, the medical industry, and the like and can be suitably used for performance evaluation, accuracy management, and the like for a genetic testing apparatus, for example. Also, it is possible to suitably use the sample to evaluate the accuracy of genetic disease testing.

<Method for preparing sample for evaluating performance of genetic testing apparatus>

A preparation method according to the present embodiment will be described below.

The preparation method according to the present embodiment includes: an incorporating step of incorporating the nucleic acid A in the nucleic acid in the nucleus in a cell; a nucleic acid A filling step of producing a liquid droplet containing one cell in which the nucleic acid A is incorporated in the nucleic acid in the nucleus in a container and performing filling with a specific number of cells through control of the number of the liquid droplets; and a nucleic acid B filling step of filling the container with the nucleic acid B such that a ratio A/B of the number of molecules in the nucleic acid A with respect to the number of molecules in the nucleic acid B is a specific ratio. The preparation method according to the present embodiment preferably further includes a cell suspension purification step and a cell number-counting step, and more preferably includes a step of calculating certainty of the number of molecules in the nucleic acid A estimated in the cell suspension generation step, the nucleic acid A filling step, and the cell number-counting step, an output step, and a recording step, and further includes other steps as needed.

[Incorporating step]

In the incorporating step, the nucleic acid A is incorporated in the nucleic acid in the nucleus of the cell.

Although the number of molecules in the nucleic acid A incorporated in the nucleic acid in the nucleus in the cell is not particularly limited as long as the number of molecules is a specific number of molecules, the number of molecules is preferably one from the viewpoint of introduction efficiency.

Examples of a method for incorporating the nucleic acid A in the nucleic acid in the nucleus include a method similar to the method exemplified as a "method for introducing a gene" in the "nucleic acid A" above.

[Nucleic acid A filling step]

In the nucleic acid A filling step, a liquid droplet containing one cell in which the nucleic acid A is incorporated in the nucleic acid in the nucleus is produced, and filling with the specific number of cells is performed through control of the number of liquid droplets. Note that the liquid droplet means a liquid lump integrated due to surface tension.

The producing of and filling with the liquid droplets can be achieved by ejecting the cell suspension containing the cell in which the nucleic acid A is incorporated in the nucleic acid in the nucleus as liquid droplets and causing the liquid droplets to sequentially land inside the container. The ejection means that the cell suspension is caused to fly as liquid droplets. Sequential means that the event happens one by one in order. Landing means that the liquid droplets are caused to reach the well.

Although it is preferable to use a one-hole microtube, an eight-well tube, a 96-hole well plate, a 384-hole well plate, or the like as the container, it is also possible to dispense the same number of cells to each well in such a plate or it is also possible to put a different number of cells in a case in which there are a plurality of wells. Additionally, wells that do not contain any cells may be present.

As the ejection means, a means for ejecting the cell suspension as liquid droplets (hereinafter, also referred to as an "ejection head") can be suitably used.

Examples of a method for ejecting the cell suspension as liquid droplets include an on-demand method in the ink jet method, a continuous method, and the like. Among these, in the case of the continuous method, there is a trend that the dead volume of the used cell suspension increases for reasons of empty ejection before a stable ejection state is achieved, adjustment of the amount of liquid droplets, continuous formation of the liquid droplets even during movement between wells, and the like. In the present embodiment, it is preferable to reduce influences of the dead volume from the viewpoint of adjusting the number of cells, and the on-demand method is thus more suitable out of the aforementioned two methods.

Examples of the on-demand method include a plurality of known methods such as a pressure application method of ejecting the liquid by applying a pressure to the liquid, a thermal method of ejecting the liquid through membrane boiling caused by heating, and an electrostatic method of forming the liquid droplets by pulling the liquid droplets using an electrostatic attraction force. Among these, the pressure application method is preferably used for the following reasons.

In the electrostatic method, it is necessary to install an electrode facing the ejecting unit that holds the cell suspension and forms a liquid droplet. In the manufacturing method according to the present embodiment, the plate for receiving the liquid droplets is disposed to face it, and it is preferable that no electrodes be disposed in order to enhance a degree of freedom in plate configuration. As for the thermal method, local heating occurs, and there is thus a concern of influences on cells that are biomaterial and burning (kogation) of the heater portion. Since the influence of heat depends on the content and the use of the plate, it is not necessary to exclude it unconditionally, but the pressure application method is preferable because there is no concern about burning of the heater portion as compared with the thermal method.

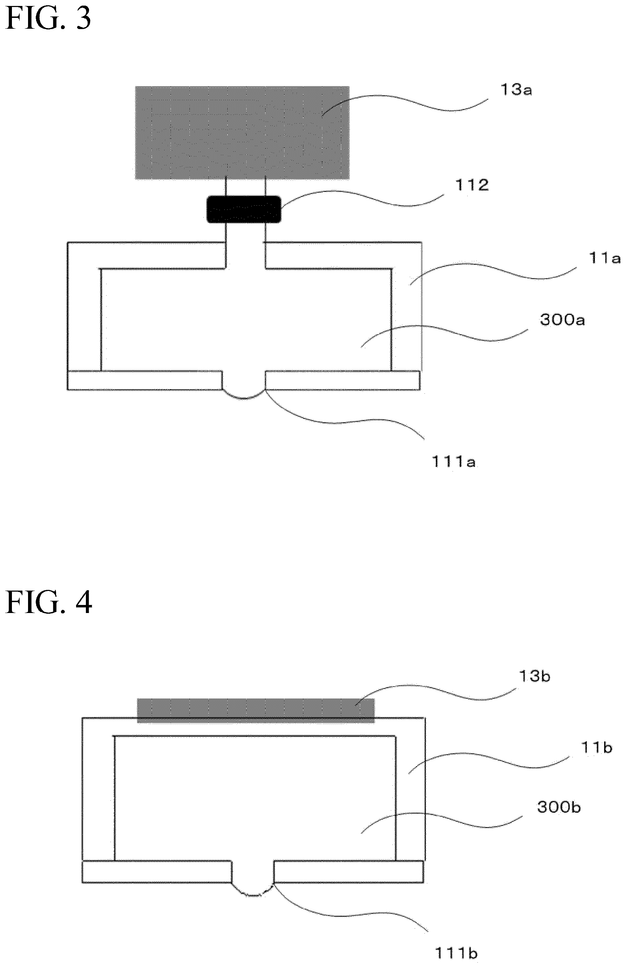

Examples of the pressure application method include a method of applying a pressure to the liquid using a piezoelectric element, a method of applying a pressure using a valve such as a solenoid valve, and the like. Configuration examples of the liquid droplet generation device that can be used for ejecting liquid droplets of the cell suspension are shown in Figs. 3 to 5.

Fig. 3 is a schematic view showing an example of an ejection head based on the solenoid valve method. The ejection head based on the solenoid valve method includes a motor 13a, a solenoid valve 112, a liquid chamber 11a, a cell suspension 300a, and a nozzle 111a. As the ejection head based on the solenoid valve method, it is possible to suitably use a dispenser from TechElan, for example.

Fig. 4 is a schematic view showing an example of an ejection head based on a piezoelectric method. The ejection head based on the piezoelectric method includes a piezoelectric element 13b, a liquid chamber lib, a cell suspension 300b, and a nozzle 111b. As the ejection head based on the piezoelectric method, it is possible to suitably use a single-cell printer from Cytena or the like.

Although any of these ejection heads can be used, it is preferable to use the piezoelectric method in order to enhance a throughput for generating the plate since it is not possible to repeatedly form liquid droplets at a high speed by the pressure application method using the solenoid valve. Also, according to the ejection head based on the piezoelectric method using a typical piezoelectric element 13b, there may be problems such as variations in the concentration of cells occurring due to sedimentation, and nozzle clogging occurring.

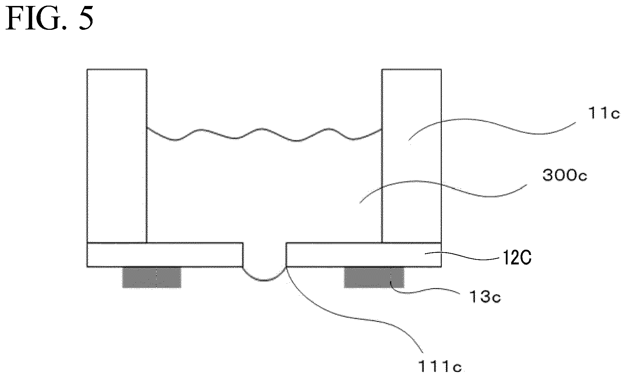

Therefore, the configuration shown in Fig. 5 and the like can be exemplified as a more preferable configuration. Fig. 5 is a schematic view of a modification example of an ejection head based on the piezoelectric method using a piezoelectric element in Fig. 4. The ejection head in Fig. 5 includes a piezoelectric element 13c, a liquid chamber 11c, a cell suspension 300c, and a nozzle 111c.

According to the ejection head in Fig. 5, it is possible to deform the membrane 12c in the up-down direction of the paper surface with a compressive stress applied in the lateral direction of the paper surface by a control device, which is not shown, applying a voltage to the piezoelectric element 13c.

Examples of methods other than the on-demand method include a continuous method in which liquid droplets are continuously formed. In the continuous method, periodic fluctuation is provided by a piezoelectric element or a heater when the liquid droplets are compressed and pushed out of the nozzle, and it is thus possible to continuously create minute liquid droplets. Moreover, it is possible to select whether to cause the liquid droplets to land on the well or to collect them in a collecting unit by controlling the ejection direction of the flying liquid droplet through an application of a voltage. For such a method, a cell sorter or a flow cytometer is used, and for example, it is possible to use a cell sorter SH800Z device manufactured by Sony Corporation.

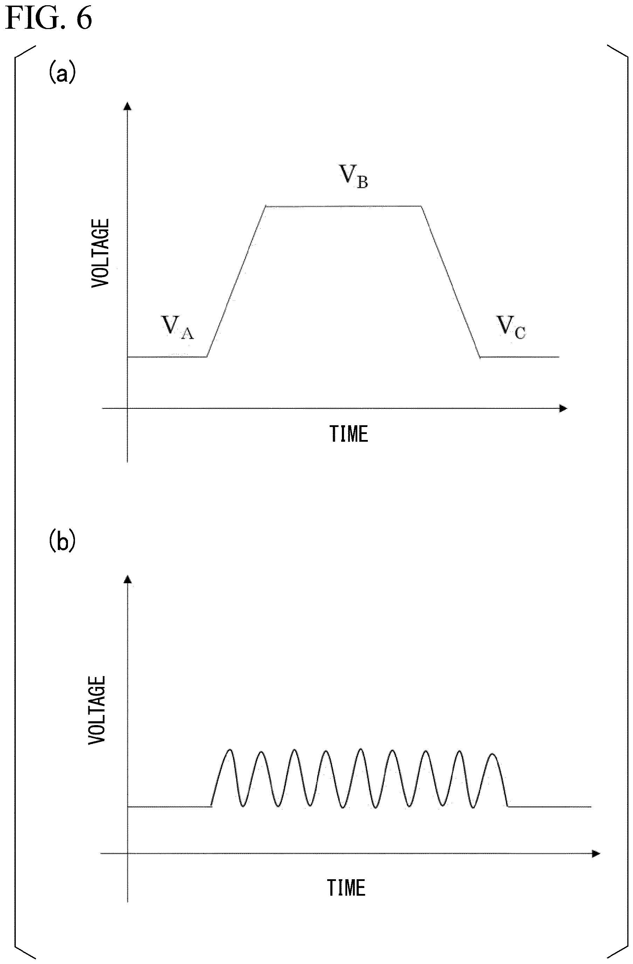

Fig. 6(a) is a schematic view showing an example of a voltage applied to the piezoelectric element. Also, Fig. 6(b) is a schematic view showing another example of a voltage applied to the piezoelectric element. Fig. 6(a) shows a drive voltage for forming liquid droplets. It is possible to form the liquid droplets based on the strength of the voltages ($V_A$, $V_B$, $V_C$). Fig. 6(b) shows a voltage for stirring the cell suspension without ejecting the liquid droplets.

It is possible to stir the cell suspension in the liquid chamber by inputting a plurality of pulses that are not strong enough to eject the liquid droplets and to curb the occurrence of concentration distribution due to sedimentation of cells during the period when the liquid droplets are not ejected.

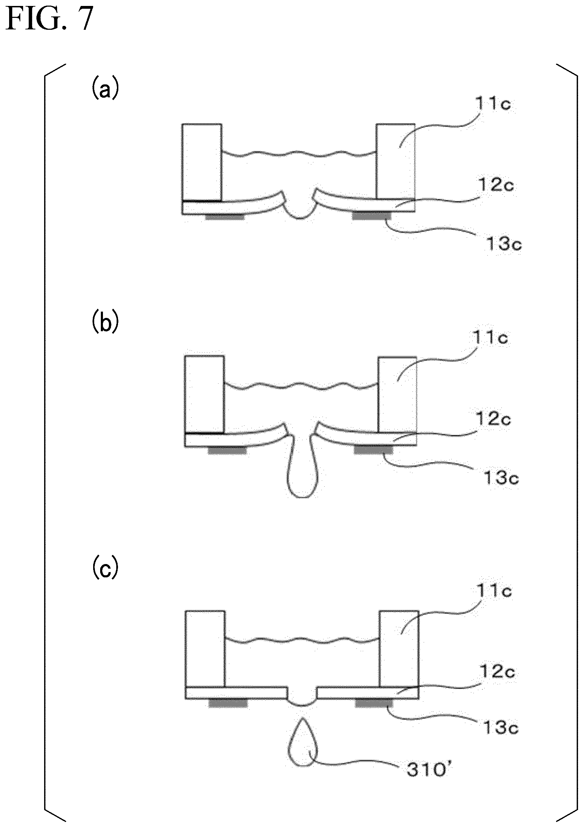

Liquid droplet-forming operations of the ejection head that can be used in the present embodiment will be described below. The ejection head can eject liquid droplets by applying a pulsed voltage to the upper and lower electrodes formed at the piezoelectric element. Figs. 7(a) to 7(c) are schematic views showing states of a liquid droplet at each of timings.

First, as shown in Fig. 7(a), the membrane 12c is suddenly deformed by applying a voltage to the piezoelectric element 13c, a high pressure is thus generated between the cell suspension held inside the liquid chamber 11c and the membrane 12c, and the liquid droplets are pushed out from the nozzle unit due to the pressure.

Next, as shown in Fig. 7(b), the pushing-out of the liquid from the nozzle unit is continued, and the liquid droplet grows during a time until the pressure is alleviated upward. Finally, as shown in Fig. 7(c), the liquid pressure in the vicinity of the interface between the cell suspension and the membrane 12c decreases when the membrane 12c returns to the original state, and a liquid droplet 310' is formed.

In the preparation method according to the present embodiment, the liquid droplet may be caused to sequentially land on the well by fixing a container made of a plate with wells formed therein to a movable stage and combining driving of the stage and forming of the liquid droplets from the ejection head. Although the method of moving the plate as movement of the stage has been described here, it is a matter of course that the ejection head may be moved.

The plate is not particularly limited, and it is possible to use a plate with wells formed therein as typically used in the field of biotechnology. The number of wells in the plate is not particularly limited, can be appropriately selected in accordance with purposes, and may be one or more. Although it is preferable to use a one-hole microtube, an eight-well tube, a 96-hole well plate, a 384-hole well plate, or the like as the plate, it is also possible to dispense the same number of cells to each well in such a plate or it is also possible to put a different number of cells in a case in which there are a plurality of wells. Additionally, wells that do not contain any cells may be present.

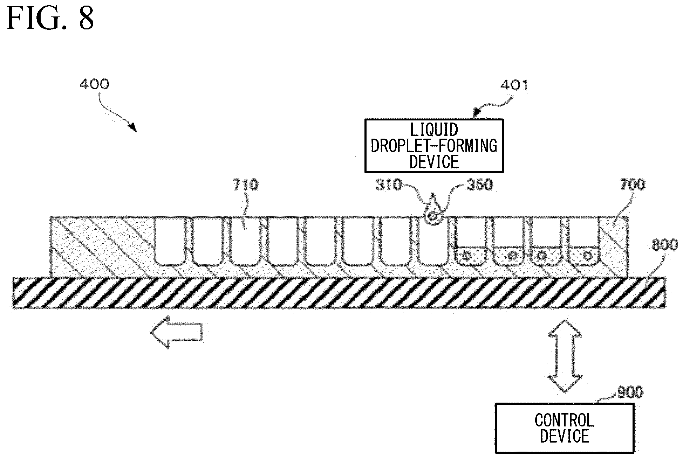

Fig. 8 is an outline view showing an example of a dispensing device 400 for causing the liquid droplets to sequentially land inside the wells in the plate. As shown in Fig. 8, the dispensing device 400 for causing the liquid droplets to land includes a liquid droplet-forming device 401, a plate 700, a stage 800, and a control device 900.

In the dispensing device 400, the plate 700 is disposed above the stage 800 configured to be movable. A plurality of wells 710 (recessed portions) on which the liquid droplets ejected from the ejection head of the liquid droplet-forming device 401 land are formed in the plate 700. The control device 900 causes the stage 800 to move and controls a relative positional relationship between the ejection head of the liquid droplet-forming device 401 and each well 710. In this manner, it is possible to sequentially eject liquid droplets 310 containing fluorescently stained cells 350 in each of the wells from the ejection head of the liquid droplet-forming device 401.

The control device 900 can be configured to include, for example, a CPU, a ROM, a RAM, a main memory, and the like. In this case, various functions of the control device 900 can be realized by the CPU reading a program recorded in the ROM or the like in the main memory and executing the program. However, a part or an entirety of the control device 900 may be realized only by hardware. Also, the control device 900 may be physically configured with a plurality of devices or the like.

As the ejected liquid droplets, it is preferable to cause the liquid droplets to land inside the wells to obtain a plurality of levels when the cell suspension is caused to land inside the wells. The plurality of levels mean a plurality of references as standards. Examples of the plurality of levels include a predetermined concentration gradient of the plurality of cells including the nucleic acid A in the wells, for example. The plurality of levels can be controlled using a value counted by the sensor.

[Nucleic acid B filling step]

In the nucleic acid B filling step, the container is filled with the nucleic acid B such that the ratio A/B of the number of molecules in the nucleic acid A with respect to the number of molecules in the nucleic acid B is a specific ratio.

Filling with the nucleic acid B can be achieved in a form in which it is dispersed or dissolved in a solvent. The filling method is not particularly limited, and it is possible to perform the filling by a manual technique using a micropipette or to automatically perform the filling using an autosampler, for example.

Since the number of molecules in the nucleic acid B is preferably an excessive amount with respect to the number of molecules in the nucleic acid A, a small measurement error is allowable within a numerical range in which the ratio A/B is a specific known ratio, and it is not necessary to strictly and accurately specify the number of molecules like the number of molecules in the nucleic acid A. A specific method of measuring the number of molecules in the nucleic acid B is not particularly limited, and examples thereof include absorption spectrometry, a real-time PCR method, a digital PCR method, high-speed liquid chromatography isotopic dilution mass spectrometry (LC-IDMS) for nucleic acid bases as analysis targets, inductively coupled plasma-atomic emission (ICP-OES) using phosphorus in the nucleic acid structure as an indicator, inductively coupled plasma mass spectrometry (ICP-MS), and the like. Among them, absorption spectrometry, the real-time PCR method, or the digital PCR method is preferably used.

By absorption spectrometry, it is possible to calculate the number of molecules by measuring light absorbance

(OD$_{260}$) at 260 nm in a solution containing the nucleic acid B, quantifying the concentration of the nucleic acid B, and dividing the quantified mass of the nucleic acid B by the molecular weight.

The real-time PCR method is a type of quantitative PCR (Q-PCR) and is adapted to quantify the nucleic acid B based on the amplification rate by measuring the amplification in PCR with time (in real time). Quantification is performed using a fluorescent dye, and there are mainly an intercalation method and a hybridization method.

In the intercalation method, an amplification reaction of the nucleic acid A is performed in the presence of an intercalator that is uniquely inserted (intercalated) into a double-stranded DNA and emits fluorescence. Examples of the intercalator include SYBR Green I (CAS No.: 163795-75-3) or derivatives thereof. On the other hand, a method using a TaqMan (registered trademark) probe is the most typical hybridization method, and a probe obtained by linking a fluorescent substance and a quenching substance to complementary oligonucleotide to a target nucleic acid sequence is used.

In a case in which the number of molecules in the nucleic acid B is calculated by using the real-time PCR method, the nucleic acid B and a plurality of nucleic acids A with a mutually different number of molecules (for example, three nucleic acids A with 10 molecules (copies), 50 molecules (copies), and 100 molecules (copies)) can be measured by the real-time PCR method as shown in the examples, which will be described later, and the number of molecules in the nucleic acid B can be determined using the calibration curve created by the plurality of nucleic acids A with a mutually different number of molecules.

[Cell suspension generation step]

The cell suspension generation step is a step of generating a cell suspension containing a plurality of cells including the nucleic acid A in the nucleus and a solvent. The solvent means a liquid used to disperse the cells. The suspension in the cell suspension means a state in which the cells are present in a dispersed manner in the solvent. The generation means creating.

(Cell suspension)

The cell suspension contains a plurality of cells having the nucleic acid A in the nucleus and a solvent, preferably contains an additive, and further contains other components as needed. The plurality of cells having the nucleic acid A in the nucleus are as described in "Cells" of "Nucleic Acid A" above.

(Solvent)

The solvent is not particularly limited and can be appropriately selected in accordance with purposes, and examples thereof include water, a culture solution, a separator solution, a diluting solution, a buffer solution, a dissolved organic substance solution, an organic solvent, a polymer gel solution, a colloidal solution, an electrolyte aqueous solution, an inorganic salt aqueous solution, a metal aqueous solution, a mixture solution thereof, and the like. Among these, one kind may be used alone, or two or more kinds may be used together. Among these, water or a buffer solution is preferably used, and water, phosphate-buffered saline (PBS), or a Tris-EDTA buffer solution (TE) is more preferably used.

(Additive)

The additive is not particularly limited, can be appropriately selected in accordance with purposes, and examples thereof include a surfactant, a nucleic acid, a resin, and the like. Among these, one kind may be used alone, or two or more kinds may be used together.

The surfactant can prevent cell-to-cell aggregation and improve continuous ejection stability. The surfactant is not particularly limited and may be appropriately selected in accordance with purposes, and examples thereof include an ionic surfactant and a nonionic surfactant. Among these, one kind may be used alone, or two or more kinds may be used together. Among these, a nonionic surfactant is preferably used since it does not degenerate or inactivate protein although it depends on the amount of addition.

Examples of the ionic surfactant include fatty acid sodium, fatty acid potassium, alpha-sulfo fatty acid ester sodium, linear sodium alkylbenzene sulfonate, sulfuric acid alkyl ester sodium, alkyl ether sulfonic acid ester sodium, alpha olefin sodium sulfonate, and the like. Among these, one kind may be used alone, or two or more kinds may be used together. In particular, fatty acid sodium is preferably used, and sodium dodecyl sulfate (SDS) is more preferably used.

Examples of the nonionic surfactant include alkyl glycoside, alkyl polyoxyethylene ether (Brij series and the like), octylphenol ethoxylates (Triton X series, Igepal CA series, Nonidet P series, Nikkol OP series, and the like), polysorbates (Tween series such as Tween 20, and the like), sorbitan fatty acid ester, polyoxyethylene fatty acid ester, alkyl maltoside, sucrose fatty acid ester, glycoside fatty acid ester, glycerin fatty acid ester, propylene glycol fatty acid ester, fatty acid monoglyceride, and the like. Among these, one kind may be used alone, or two or more kinds may be used together. In particular, polysorbates are preferably used.

The content of the surfactant is not particularly limited, can be appropriately selected in accordance with purposes, and is preferably 0.001% by mass or more and 30% by mass or less with respect to the entire amount of the cell suspension. It is possible to obtain an effect of addition of the surfactant if the content is 0.001% by mass or more, to curb aggregation of cells if the content is 30% by mass or less, and thereby to strictly control the number of copies of the nucleic acid in the cell suspension.

The nucleic acid is not particularly limited as long as it does not affect detection of the nucleic acids (the nucleic acid A and the nucleic acid B) that are targets of testing and can be appropriately selected in accordance with purposes, and examples thereof include ColE1 DNA and the like. It is possible to prevent the nucleic acid A from adhering to the well wall surface and the like by adding the nucleic acid.

The resin is not particularly limited and can be appropriately selected in accordance with purposes, and examples thereof include polyethyleneimide and the like.

(Other components)

Other components are not particularly limited and can be appropriately selected in accordance with purposes, and examples thereof include a crosslinking agent, a pH adjuster, a preservative, an antioxidant, an osmotic pressure adjuster, a wetting agent, a dispersant, and the like.

A method of dispersing the cells is not particularly limited and can be appropriately selected in accordance with purposes, and examples thereof include a media method such as a bead mill, an ultrasonic method such as an ultrasonic homogenizer, a method using a pressure difference such as a French press, and the like. Among these, one kind may be used alone, or two or more kinds may be used together. Among these, the ultrasonic method is preferably used because it causes less damage to cells. In the media method, a crushing ability is strong, and the cell membranes or cell walls may be destroyed or media may be included as contamination.

A method of screening cells is not particularly limited and can be appropriately selected in accordance with purposes, and examples thereof include wet classification, a cell sorter, screening using a filter, and the like. Among these, one kind may be used alone, or two or more kinds may be used together. In particular, the cell sorter and the screening using a filter are preferably used since there is less damage to the cells.

For the cells, it is preferable to estimate the number of molecules in the nucleic acid A based on the number of cells contained in the cell suspension by measuring the cell cycle of the cells. The measuring of the cell cycle means that the number of cells due to cell division is expressed as a numerical value. The estimating of the number of molecules in the nucleic acid A means that the number of copies of the nucleic acid A is obtained from the number of cells.

The counting target may be how many nucleic acids A are in the cells instead of the number of the cells. Since introduction of one copy of nucleic acid A per cell is selected or the nucleic acid A is introduced into the cell through genetic recombination, the number of nucleic acids A may be considered to be equal to the number of cells. However, since the cells divide at a specific cycle, copying of the nucleic acid is performed in the cells. Although the cell cycle differs depending on the type of cells, it is possible to calculate an expected value and certainty of the number of nucleic acids A contained in one cell by picking up a predetermined amount of solution from the cell suspension and measuring the cycle of a plurality of cells. This can be achieved by observing the cells with nucleus stained using a flow cytometer, for example.

The certainty means a probability that in a case in which some events may occur, a degree of likelihood with which a specific one event occurs is predicted in advance and the event actually occurs. The calculation means obtaining a numerical value after calculation.

Fig. 2 is a graph showing an example of a relationship between a frequency of cells for which DNA has already been copied and fluorescence intensity. As shown in Fig. 2, two peaks appear depending on the presence/absence of copying of the DNA on a histogram, and it is thus possible to calculate the proportion at which the cells after DNA copying are present. It is possible to calculate the average number of molecules in the nucleic acid A contained in one cell from the calculation result and to calculate an estimated number of molecules in the nucleic acid A by multiplying the aforementioned result of counting the number of cells by this.

Also, it is preferable to perform processing of controlling the cell cycle before producing the cell suspension, and it is possible to accurately calculate the number of molecules in the nucleic acid A from the number of cells by uniformly considering the numbers in a state before or after the copying as described above occurs.

For the estimated specific number of molecules, certainty (probability) is preferably calculated. It is possible to express and output certainty as dispersion or a standard deviation based on these numerical values by calculating the certainty (probability). In a case in which influences of a plurality of factors are added up, it is possible to use a square sum root of a standard deviation that is generally used. For example, it is possible to use a correct answer rate of the number of ejected cells, the number of DNAs in the cells, a landing rate at which the ejected cells land inside the well, and the like as the factors. It is also possible to select and calculate an item with a large influence from among these.

[Cell number-counting step]

The cell number-counting step is a step of counting the number of cells contained in a liquid droplet with a sensor after the liquid droplet is produced and before the liquid droplet lands on the container. The sensor means an apparatus that replaces mechanical, electromagnetic, thermal, acoustic, or chemical characteristics of natural phenomena or artificial substances or spatial information/time information indicated by them into signals of other media that can be easily handled by humans or machines by applying some scientific principles. Counting means counting numbers.
The cell number-counting step is not particularly limited as long as the number of cells contained in the liquid droplet is counted with a sensor after the liquid droplet is ejected and before the liquid droplet lands on the well, can be appropriately selected in accordance with purposes, and may include the process of observing cells before the ejection and the process of counting the cells before the landing.
As the counting of the number of cells contained in the liquid droplet after the liquid droplet is ejected and before the liquid droplet lands on the well, the cells in the liquid droplet are preferably observed at a timing at which the liquid droplet is at a location immediately above the well opening portion through which the liquid droplet is expected to reliably enter the well in the plate.
Examples of the method for observing the cells in the liquid droplet include an optical detecting method, an electrical or magnetic detecting method, and the like.

(Method for optical detection)

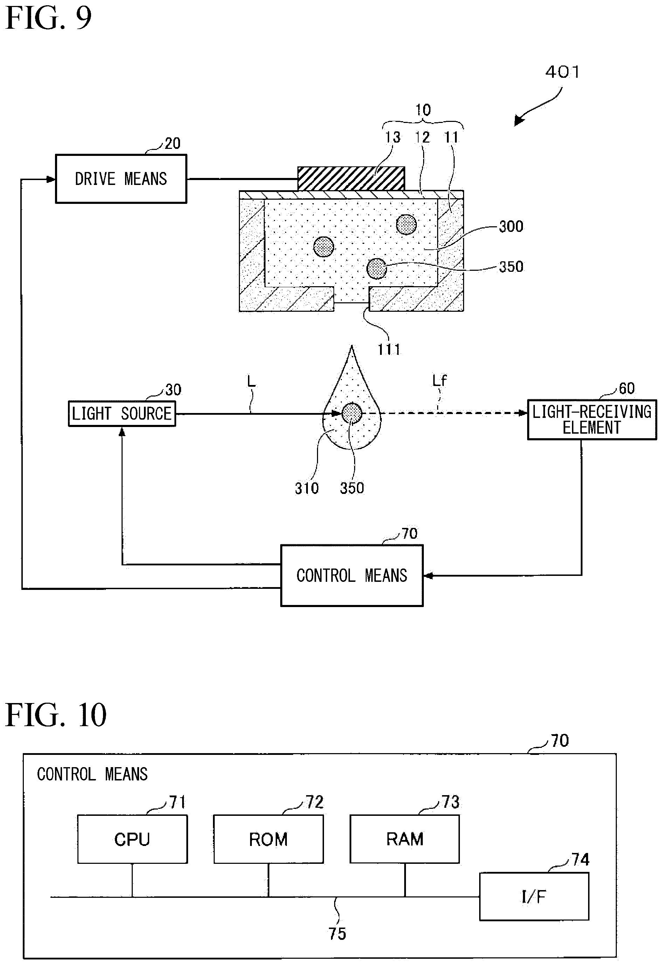

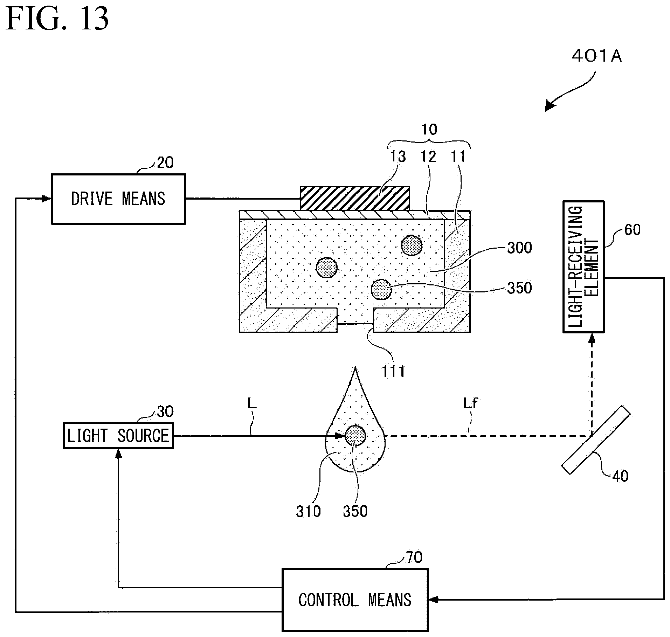

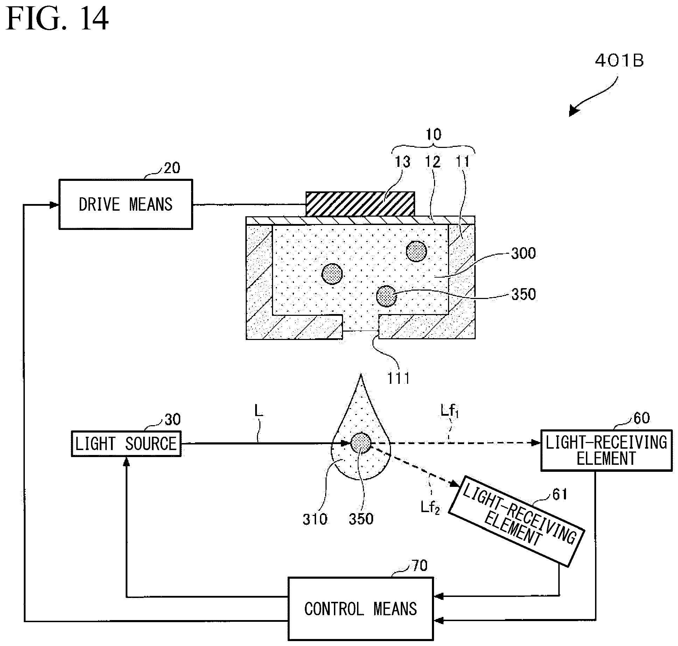

A method for optical detection will be described below using Figs. 9, 13, and 14. Fig. 9 is a schematic diagram showing an example of the liquid droplet-forming device 401. Figs. 13 and 14 are schematic diagrams showing other examples (401A, 401B) of the liquid droplet-forming device. As shown in Fig. 9, the liquid droplet-forming device 401 includes an ejection head (liquid droplet ejection means) 10, a drive means 20, a light source 30, a light-receiving element 60, and a control means 70.
In Fig. 9, a liquid obtained by causing cells to be subject to fluorescent staining and dispersing them in a predetermined solution was used as a cell suspension, and counting is performed by irradiating the liquid droplet formed from the ejection head with light with a specific wavelength emitted from the light source and detecting the fluorescence emitted from the cells with the light-receiving element. At this time, in addition to the method of staining the cells with the fluorescent dye, autofluorescence emitted by molecules originally contained in the cells may be used, or a gene that codes fluorescent protein (for example, a green fluorescent protein (GFP)) may be introduced into the cells in advance such that the cells emit fluorescence. Irradiation with light means irradiating light thereon.
The ejection head 10 includes a liquid chamber 11, a membrane 12, and a drive element 13 and can eject, as liquid droplets, a cell suspension 300 with fluorescently stained cells 350 suspended therein.
The liquid chamber 11 is a liquid-holding unit that holds the cell suspension 300 with the fluorescently stained cells 350 suspended therein, and a nozzle 111 that is a through-hole is formed on the lower surface side. The liquid chamber 11 can be formed of, for example, metal, silicon, or ceramics. Examples of the fluorescently stained cells 350 include inorganic microparticles, organic polymer particles, and the like stained with a fluorescent dye.
The membrane 12 is a film-shaped member fixed to the upper end portion of the liquid chamber 11. The planar shape of the membrane 12 can be, for example, a circular shape or may be an oval shape, a quadrangular shape, or the like.
The drive element 13 is provided on the upper surface side of the membrane 12. The shape of the drive element 13 can be designed in accordance with the shape of the membrane 12. In a case in which the planar shape of the membrane 12 is a circular shape, it is preferable to provide a circular drive element 13.
It is possible to cause the membrane 12 to vibrate by the drive means 20 supplying a drive signal to the drive element 13. It is possible to cause the liquid droplets 310 containing the fluorescently stained cells 350 to be ejected from the nozzle 111 through the vibration of the membrane 12.
In a case in which a piezoelectric element is used as the drive element 13, it is possible to employ a structure provided with electrodes for applying a voltage to an upper surface and a lower surface of a piezoelectric material, for example. In this case, a compressive stress is applied in the lateral direction of the paper surface by the drive means 20 applying the voltage between the upper and lower electrodes of the piezoelectric element, and the membrane 12 can thus be vibrated in the up-down direction of the paper surface. As the piezoelectric material, it is possible to use lead zirconate titanate (PZT), for example. Additionally, it is possible to use various piezoelectric materials such as a bismuth iron oxide, a metallic niobate, barium titanate, or a material obtained by adding metal or a different oxide to these materials.

The light source 30 irradiates the flying liquid droplets 310 with light L. Note that "flying" means a state of the liquid droplets 310 between ejection from the liquid droplet ejection means 10 and landing on the landing target. The flying liquid droplets 310 have substantially spherical shapes at a position at which they are irradiated with the light L. Moreover, the beam shape of the light L is a substantially circular shape.

Here, the beam diameter of the light L is preferably about 10 to 100 times the diameter of each liquid droplet 310. This is for reliably irradiating the liquid droplet 310 with the light L from the light source 30 even in a case in which there are variations in position of the liquid droplet 310.

However, it is not preferable that the beam diameter of the light L significantly exceed 100 times the diameter of the liquid droplet 310. This is because the energy density of the light with which the liquid droplet 310 is irradiated decreases, the amount of light of fluorescence Lf emitted using the light L as excitation light thus decreases, and it becomes difficult to perform detection with the light-receiving element 60.

The light L emitted from the light source 30 is preferably pulse light, and for example, a solid laser, a semiconductor laser, a dye laser, or the like are preferably used. The pulse width in a case in which the light L is pulse light is preferably 10 $\mu$s or less and is more preferably 1 $\mu$s or less. Although energy per unit pulse significantly depends on an optical system, such as the presence/absence of light collection, the energy is preferably about 0.1 $\mu$J or more and is more preferably 1 $\mu$J or more.

The fluorescently stained cells 350 are contained in the flying liquid droplet 310, and the light-receiving element 60 receives fluorescence Lf emitted by the fluorescently stained cells 350 absorbing the light L as excitation light. The fluorescence Lf is emitted to all directions from the fluorescently stained cells 350, and the light-receiving element 60 can thus be disposed at any selected position at which it can receive the fluorescence Lf. At this time, the light-receiving element 60 is preferably disposed at a position on which the light L emitted from the light source 30 is not directly incident, in order to improve contrast.

The light-receiving element 60 is not particularly limited as long as the element can receive the fluorescence Lf emitted from the fluorescently stained cells 350 and can be appropriately selected in accordance with purposes, and an optical sensor that receives fluorescence from the cells in the liquid droplet by irradiating the liquid droplet with light with a specific wavelength is preferably used. Although examples of the light-receiving element 60 include a primary element such as a photodiode or a photosensor, for example, it is preferable to use a photomultiplier tube or an avalanche photodiode in a case in which measurement with high sensitivity is needed. As the light-receiving element 60, a secondary element such as a charge-coupled device (CCD), a complementary metal oxide semiconductor (CMOS), or a gate CCD may be used, for example.

Note that since the fluorescence Lf emitted by the fluorescently stained cells 350 is weak as compared with the light L emitted by the light source 30, a filter for attenuating a wavelength range of the light L may be installed in a previous stage (on the light-receiving surface side) of the light-receiving element 60. It is thus possible to obtain an image of the fluorescently stained cells 350 with significantly high contrast by the light-receiving element 60. As the filter, it is possible to use a notch filter and the like that attenuate a specific wavelength range including the wavelength of the light L, for example.

Also, although the light L emitted from the light source 30 is preferably pulse light as described above, the light L emitted from the light source 30 may be successively oscillated light. In this case, it is preferable to control the light-receiving element 60 such that it can take light at a timing at which the flying liquid droplet 310 is irradiated with the successively oscillated light and causes the light-receiving element 60 to receive the fluorescence Lf.

The control means 70 has a function of controlling the drive means 20 and the light source 30. Also, the control means 70 has a function of obtaining information based on the amount of light received by the light-receiving element 60 and counting the number of fluorescently stained cells 350 (including a case of zero) contained in the liquid droplet 310. Hereinafter, operations of the liquid droplet-forming device 401 including operations of the control means 70 will be described with reference to Figs. 10 to 12.

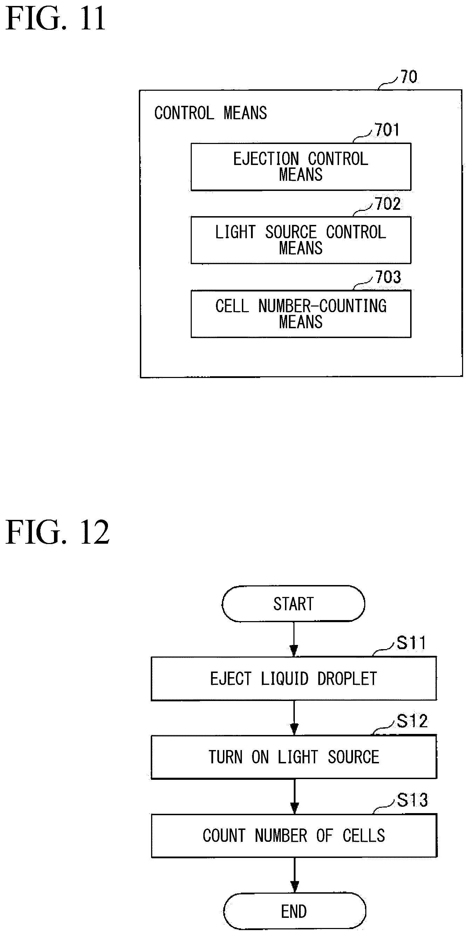

Fig. 10 is a diagram showing, as an example, hardware blocks of the control means in the liquid droplet-forming device in Fig. 9. Fig. 11 is a diagram showing, as an example, functional blocks of the control means in the liquid droplet-forming device in Fig. 9. Fig. 12 is a flowchart showing an example of operations of the liquid droplet-forming device.

As shown in Fig. 10, the control means 70 includes a CPU 71, a ROM 72, a RAM 73, a communication interface (communication I/F) 74, and a bus line 75. The CPU 71, the ROM 72, the RAM 73, and the I/F 74 are connected to each other via the bus line 75.

The CPU 71 controls each function of the control means 70. The ROM 72 that is a storage means stores programs executed by the CPU 71 to control each function of the control means 70 and various kinds of information. The RAM 73 that is a storage means is used as a work area or the like of the CPU 71. Also, the RAM 73 can temporarily store predetermined information. The I/F 74 is an interface for connecting the liquid droplet-forming device 401 to other equipment or the like. The liquid droplet-forming device 401 may be connected to an external network or the like via the I/F 74.

As shown in Fig. 11, the control means 70 includes, as functional blocks, an ejection control means 701, a light source control means 702, and a cell number-counting means (cell number detection means) 703.

Counting of the number of cells performed by the liquid droplet-forming device 401 will be described with reference to Figs. 11 and 12. First, in Step S11, the ejection control means 701 of the control means 70 provides an ejection command to the drive means 20. The drive means 20 that has received the ejection command from the ejection control means 701 supplies a drive signal to the drive element 13 and causes the membrane 12 to vibrate. The liquid droplet 310 containing the fluorescently stained cells 350 is ejected from the nozzle 111 through the vibration of the membrane 12.

Next, in Step S12, the light source control means 702 of the control means 70 issues a turning-on command to the light source 30 in synchronization with the ejection of the liquid droplet 310 (in synchronization with the drive signal supplied from the drive means 20 to the liquid droplet ejection means 10). In this manner, the light source 30 is turned on, and the flying liquid droplet 310 is irradiated with the light L.

Note that the synchronization described here does not mean that light is emitted at the same time as the ejection of the liquid droplet 310 performed by the liquid droplet ejection means 10 (at the same time as the supply of the drive signal from the drive means 20 to the liquid droplet ejection means 10) but means light emission from the light source 30 at a timing at which the liquid droplet 310 is irradiated with the light L when the liquid droplet 310 flies and reaches a predetermined position. In other words, the light source control means 702 controls the light source 30 to emit light with a delay of a predetermined period of time with respect to the ejection of the liquid droplet 310 performed by the liquid droplet ejection means 10 (the drive signal supplied from the drive means 20 to the liquid droplet ejection means 10).

For example, a speed v of the liquid droplet 310 ejected when the drive signal is supplied to the liquid droplet ejection means 10 is measured in advance. Then, a time t between the ejection of the liquid droplet 310 and the arrival at a predetermined position is calculated based on the measured speed v, and the timing at which the light source 30 is irradiated with light is delayed by time t with respect to the timing at which the drive signal is supplied to the liquid droplet ejection means 10. In this manner, it is possible to perform satisfactory light emission control and to reliably irradiate the liquid droplet 310 with the light from the light source 30.

Next, in Step S13, the cell number-counting means 703 of the control means 70 counts the number (including a case of zero) of the fluorescently stained cells 350 contained in the liquid droplet 310 based on the information from the light-receiving element 60. Here, information from the light-receiving element 60 includes a luminance value (amount of light) and the area value of the fluorescently stained cells 350.

The cell number-counting means 703 can count the number of fluorescently stained cells 350 by comparing the amount of light received by the light-receiving element 60 with a preset threshold value, for example. In this case, a primary element may be used or a secondary element may be used as the light-receiving element 60.

In a case in which the secondary element is used as the light-receiving element 60, the cell number-counting means 703 may use the method for performing image processing for calculating a luminance value or an area of the fluorescently stained cells 350 based on a secondary image obtained from the light-receiving element 60. In this case, the cell number-counting means 703 can count the number of fluorescently stained cells 350 by calculating the luminance value or the area value of the fluorescently stained cells 350 through image processing and comparing the calculated luminance value or area value with a preset threshold value.

Note that the fluorescently stained cells 350 may be cells or stained cells. The stained cells mean cells stained by a fluorescent dye or cells from which fluorescent protein can be expressed. In the stained cells, the aforementioned fluorescent dyes can be used. Moreover, the aforementioned fluorescent proteins can be used.

In this manner, the drive signal is suppled from the drive means 20 to the liquid droplet ejection means 10 holding the cell suspension 300 with the fluorescently stained cells 350 suspended therein, the liquid droplet 310 containing the fluorescently stained cells 350 is ejected, and the flying liquid droplet 310 is irradiated with the light L from the light source 30, in the liquid droplet-forming device 401. Then, the fluorescently stained cells 350 contained in the flying liquid droplet 310 emit the fluorescence Lf using the light L as excitation light, and the light-receiving element 60 receives the fluorescence Lf. Moreover, the cell number-counting means 703 counts the number of fluorescently stained cells 350 contained in the flying liquid droplet 310 based on information from the light-receiving element 60.

In other words, according to the liquid droplet-forming device 401, since the number of fluorescently stained cells 350 contained in the flying liquid droplet 310 is actually observed at the site, it is possible to further improve the counting accuracy of the number of the fluorescently stained cells 350 as compared with the related art. Also, since the fluorescently stained cells 350 contained in the flying liquid droplet 310 are irradiated with the light L to cause the fluorescence Lf to be emitted, and the light-receiving element 60 receives the fluorescence Lf, it is possible to obtain an image of the fluorescently stained cells 350 with high contrast, and it is thus possible to reduce an occurrence frequency of erroneous counting of the number of fluorescently stained cells 350.

Fig. 13 is a schematic view showing a modification example of the liquid droplet-forming device 401 in Fig. 9. As shown in Fig. 13, a liquid droplet-forming device 401A is different from the liquid droplet-forming device 401 (see Fig. 9) in that a mirror 40 is disposed in a previous stage of the light-receiving element 60. Note that description of the same components as those in the embodiment described above may be omitted.

In this manner, the mirror 40 is disposed in the previous stage of the light-receiving element 60 in the liquid droplet-forming device 401A, and it is thus possible to improve a degree of freedom in layout of the light-receiving element 60.

For example, although there is a concern that interference occurs between the landing target and the optical system (the light-receiving element 60, in particular) of the liquid droplet-forming device 401 when the nozzle 111 is caused to approach the landing target in the layout in Fig. 9, it is possible to avoid the occurrence of interference by employing the layout in Fig. 13.

As shown in Fig. 13, it is possible to shorten the distance (gap) between the landing target at which the liquid droplet 310 lands and the nozzle 111 and to curb variations in landing position by changing the layout of the light-receiving element 60. As a result, it is possible to improve accuracy of dispensing.

Fig. 14 is a schematic view showing another modification example of the liquid droplet-forming device 401 in Fig. 9. As shown in Fig. 14, a liquid droplet-forming device 401B is different from the liquid droplet-forming device 401 (see Fig. 9) in that a light-receiving element 61 that receives fluorescence $Lf_2$ emitted from the fluorescently stained cells 350 is included in addition to the light-receiving element 60 that receives the fluorescence $Lf_1$ emitted from the fluorescently stained cells 350. Note that description of the same components as those in the embodiment described above may be omitted.

Here, the fluorescence $Lf_1$ and $Lf_2$ indicate some of the fluorescence emitted in all directions from the fluorescently stained cells 350. The light-receiving elements 60 and 61 can be disposed at any selected positions at which they can receive the fluorescence emitted in different directions from the fluorescently stained cells 350. Note that three or more light-receiving elements may be disposed at positions at which they can receive fluorescence emitted in different directions from the fluorescently stained cells 350. Moreover, the light-receiving elements may have the same specification or may have different specifications.

If one light-receiving element is provided, there is a concern that the cell number-counting means 703 erroneously counts the number of fluorescently stained cells 350 contained in the liquid droplet 310 due to overlapping of the fluorescently stained cells 350 in a case in which a plurality of fluorescently stained cells 350 are contained in the flying liquid droplet 310.

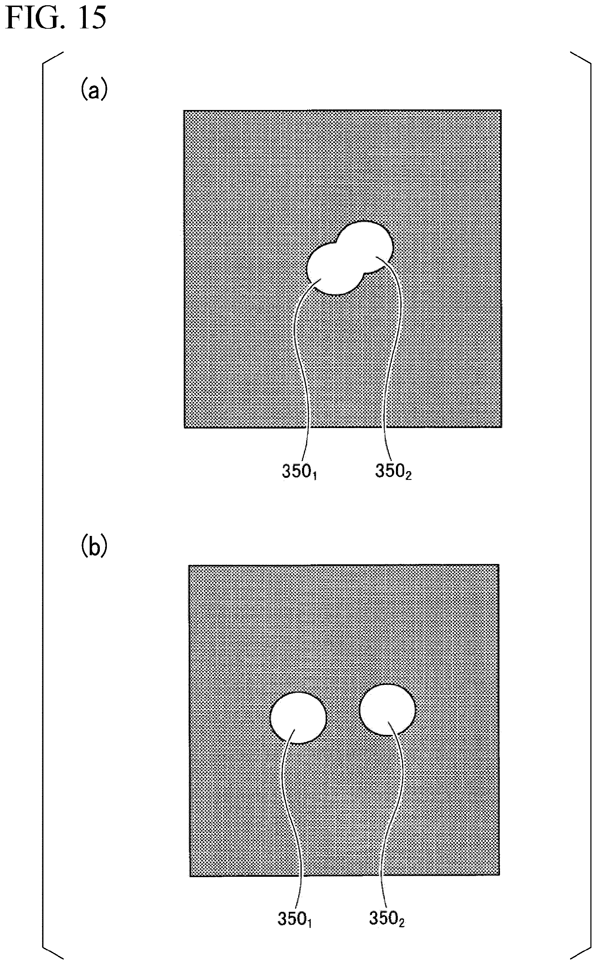

Figs. 15(a) and 15(b) are diagrams showing, as an example, a case in which two fluorescently stained cells are included in a flying liquid droplet. There may be a case in which overlapping occurs between the fluorescently stained cells 350a and 350b as shown in Fig. 15(a) and a case in which overlapping does not occur between the fluorescently stained cells 350a and 350b as shown in Fig. 15(b), for example. It is possible to reduce influences of the overlapping of the fluorescently stained cells by providing two or more light-receiving elements.

As described above, the cell number-counting means 703 can count the number of fluorescent particles by calculating a luminance value or an area value of the fluorescent particles through image processing and comparing the calculated luminance value or the area value with the preset threshold value.

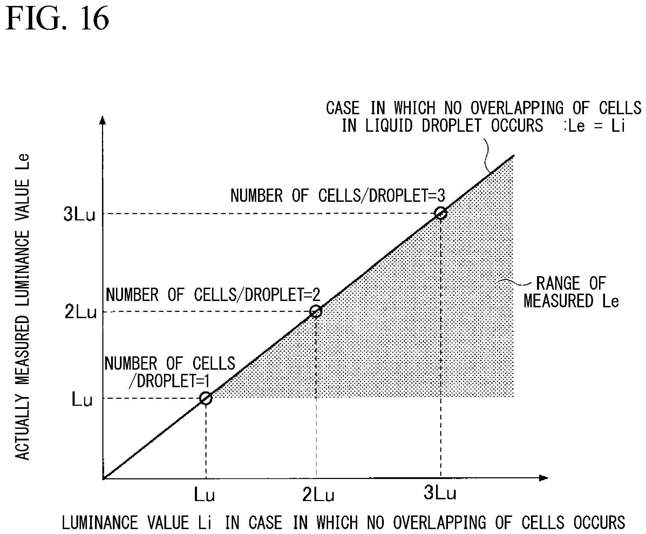

In a case in which two or more light-receiving elements are provided, it is possible to curb the occurrence of a counting error by employing data indicating the maximum value out of the luminance value or the area value obtained from each of the light-receiving elements. In this regard, a more detailed description will be given with reference to Fig. 16.

Fig. 16 is a diagram showing a relationship between a luminance value Li and an actually measured luminance value Le in a case in which no overlapping occurs between particles. As shown in Fig. 16, Le = Li in a case in which there is no overlapping between the particles in the liquid droplet. If it is assumed that the luminance value of one cell is Lu, for example, Le = Lu in a case in which the number of cells/droplet = 1, or Le = nLu (n is a natural number) in a case in which the number of cells/droplet = n.

However, since overlapping may occur between particles in a case in which n is 2 or more, in practice, the actually measured luminance value may be Lu ≤ Le ≤ nLu (the hatched part in Fig. 16). Thus, in a case in which the number of cells/droplet = n, it is possible to set the threshold value as (nLu - Lu/2) ≤ threshold value < (nLu + Lu/2), for example. Also, in a case in which a plurality of light-receiving elements are provided, it is possible to curb the occurrence of a counting error by employing data indicating the maximum value from among data obtained from each of the light-receiving elements. Note that the area value may be used instead of the luminance value.

Also, in a case in which a plurality of light-receiving elements are provided, the number of cells may be determined by an algorithm that estimates the number of cells based on a plurality of pieces of shape data. In this manner, since the liquid droplet-forming device 401B includes the plurality of light-receiving elements that receive fluorescence emitted by the fluorescently stained cells 350 in different directions, it is possible to further reduce an occurrence frequency of erroneous counting of the number of fluorescently stained cells 350.

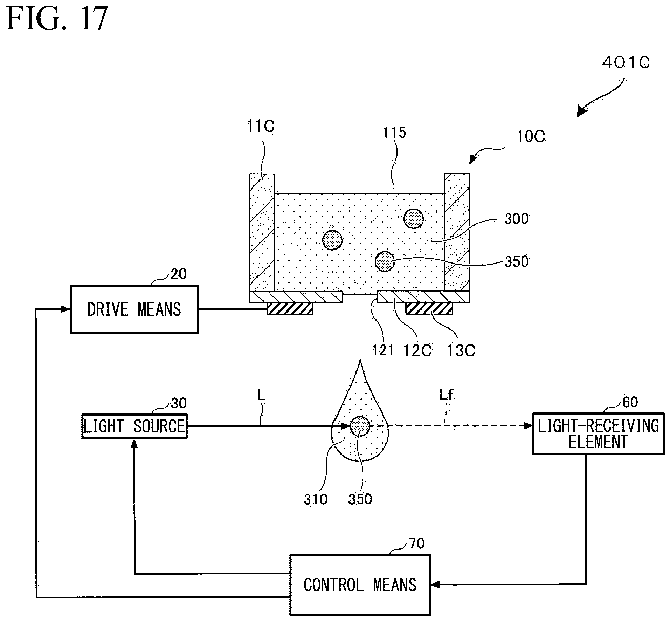

Fig. 17 is a schematic view showing another modification example of the liquid droplet-forming device 401 in Fig. 9. As shown in Fig. 17, a liquid droplet-forming device 401C is different from the liquid droplet-forming device 401 (see Fig. 9) in that the liquid droplet ejection means 10 is replaced with a liquid droplet ejection means 10C. Note that description of the same components as those in the embodiment described above may be omitted.

The liquid droplet ejection means 10C includes a liquid chamber 11C, a membrane 12C, and a drive element 13C. The liquid chamber 11C includes, at an upper portion thereof, an atmosphere opening unit 115 that opens the liquid chamber 11C to the atmosphere and is configured to be able to discharge air bubbles included in the cell suspension 300 from the atmosphere opening unit 115.

The membrane 12C is a film-shaped member fixed to a lower end portion of the liquid chamber 11C. A nozzle 121 that is a through-hole is formed at substantially the center of the membrane 12C, and the cell suspension 300 held by the liquid chamber 11C is ejected as a liquid droplet 310 from the nozzle 121 through vibration of the membrane 12C. Since the liquid droplet 310 is formed using inertia of the vibration of the membrane 12C, it is possible to eject the cell suspension 300 with high surface tension (high viscosity). The planar shape of the membrane 12C can be, for example, a circular shape and may be an oval, a quadrangular shape, or the like.

Although the material of the membrane 12C is not particularly limited, since the membrane 12C is easily vibrated, it is difficult to reduce vibration immediately when ejection is not achieved if the material is excessively soft, and a material that has hardness to some extent is thus preferably used. As a material of the membrane 12C, it is possible to use a metal material, a ceramic material, or a polymer material that has hardness to some extent, for example.

When cells are used as the fluorescently stained cells 350, a material with low adhesion against the cells and protein is preferably used. Adhesion of cells is typically believed to have dependency on a contact angle of the material with water, and adhesion of the cells decreases when hydrophilicity of the material is high or hydrophobicity thereof is high. It is possible to use various metal materials or ceramics (metal oxides) as the material with high hydrophilicity, and it is possible to use a fluororesin resin or the like as the material with high hydrophobicity.

Other examples of such a material include stainless steel, nickel, aluminum, silicon dioxide, alumina, zirconia, and the like. Additionally, it is also conceivable to reduce the cell adhesion by coating the material surface. For example, it is possible to coat the material surface with the aforementioned metal or metal oxide material or to coat the material surface with a synthetic phospholipid polymer (for example, Lipidure manufactured by NOF) simulating a cell membrane.

The nozzle 121 is preferably formed as a substantially true circular through-hole at substantially the center of the membrane 12C. In this case, although the diameter of the nozzle 121 is not particularly limited, the diameter is preferably double or more the size of the fluorescently stained cells 350 in order to avoid clogging of the fluorescently stained cells 350 in the nozzle 121. In a case in which the fluorescently stained cells 350 are, for example, animal cells, particularly, human cells, the size of the cells is typically about 5 $\mu$m to 50 $\mu$m, and the diameter of the nozzle 121 is thus preferably 10 $\mu$m or more and is more preferably 100 $\mu$m or more in accordance with the cells used.

On the other hand, if the liquid droplets become excessively large, it becomes difficult to achieve the purpose of forming the minute liquid droplets, and the diameter of the nozzle 121 is preferably 200 $\mu$m or less. In other words, the diameter of the nozzle 121 is typically within a range of 10 $\mu$m or more and 200 $\mu$m or less in the liquid droplet ejection means 10C.

The drive element 13C is formed on the lower surface side of the membrane 12C. The shape of the drive element 13C can be designed in accordance with the shape of the membrane 12C. In a case in which the planar shape of the membrane 12C is a circular shape, for example, the drive element 13C with an annular (ring) planar shape is preferably formed in the surroundings of the nozzle 121. As a drive method for the drive element 13C, a similar drive method to that for the drive element 13 can be used.

The drive means 20 can selectively (alternately, for example) apply an ejection waveform for forming the liquid droplet 310 through vibration of the membrane 12C and a stirring waveform for vibrating the membrane 12C within a range in which the liquid droplet 310 is not formed to the drive element 13C.

For example, it is possible to prevent the liquid droplet 310 from being formed through application of the stirring waveform by causing both the ejection waveform and the stirring waveform to be rectangular waves and lowering the drive voltage for the stirring waveform as compared with the drive voltage for the ejection waveform. In other words, it is possible to control a vibration state (a degree of vibration) of the membrane 12C depending on how high or low the drive voltage is.

Since the drive element 13C is formed on the lower surface side of the membrane 12C in the liquid droplet ejection means 10C, it is possible to generate a flow from the lower portion direction to the upper portion direction of the liquid chamber 11C by the drive element 13C vibrating the membrane 12.

At this time, motions of the fluorescently stained cells 350 become movement from the lower side to the upper side, convection occurs inside the liquid chamber 11C, and stirring of the cell suspension 300 containing the fluorescently stained cells 350 occurs. Due to the flow from the lower portion direction to the upper portion direction in the liquid chamber 11C, the settled and aggregated fluorescently stained cells 350 are uniformly dispersed inside the liquid chamber 11C.

In other words, the drive means 20 can cause the cell suspension 300 held in the liquid chamber 11C to be ejected as a liquid droplet 310 from the nozzle 121 by controlling the ejection waveform based on the vibration state of the membrane 12C in addition to the drive element 13C. Also, the drive means 20 can stir the cell suspension 300 held in the liquid chamber 11C by controlling the stirring waveform based on the vibration state of the membrane 12C in addition to the drive element 13C. Note that the liquid droplet 310 is not ejected from the nozzle 121 at the time of the stirring.

In this manner, it is possible to prevent the fluorescently stained cells 350 from being settled and aggregated on the membrane 12C and to cause the fluorescently stained cells 350 to be evenly dispersed in the cell suspension 300 by stirring the cell suspension 300 when the liquid droplet 310 is not formed. It is thus possible to reduce clogging in the nozzle 121 and variations in the number of fluorescently stained cells 350 in the ejected liquid droplet 310. As a result, it is possible to successively and stably eject the cell suspension 300 containing the fluorescently stained cells 350 as the liquid droplet 310.

Moreover, there may be a case in which air bubbles are included in the cell suspension 300 in the liquid chamber 11C in the liquid droplet-forming device 401C. Even in this case, the atmosphere opening unit 115 is provided at the upper portion of the liquid chamber 11C in the liquid droplet-forming device 401C, and it is thus possible to discharge the air bubbles included in the cell suspension 300 to the external air through the atmosphere opening unit 115. In this manner, it is possible to successively and stably form the liquid droplet 310 without disposing of a large amount of liquid for discharging air bubbles.

In other words, since there are influences on the ejection state in a case in which air bubbles are included in the vicinity of the nozzle 121 and in a case in which multiple air bubbles are included in the membrane 12C, it is necessary to discharge the included air bubbles in order to stably form liquid droplets for a long period of time. Although air bubbles included in the membrane 12C typically move upward naturally or through vibration of the membrane 12C, it is possible to discharge the included air bubbles from the atmosphere opening unit 115 since the atmosphere opening unit 115 is provided in the liquid chamber 11C. Therefore, it is possible to prevent non-ejection from occurring even if air bubbles are included in the liquid chamber 11C and to successively and stably form the liquid droplet 310.

Note that the membrane 12C may be vibrated within a range in which no liquid droplets are formed at the timing at which the liquid droplets are not formed, and the air bubbles may be actively moved upward in the liquid chamber 11C.

(Method for electrical or magnetic detection)

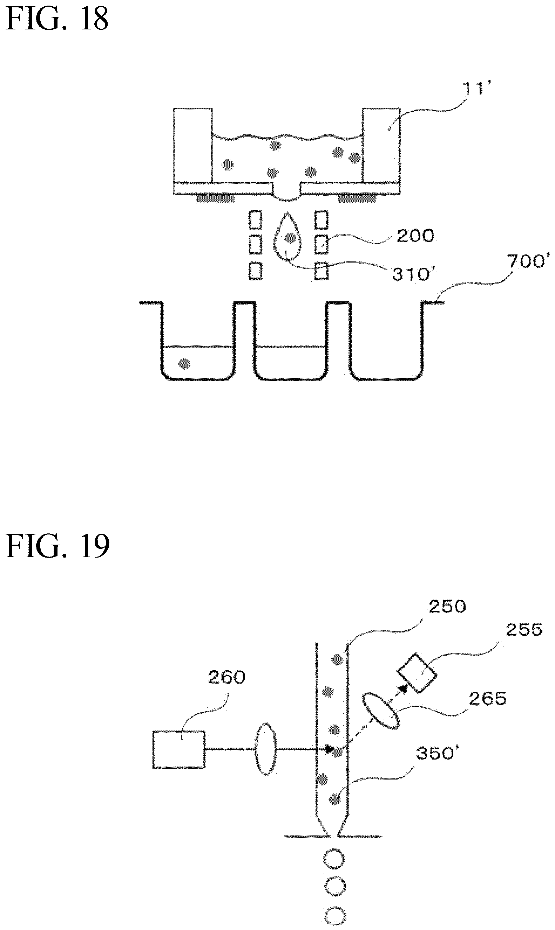

As a method for electrical or magnetic detection, a coil 200 for counting the number of cells is provided as a sensor immediately below the ejection head that ejects a cell suspension as a liquid droplet 310' from the liquid chamber 11' to the plate 700' as shown in Fig. 18. It is possible to detect the presence/absence of cells in the flying liquid droplets with an inductive current generated when the cells with magnetic beads adhering thereto pass inside the coil, by covering the cells with the magnetic beads modified with specific protein and capable of adhering to the cells. In general, the cells have protein unique to the cells on the surfaces thereof, and it is possible to cause the magnetic beads to adhere to the cells by modifying the magnetic beads with an antibody capable of adhering to the protein. It is possible to use ready-made products as such magnetic beads, and available examples include Dynabeads (registered trademark) manufactured by Veritas Corporation.

(Processing for observing cells before ejection)

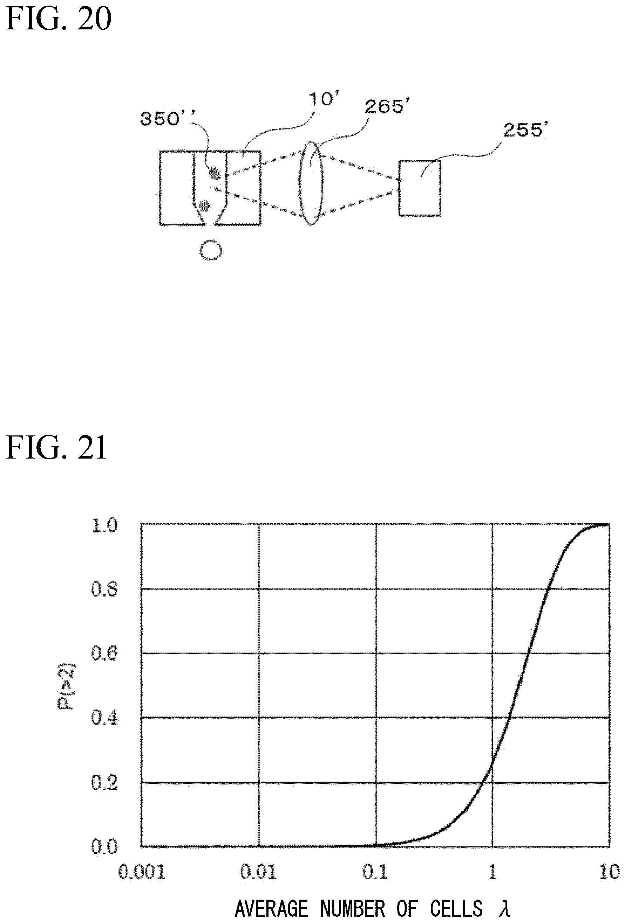

Examples of the process of observing cells before ejection include a method of counting the cells 350' that have passed through the microchannel 250 shown in Fig. 19, a method of acquiring an image in the vicinity of the nozzle unit of the ejection head shown in Fig. 20, and the like.

The method shown in Fig. 19 is a method used in a cell sorter apparatus, and for example, it is possible to use a cell sorter SH800Z manufactured by Sony Corporation. In Fig. 19, it is possible to form liquid droplet while identifying the presence/absence of cells and the type of cells by irradiating the microchannel 250 with laser light from the light source 260 and detecting scattered light or fluorescence by a detector 255 using a condenser lens 265. It is possible to predict the number of cells landing inside a predetermined well based on the number of cells that have passed through the microchannel 250 by using this method.

Also, it is possible to use a single-cell printer manufactured by Cytena as an ejection head 10' shown in Fig. 20. In Fig. 20, it is possible to estimate that cells 350" in the vicinity of the nozzle unit have been ejected as a result of acquiring an image in the vicinity of the nozzle unit by an image acquisition unit 255' via a lens 265' before ejection and it is also possible to presume the number of cells landing in a predetermined well by estimating the number of cells that are considered to have been ejected based on a difference between images before and after ejection. While liquid droplets are continuously generated in the method of counting the cells that have passed through the microchannel shown in Fig. 19, the method as shown in Fig. 20 is more preferable because it is possible to form liquid droplets on demand.

(Process of counting cells after landing)

As a process of counting the cells after landing, it is possible to employ a method of detecting fluorescently stained cells by observing the well in the plate with a fluorescent microscope or the like. This method is described in, for example, Reference Literature 1 (Moon S., et al., Drop-on-demand single cell isolation and total RNA analysis, PLoS One, Vol. 6, Issue 3, e17455, 2011).

The method of observing cells before ejection of the liquid droplet and after landing has the following problems, and observing the cells in the liquid droplet during the ejection is the most preferable depending on the type of generated plate.

In the method of observing cells before ejection, the number of cells that have passed through the channel and the number of cells that are presumed to have landed are counted from the image observation before (and after) ejection. Accordingly, it has not been confirmed whether the cells are actually ejected, and thereby an unexpected error may occur. For example, there may be a case in which the liquid droplet is not properly ejected due to contamination of the nozzle unit, the liquid droplet adheres to the nozzle plate, and the cells in the liquid droplets do not land thereon. Additionally, there may be cases in which cells remain in a narrow region in the nozzle unit, and the cells move more than expected due to the ejection operation and move out of the observation range.

There are also problems in the method of detecting cells on the plate after landing. First, preparation for enabling microscope observation of the plate is needed. Although a plate with a transparent and flat bottom surface, particularly a plate with a bottom surface made of glass is generally used as the plate that can be observed, this is a special plate, and there is a problem in that a general well cannot be used. Also, when the number of cells is larger by several tens of cells, overlapping of cells occurs, and there is thus a problem in that counting cannot accurately be performed.

Therefore, it is preferable to perform the process of observing cells before ejection and the process of counting the cells after landing in addition to counting of the number of cells contained in the liquid droplet using the sensor and the cell number-counting means after the ejection of the liquid droplet and before the landing of the liquid droplet on the well.

As the light-receiving element, it is possible to use a light-receiving element including one or a small number of light-receiving units, for example, a photodiode, an avalanche photodiode, or a photomultiplier tube, and as other examples, it is also possible to use a two-dimensional sensor such as a charge-coupled device (CCD) with light-receiving elements provided in a two-dimensional array shape, a complementary metal oxide semiconductor (CMOS), or a gate CCD.

When the light-receiving element including one or a small number of light-receiving units is used, determining, from fluorescence intensity, how many cells are in the liquid droplet using a calibration curve prepared in advance is also conceivable, and mainly the presence/absence of cells in the flying liquid droplet is detected in a binary manner. When the concentration of cells in the cell suspension is sufficiently low, and ejection is performed in a state in which only one or zero cells are in the liquid droplet, it is possible to perform the counting with sufficient accuracy through the binary detection.

On the assumption that the cells are randomly disposed in the cell suspension, the number of cells in the flying liquid droplet is considered to follow the Poisson distribution, and the probability P (> 2) that two or more cells are in the liquid droplet is represented by Equation 4 below.

$$P(>2)=1-(1+\lambda)\times e^{-\lambda} \qquad (\text{Equation 4})$$

Fig. 21 is a graph representing a relationship between a probability P (> 2) and an average number of cells. Here, $\lambda$ is an average number of cells in the liquid droplet, and this is obtained by multiplying the concentration of cells in the cell suspension by the volume of the ejected liquid droplet.

In a case in which the number of cells is counted through binary detection, the probability P (> 2) is preferably a sufficiently small value in order to secure accuracy, and $\lambda$ < 0.15, which leads to the probability P (> 2) of 1% or less being preferably satisfied. The light source is not particularly limited as long as the light source can excite the fluorescence of the cells and can be appropriately selected in accordance with purposes, and it is possible to use a typical lamp, such as a mercury lamp or a halogen lamp, filtered for irradiation with a specific wavelength, a light-emitting diode (LED), a laser, or the like. However, when a minute liquid droplet of 1 nL, in particular, is formed, it is necessary to irradiate a narrow region with high light intensity, and it is thus preferable to use a laser. As a laser light source, it is possible to multiple typically known lasers such as a solid laser, a gas laser, or a semiconductor laser. Also, the excitation light source may successively irradiate the region where the liquid droplet passes or may irradiate the region in a pulsed manner at a timing obtained by adding a predetermined time delay to the liquid droplet ejecting operation in synchronization with the ejection of the liquid droplet.

[Step of calculating certainty of the number of molecules in nucleic acid A estimated in cell suspension generation step, nucleic acid A filling step, and cell number-counting step]

This step is a step for calculating certainty in each of the steps, namely the nucleic acid A filling step and the cell number-counting step. The certainty of the estimated number of molecules in the nucleic acid A can be calculated similarly to certainty in the cell suspension generation step.

Note that as the calculation timing for certainty, the certainty may be collectively calculated in the next step of the cell number-counting step, or the certainty may be calculated at the end of each step, namely the cell suspension generation step, the nucleic acid A filling step, and the cell number-counting step, and each piece of uncertainty may be synthesized and calculated in the next step of the cell number-counting step. In other words, the certainty in each of the aforementioned steps may be appropriately calculated before the synthetic calculation.

[Output step]

The output step is a step in which the value counted by the cell number-counting means based on the detection result measured by the sensor is output as the number of cells contained in the cell suspension landing on the inside of a container. The counted value means the number of cells contained in the container by the cell number-counting means from the detection result measured by the sensor.

The output means that the value counted in response to an input from an apparatus such as a motor, a communication machine, a calculator, or the like is transmitted as electronic information to a server as an external counting result storage means or the counted value is printed as a printed article.

In the output step, the number of cells or the number of target nucleic acids in each well in the plate is observed or estimated when the plate is generated, and the observed value or the estimated value is output to an external storage unit. The output may be performed at the same time as the cell counting step or may be performed after the cell number-counting step.

[Recording step]

The recording step is a step in which the observed value or the estimated value output in the output step is recorded. The recording step can be suitably performed by a recording unit. The recording may be performed at the same time as the output step or may be performed after the output step. The recording includes not only application of information to a recording medium but also saving of information in the recording unit. In this case, the recording medium can also be referred to as a storage unit.

[Other steps]

Other steps are not particularly limited, can be appropriately selected in accordance with purposes, and examples thereof include an enzyme inactivation step and the like.

The enzyme inactivation step is a step for inactivating enzyme. Examples of enzyme include DNase, RNase, and the like. A method for inactivating enzyme is not particularly limited and can be appropriately selected in accordance with purposes, and a known method can be suitably used.

<Device for evaluating performance of genetic testing apparatus>

The device according to the present embodiment includes a plurality of reaction spaces, and the aforementioned performance evaluation sample for a genetic testing apparatus (hereinafter, also simply referred to as a "performance evaluation sample") is contained in at least some of the plurality of reaction spaces.

The device according to the present embodiment is particularly suitable for an application for evaluating performance of the genetic testing apparatus as described above. Specifically, the device according to the present embodiment can be applied to liquid biopsy using a quantitative PCR apparatus or a next generation sequencer as a genetic testing apparatus, for example.

The reaction spaces may contain reagents used in processing and pre-processing thereof performed by the genetic testing apparatus in addition to the performance evaluation sample.

Examples of the reagents include a primer, an amplification reagent, and the like. The primer is synthetic oligonucleotide having a complementary base sequence having 18 to 30 bases unique to a template DNA (the nucleic acid A and the nucleic acid B in the present embodiment) in a polymerase chain reaction (PCR) and is set at two locations (pair), namely a forward primer (sense primer) and a reverse primer (antisense primer) to sandwich the amplification target region.

Examples of the amplification reagents include, in a polymerase chain reaction (PCR), DNA polymerase as enzyme, four types of base (dGTP, dCTP, dATP, and dTTP), $Mg^{2+}$ (magnesium chloride with final concentration of about 1 mM or more and 2 mM or less), a buffer holding an optimal pH (pH 7.5 to 9.5) and the like.

A state of the performance evaluation sample in the reaction spaces and, if any, states of the primer and the amplification reagent are not particularly limited and can be appropriately selected in accordance with purposes. For example, the states may be any of a solution state or a solid state.

From the viewpoint of usability, they are particularly preferably in the solution state. If they are in the solution state, the user can immediately use them in tests. From the viewpoint of transportation, they are particularly preferably in the solid state and are further preferably in a solid dried state. If they are in the solid dried state, it is possible to reduce the decomposition speed of the reagents with decomposition enzyme or the like and to improve keeping properties of the reagents.

A drying method is not particularly limited and can be appropriately selected in accordance with purposes, and examples thereof include freeze drying, heat drying, hot wind drying, vacuum drying, steam drying, suction drying, infrared ray drying, barrel drying, spin drying, and the like.

The reaction spaces preferably contain appropriate amounts of reagents such that the reagents can be used immediately as reaction solutions by dissolving the reagents in a solid dried state in a buffer or water immediately before utilization of the device.

The device according to the present embodiment may contain the performance evaluation sample in all the plurality of reaction spaces or may contain the performance evaluation sample in some of the plurality of reaction spaces. In the latter case, the remaining reaction spaces may be empty or may contain reagents with different compositions, for example.

In the device according to the present embodiment, the form of the reaction spaces is not particularly limited, and examples thereof include wells, liquid droplets, partitions on a substrate, and the like. In a case in which the reaction spaces are wells, for example, the device according to the present embodiment may be in a form of a well plate.

[Well]

The shapes, the number, the volume, the material, the color, and the like of the wells are not particularly limited and can be appropriately selected in accordance with purposes. The shape of the wells is not particularly limited as long as the wells can contain the performance evaluation sample and, if any, the reagents and can be appropriately selected in accordance with purposes, and examples thereof include recessed portions such as a flat bottom, a round bottom, a U-shaped bottom, and a V-shaped bottom.

A plurality of wells are provided, and the number thereof is preferably five or more and is more preferably 50 or more. A multi-well plate in which the number of wells is two or more is suitably used. Examples of the multi-well plate include well plates in which the numbers of wells are 24, 48, 96, 384, 1536, and the like.

The volume of each well is not particularly limited and can be appropriately selected in accordance with purposes, and in consideration of the amount of sample used in a typical genetic testing apparatus, the volume is preferably 10 $\mu$L or more and 1000 $\mu$L or less.

The material of the wells is not particularly limited and can be appropriately selected in accordance with purposes, and examples thereof include polystyrene, polypropylene, polyethylene, a fluorine resin, an acrylic resin, polycarbonate, polyurethane, polyvinyl chloride, polyethylene terephthalate, and the like.

The color of the wells may be, for example, transparent, translucent, colored, completely shaded, or the like. Wettability of the wells is not particularly limited and can be appropriately selected in accordance with purposes, and the wells may have water repellency, for example. If the wells have water repellency, it is possible to reduce adsorption of the reagents to the well inner walls. Moreover, if the wells have water repellency, it is easy to move the reagents in the wells in the solution state.

A method of causing the well inner walls to have water repellency is not particularly limited and can be appropriately selected in accordance with purposes, and examples thereof include a method of forming a fluorine-based resin coating film, a fluorine plasma processing, embossing, and the like. It is possible to reduce a decrease in amounts of the reagents due to a failure to take a liquid, and an increase in uncertainty and variation coefficient by performing the processing of providing water repellency with which a contact angle of 100° or more is obtained.

[Base material]

The device according to the present embodiment preferably has a plate shape in which wells are provided in a base material but may be a connected-type well tube such as an eight-well tube. The material, the shape, the size, the structure, and the like of the base material are not particularly limited and can be appropriately selected in accordance with purposes.

The material of the base material is not particularly limited and can be appropriately selected in accordance with purposes, and examples thereof include a semiconductor, a ceramic, metal, glass, quartz glass, plastic, and the like. Among these, plastic is preferably used.

Examples of plastic include polystyrene, polypropylene, polyethylene, a fluorine resin, an acrylic resin, polycarbonate, polyurethane, polyvinyl chloride, polyethylene terephthalate, and the like.

The shape of the base material is not particularly limited and can be appropriately selected in accordance with purposes, and examples thereof include a sheet shape, a plate shape, and the like. The structure of the base material is not particularly limited, can be appropriately selected in accordance with purposes, and may be, for example, a single-layer structure or a multi-layer structure.

[Identification means]

The device according to the present embodiment may include an identification means capable of identifying information regarding the specific number of molecules in the nucleic acid A (for example, the number of cells) and information regarding the ratio A/B of the number of molecules in the nucleic acid A with respect to the number of the molecules in the nucleic acid B. The identification means is not particularly limited and can be appropriately selected in accordance with purposes, and examples thereof include a memory, an IC chip, a barcode, a QR code (registered trademark), a radio-frequency identifier (hereinafter, also referred to as an "RFID"), color separation, printing, and the like.
The position where the identification means is provided and the number of identification means are not particularly limited and can be appropriately selected in accordance with purposes.
Examples of the information that the identification means stores include, in addition to the information regarding the specific number of molecules in the nucleic acid A and the information regarding the ratio A/B of the number of molecules in the nucleic acid A with respect to the number of molecules in the nucleic acid B, analysis results (for example, PM values, variations in PM values, and the like), life and death of cells, which of the plurality of wells is filled with the performance evaluation sample, the type of performance evaluation sample, measurement date and time, the name of the person who performs the measurement, and the like.
The information stored in the identification means can be read using various reading means, and if the identification means is a barcode, for example, a barcode reader is used as the reading means.
A method for writing information in the identification means is not particularly limited and can be appropriately selected in accordance with purposes, and examples thereof include a manual input, a method of directly writing data from the liquid droplet-forming device that counts the specific number of molecules in the nucleic acid A when the performance evaluation sample is dispensed to each well, transfer of data saved in a server, transfer of data saved in cloud, and the like.

[Other members]

Other members are not particularly limited and can be appropriately selected in accordance with purposes, and examples thereof include a sealing member and the like.
The device according to the present embodiment preferably includes a sealing member in order to prevent foreign matter from being included in the wells, flowing-out of the filling substance, and the like. The sealing member may be configured to be able to be cut and separated along a cutting line such that at least one well can be sealed and each well can be individually sealed or opened.
Examples of the shape of the sealing member include a cap form that conforms to the diameter of the well inner wall, a film form that covers the well opening portion, and the like.
Examples of the material for the sealing member include a polyolefin resin, a polyester resin, a polystyrene resin, a polyamide resin, and the like. The sealing member is preferably in the film form in which all the wells can be sealed at once. Additionally, a configuration in which wells that require to be opened again and wells that do not require to be opened have different adhesion strengths may be employed to reduce user error.
In the device according to the present embodiment, the specific number of molecules in the nucleic acid A in one well and the specific number of molecules in the nucleic acid A in all the other wells may be the same, or may be two or more which are different from each other. In the former case, examples of the specific number of molecules include a case in which the number of molecules in all the wells is one, a case in which the number is five, a case in which the number is ten, a case in which the number is twenty, a case in which the number is forty, a case in which the number is eighty, a case in which the number is one hundred sixty, and the like. In the latter case, examples of the specific number of molecules include a case in which the number is 1, 5, 20, 40, 80, and 160, a case in which the number is 1, 2, 3, 4, 5, 6, 7, 8, 9, and 10, a case in which the number is 1, 3, 5, 7, and 9, a case in which the number is 2, 4, 6, 8, and 10, and the like. Also, some of the wells may not contain the nucleic acid A and may be used as negative controls.
In a device in which the specific number of molecules in the nucleic acid A in one well and the specific number of molecules in the nucleic acid A in all the other wells is the same, it is easy to compare evaluation results among the wells. It is thus possible to suitably use the device for evaluating performance of the genetic testing apparatus.
The device according to the present embodiment may include a group of two or more wells in which the specific number of molecules in the nucleic acid A is different. In a case in which a base material of the device is a plate including a plurality of wells, for example, each group "region" is formed by each group on the plate. Note that wells may be adjacent to each other or may be separated from each other in the "region" formed by two or more groups with a different specific number of molecules in the nucleic acid A.

In this manner, it is possible to determine whether to calibrate the genetic testing apparatus again or whether to exclude a sample in a well that is not adapted to actual samples from application targets, in a case in which wells with the same specific number of molecules at different locations are compared and there is a well that is not suitable for utilization (non-adapted well), in results obtained by performing measurement of the genetic testing apparatus using the device according to the present embodiment, for example.

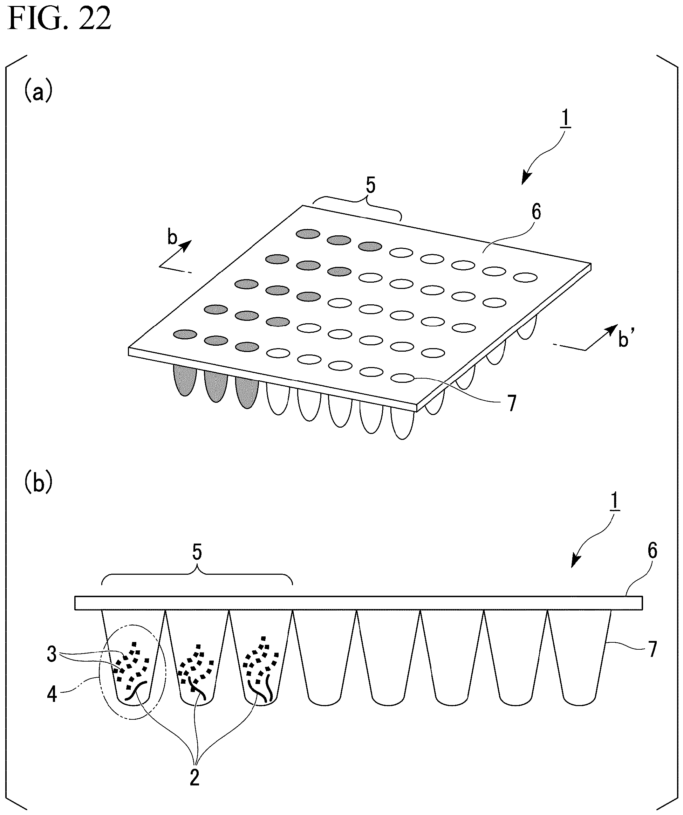

Fig. 22(a) is a perspective view showing an example of the device according to the present embodiment. Fig. 22(b) is an arrow sectional view along the line b-b' in Fig. 22(a).

A device 1 includes a base material 6 and a plurality of reaction spaces 7 formed in the base material 6, and the reaction spaces 7 are filled with the performance evaluation sample 1. The performance evaluation sample 1 contains a nucleic acid A (2) and a nucleic acid B (3) with specific numbers of molecules. In the example in Fig. 22(a) and Fig. 22(b), the reaction spaces are wells. Moreover, the opening portions of the wells may be covered with a sealing member (not shown).

Also, information regarding the specific number of molecules in the nucleic acid A (2) with which each reaction space 7 is filled, information regarding the ratio A/B of the number of molecules in the nucleic acid A (2) with respect to the number of molecules in the nucleic acid B (3), and an IC chip or a barcode (identification means) that stores other information may be disposed between the sealing member and the base material 6 and at a position other than the opening portions of the wells (not shown). It is possible to prevent unintended modification or the like of the identification means, for example, by the identification means being disposed at this position. Moreover, it is possible to distinguish the device 1 from a general well plate with no identification means by the device 1 including the identification means. It is thus possible to prevent mixing-up of the devices.

<Method for manufacturing performance evaluation device for genetic testing apparatus>

Here, a method for manufacturing a device including a plurality of reaction spaces and including a performance evaluation sample in at least some of the plurality of reaction spaces will be described.

The manufacturing method according to the present embodiment includes: a cell suspension generation step of generating a cell suspension containing a plurality of cells including nucleic acids A in the nucleus and a solvent; a liquid droplet-landing step of causing liquid droplets to sequentially land inside a well in a plate by ejecting the cell suspension as the liquid droplets; and a cell number-counting step of counting the number of cells contained in the liquid droplets with a sensor after the ejection of the liquid droplets and before the landing of the liquid droplets on the well. A step of calculating certainty of the number of molecules in the nucleic acid A estimated in the cell suspension generation step, the liquid droplet-landing step, and the cell number-counting step, a nucleic acid extraction step of extracting the nucleic acid A from the cells inside the well, an output step, and a recording step are also preferably included, and other steps are further included as needed.

The liquid droplet-landing step is the same as the "nucleic acid A filling step" in the "method for preparing the performance evaluation sample for the genetic testing apparatus" described above. Therefore, since the same steps as the above-mentioned "method for preparing a sample for performance evaluation of a genetic testing device", the cell suspension generation step, the liquid droplet-landing step, the cell number-counting step, the step of calculating certainty, the output step, and the recording step are used, description of the steps will be omitted.

[Nucleic acid extraction step]

The nucleic acid extraction step is a step of extracting the nucleic acid A from the cells in the well. Extraction means destroying cell membranes, cell walls, and the like and picking up the nucleic acid. As a method for extracting the nucleic acid A from cells, a method of performing a heat treatment at 90°C or more and 100°C or less is known. DNAs may not be extracted if the heat treatment is performed at 90°C or less, and DNAs may be decomposed if the heat treatment is performed at 100°C or more. The heat treatment is preferably performed by adding a surfactant.

The surfactant is not particularly limited and may be appropriately selected in accordance with purposes, and examples thereof include an ionic surfactant and a nonionic surfactant. Among these, one kind may be used alone, or two or more kinds may be used together. Among these, a nonionic surfactant is preferably used since it does not degenerate and inactivate protein although it depends on the amount of addition.

Examples of the ionic surfactant include fatty acid sodium, fatty acid potassium, alpha-sulfo fatty acid ester sodium, linear sodium alkylbenzene sulfonate, sulfuric acid alkyl ester sodium, alkyl ether sulfonic acid ester sodium, alpha olefin sodium sulfonate, and the like. Among these, one kind may be used alone, or two or more kinds may be used together. In particular, fatty acid sodium is preferably used, and sodium dodecyl sulfate (SDS) is more preferably used.

Examples of the nonionic surfactant include alkyl glycoside, alkyl polyoxyethylene ether (Brij series and the like), octylphenol ethoxylates (Totiton X series, Igepal CA series, Nonidet P series, Nikkol OP series, and the like), polysorbates (Tween series such as Tween 20, and the like), sorbitan fatty acid ester, polyoxyethylene fatty acid ester, alkyl maltoside, sucrose fatty acid ester, glycoside fatty acid ester, glycerin fatty acid ester, propylene glycol fatty acid ester, fatty acid monoglyceride, and the like. Among these, one kind may be used alone, or two or more kinds may be used together. In particular, polysorbates are preferably used.

The content of the surfactant is preferably 0.01 % by mass or more and 5.00% by mass or less with respect to the entire amount of cell suspension in the well. Since it is possible to achieve DNA extraction if the content is 0.01% by mass or more and it is possible to prevent inhibition of amplification in PCR if the content is 5.00% by mass or less, these ranges are suitable to achieve both effects.

In regard to cells having cell walls, DNAs may not sufficiently be extracted by the aforementioned method. Examples in that case include an osmotic pressure shock method, a freeze-thaw method, an enzyme digestion method, utilization of a DNA extraction kit, an ultrasonic processing method, a French press method, a homogenization method, and the like. Among these, the enzyme digestion method is preferably used since a loss of extracted DNAs is small.

[Other steps]

Other steps are not particularly limited and can be appropriately selected in accordance with purposes, and examples thereof include an enzyme inactivation step, a step of adding a reagent, and the like.

The enzyme inactivation step is a step for inactivating enzyme. Examples of the enzyme include DNase, RNase, the enzyme used for extracting a nucleic acid in the nucleic acid extraction step, and the like. A method for inactivating enzyme is not particularly limited and can be appropriately selected in accordance with purposes, and a known method can be suitably used.

Examples of the reagent include reagents similar to those exemplified in the "performance evaluation device for a genetic testing apparatus" described above.

<Performance evaluation method for genetic testing apparatus>

The performance evaluation method according to the present embodiment includes: a PM value information acquisition step of acquiring PM value information in a performance evaluation device for the genetic testing apparatus (hereinafter, simply referred to as a "device" in some cases) using the device; and

a performance evaluation step of evaluating performance of the genetic testing apparatus based on the PM value information, and the method further includes other steps as needed.

[PM value information acquisition step]

In the PM value information acquisition step, nucleic acid sequence analysis of the performance evaluation sample is performed using a genetic testing apparatus that is an evaluation target, and PM value information is acquired. Processing in the nucleic acid sequence analysis can be appropriately selected in accordance with the type of genetic testing apparatus. Specifically, the nucleic acids in the performance evaluation sample are fragmented, the fragmented nucleic acids are collected (pre-processing), and the sequence of the collected nucleic acids is read (sequencing), for example.

The sequencing primer used for sequencing is not particularly limited and is appropriately set based on a sequence suitable for amplifying the target region. As for a reagent used for the sequencing, a suitable reagent may be selected in accordance with the types of sequencing technique used and the sequencer.

(Pre-processing)

Specifically, the nucleic acid A and the nucleic acid B in the performance evaluation sample are fragmented into lengths for reading the sequence with the sequencer in the pre-processing. Fragmentation of the sample DNA can be performed by a known method such as ultrasonic processing or processing with a reagent for fragmenting the nucleic acids, for example. The obtained DNA fragments (nucleic acid fragments) can have lengths ranging from several tens to several hundreds of bp, for example.

Alternatively, in another example of pre-processing, fragmentation is not performed in a case of a target sequence using a PCR reaction, and the performance evaluation sample is subjected to a PCR reaction using a primer designed to amplify target sequences (the nucleic acid A and the nucleic acid B). The obtained PCR products may have lengths ranging from several tens to several hundreds of bp. At this time, the following adaptor-adding step can be performed together by performing tailed PCR in which an adaptor sequence is added to the primer.

Next, the adaptor sequence corresponding to the type of sequencer or a sequencing protocol used is added to both ends of the obtained DNA fragment (a 3' terminal and a 5' terminal). However, although the step is an essential step in a case of using a sequencer manufactured by Ilumina, Inc. or an apparatus that employs a similar method to that of the sequencer manufactured by Iluimina, Inc., this may also be omitted in a case in which another type of sequencer is used.

The adaptor sequence is a sequence used to execute sequencing in the later step. In an embodiment, the adaptor sequence may be a sequence for hybridization into an oligo DNA fixed to a bead to be subjected to emulsion PCR. In another embodiment, the adaptor sequence may be a sequence for hybridization into an oligo DNA fixed to a flow cell to be subjected to bridge PCR.

The adaptor sequence may be added directly to both ends of the DNA fragment. For the addition of the adaptor sequence to the DNA fragment, it is possible to use a method known in the field. For example, the DNA sequence may be smoothed to ligate the adaptor sequence.

(Sequencing)

As a sequencing technique, it is possible to appropriately select and use a known method, and specific examples thereof include the same sequencing techniques exemplified above in the "performance evaluation sample for a genetic testing apparatus".

In an embodiment, a sequencing method based on emulsion PCR and ion semiconductor sequencing will be described below.

First, a DNA fragment with the adaptor sequence added thereto and a bead to which a short oligo DNA that is complementary to the adaptor sequence is linked are mixed to link them at 1:1, and a micro-reactor encapsulating one bead and only one DNA fragment in oil is formed by encapsulating the mixture along with the amplification reagent in the water droplet-in-oil emulsion. In this manner, each DNA fragment is amplified to several million copies on the bead without other sequences being mixed therein.

Next, the emulsion is destroyed to condense the bead, the bead is placed inside a micro-well on a semiconductor sequencing chip, and sequence analysis is then performed. On the micro-well, an extension reaction with DNA polymerase is performed from a primer that is complementary to the adaptor. At this time, hydrogen ions are released in a process in which nucleotide is taken into the DNA with polymerase, and a detectable change in pH is thus shown. In the micro-well on the semiconductor sequencing chip, about a million copies per well of the DNA molecule are placed. New nucleotide is added to the chip one after another with an ion semiconductor sequencer (for example, an Ion Personal Genome Machine (PGM, registered trademark) sequencer or the like). In a case in which the added nucleotide has a sequence that is complementary to the sequence in the DNA in the micro-well, the nucleotide is taken into the DNA, and hydrogen ions are released. As a result, pH in the well changes, and an ion sensor detects the change. The thus detected chemical information is directly converted into digital information. If the two same bases are successively present in the DNA chain, the electrical charge doubles, and the chip detects two bases. Next, in a case in which the added nucleotide does not conform to a template, no change in electrical charge is recorded, and no bases are read. In this manner, the ion semiconductor sequencing system directly detects bases and thus does not require a scanner, a camera, a light source, and the like, and the time required for run to record the taking of each base in several seconds is significantly shortened.

In another embodiment, a sequencing method based on emulsion PCR and pyrosequencing will be described below.

As a procedure for emulsion PCR, a procedure similar to that in "sequencing method by emulsion PCR and ion semiconductor sequencing" described above is performed, emulsion is then destroyed to concentrate the beads, and sequence analysis is performed with the beads placed on a picotiter plate with a large number of holes, in each of which one bead can be contained. On the plate, an extension reaction using DNA polymerase is performed from a primer that is complementary to the adaptor. At this time, dNTPs are replaced one by one as a material for the extension for a reaction achieved by adding only dATP and a reaction achieved by adding only dGTP. Since a pyrophosphate is isolated if an extension reaction occurs, it is possible to perform detection based on a light-emitting reaction caused by luciferase, and sequence information of the nucleic acid can thus be obtained.

A sequencing method by bridge PCR and sequencing-by-synthesis in another embodiment will be described below.

First, a sequence of a DNA fragment is applied to a flow cell. Next, the DNA fragment that is an analysis target is amplified by the bridge PCR method on the flow cell. In other words, the DNA fragment that is an analysis target is subjected to the aforementioned pre-processing to immobilize two different types of adaptor sequences to both terminals, cause the DNA fragment to have a single strand, and immobilize the adaptor sequence on the 5' terminal side to the flow cell.

The adaptor sequence on the 5' terminal side is immobilized on the flow cell in advance, a bridged state is achieved, and a bridge is formed by the adaptor sequence on the 3' terminal side of the DNA fragment being linked to the adaptor sequence on the 5' terminal side on the flow cell.

A DNA extension reaction is performed with DNA polymerase in this state to cause degeneration, thereby obtaining two single-stranded DNA fragments.

It is possible to form a cluster with amplification of multiple single-stranded DNA fragments locally immobilized through repetition of such bridge formation, a DNA extension reaction, and the degeneration in this order.

Then, the single-stranded DNAs forming the cluster are used as a template, and the sequence is read by sequencing-by-synthesis.

Specifically, DNA polymerase and dNTP that is fluorescently labeled and is blocked on the 3' terminal side are added to the single-stranded DNA immobilized on the flow cell first, and a sequence primer is further added.

It is only necessary for the sequence primer to be designed to be hybridized with part of the adaptor sequence, for example. In other words, it is only necessary for the sequence primer to be designed to amplify a DNA fragment derived from a sample DNA, and in a case in which an index sequence is added, it is only necessary for the sequence primer to be further designed to amplify the index sequence.

After addition of the sequence primer, a one-base extension reaction of 3' terminal block fluorescence dNTP is performed with DNA polymerase. Since the 3' terminal side uses blocked dNTP, the polymerase reaction is stopped once extension corresponding to one base is achieved. Then, the DNA polymerase is removed, the fluorescent substance linked to the base is excited with laser light for the single-stranded DNA extended by one base, and light emission caused at that time is recorded as photographs.

In the photographs, fluorescent colors corresponding to A, C, G, and T are imaged with wavelength filters changed in order to determine four types of bases, using a fluorescent microscope. After all the photographs are taken, the base is determined from the photograph data. Then, the fluorescent substance and a protecting group blocking the 3' terminal side are removed, and the processing proceeds to the next polymerase reaction. It is possible to perform sequencing on the entire length by assuming this flow as one cycle and repeating this in the second cycle, the third cycle, and the like.

Read sequence information obtained through the sequencing is obtained. The read sequence information is data indicating the base sequence read by the sequencer. The read sequence information may include a quality score of each base in the sequence along with the read sequence.

[Performance evaluation step]

In the performance evaluation step, nucleic acid sequence analysis is evaluated. The evaluation of the nucleic acid sequence analysis is preferably performed based on values such as PM values (also referred to as PM scores). As will be described later in the examples, the inventor has discovered that the variations in PM values are particularly useful as indicators for accurately evaluating performance of the genetic testing apparatus.

The PM values are calculated from the read sequence information, are Plasma Mutation scores, and are defined by the read count of each mutation per 100,000 reads. A small PM value indicates a small amount of nucleic acid A, and a large PM value indicates a large amount of nucleic acid A. In the performance evaluation method according to the present embodiment, the variations in PM values mean variations among PM values obtained in each reaction space in a case in which nucleic acid sequence analysis is performed under the same conditions in the plurality of reaction spaces. Smaller variations in PM values mean higher performance of the genetic testing apparatus. Moreover, small variations mean more satisfactory accuracy of analysis in the genetic testing apparatus. On the other hand, large variations in PM values mean that the analysis was not performed under optimal pre-processing conditions and sequence conditions.

<Performance evaluation program and performance evaluation apparatus for genetic testing apparatus>

The performance evaluation program according to the present embodiment includes: a PM value information acquisition step of acquiring PM value information in the device using the aforementioned device; and

a performance evaluation step of evaluating performance of the genetic testing apparatus based on the PM value information, and the performance evaluation program further includes other steps as needed.

The performance evaluation apparatus according to the present embodiment includes: a PM value information acquisition unit that acquires PM value information in the device using the aforementioned device; and

a performance evaluation unit that evaluates performance of the genetic testing apparatus based on the PM value information, and the performance evaluation apparatus further includes other components as needed.

Since control performed by the control unit and the like in the performance evaluation apparatus according to the present embodiment has the same meaning as execution of the aforementioned performance evaluation method for the genetic testing apparatus, details of the performance evaluation method for the genetic testing apparatus will also be clarified through the description of the performance evaluation apparatus according to the present embodiment.

Also, since the performance evaluation program according to the present embodiment is realized by the performance evaluation apparatus for the genetic testing apparatus using a computer or the like as a hardware resource, details of the performance evaluation program according to the present embodiment will also be clarified through the description of the performance evaluation apparatus according to the present embodiment.

[PM value information acquisition step and information acquisition unit]

The step of acquiring the PM value information is the aforementioned step of acquiring the PM value information using the device (PM value information acquisition step) and is performed by an information acquisition unit. The PM value information can be obtained by the genetic testing apparatus performing analysis (sequencing) of a nucleic acid sequence using the aforementioned device.

Examples of the PM value information include PM values, variations in PM values, and the like. Among these pieces of information, one kind may be used alone, or two or more kinds may be used for the evaluation in combination. The variations in PM values are similar to those described above. Examples of the variations in PM values include a standard deviation, a CV value, and the like.

(Evaluation step and evaluation unit)

An evaluation step is a step for evaluating performance of the genetic testing apparatus based on PM value information and is performed by an evaluation unit.

For example, in qualitative evaluation, the aforementioned device may be used to perform analysis (sequencing) of a nucleic acid sequence in the genetic testing apparatus, measure PM values, and calculate an average PM value. It is possible to evaluate in-plane characteristics on the assumption that the PM value in each well within 10% of the average PM value is represented as "A" and the PM value in each well over 10% of the average PM value is represented as "B".

Additionally, it is possible to obtain a temporal change in information regarding analysis of the nucleic acid sequence by performing measurement for a specific period of time using the device according to the present embodiment. In this manner, in a case in which the PM value in each well exceeds 10% of the average PM value, for example, it is possible to calibrate the genetic testing apparatus or not to use the measurement location similarly to the in-plane characteristics. Also, since the specific number of molecules disposed is an absolute value, it is possible to compare performance among genetic testing apparatuses by using devices in which the same specific number of molecules are disposed.

In quantitative evaluation, it is possible to obtain temporal changes in information regarding analysis of the nucleic acid sequence by performing measurement for a specific period of time using the device according to the present embodiment. In this manner, in a case in which a numerical value that deviates from a quality management value is obtained, it is possible to calibrate the genetic testing apparatus or not to use the measurement location similarly to the in-plane characteristics. Also, since the number of molecules disposed is an absolute value, it is possible to compare performance among genetic testing apparatuses by using the devices in which molecules of the same number are disposed.

(Other steps and other parts)

Other steps and other parts are not particularly limited and can be appropriately selected in accordance with purposes, and examples thereof include a display step, a display unit, and the like.

Processing based on the performance evaluation program according to the present embodiment can be executed using a computer including a control unit constituting the performance evaluation apparatus. Hereinafter, a hardware configuration and a functional configuration of the performance evaluation apparatus will be described.

(Hardware configuration of performance evaluation apparatus)

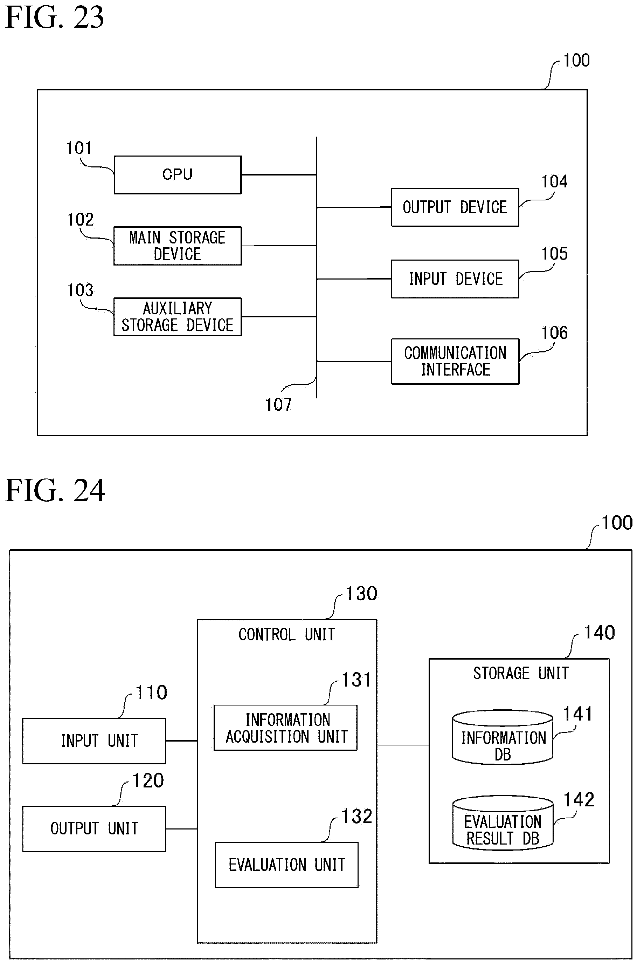

Fig. 23 is a block diagram showing an example of a hardware configuration of the performance-evaluating apparatus 100 for a genetic testing apparatus. As shown in Fig. 23, the performance-evaluating apparatus 100 includes a central processing unit (CPU) 101, a main storage device 102, an auxiliary storage device 103, an output device 104, an input device 105, and a communication interface (communication I/F) 106. These components are connected to each other via a bus 107.

The CPU 101 is a processing device that performs various kinds of control and arithmetic operations. The CPU 101 realizes various functions by executing an operating system (OS) and a program stored in the main storage device 102 and the like. In other words, the CPU 101 functions as a control unit 130 of the performance-evaluating apparatus 100 for a genetic testing apparatus by executing a performance evaluation program for a genetic testing apparatus.

Also, the CPU 101 controls overall operations of the performance-evaluating apparatus 100 for a genetic testing apparatus. Note that although the device that controls the overall operations of the performance-evaluating apparatus 100 is assumed to be the CPU 101 herein, the present invention is not limited thereto, and a field-programmable gate array (FPGA), for example, may control the overall operations.

The performance evaluation program for a genetic testing apparatus and various databases may not necessarily be stored in the main storage device 102, the auxiliary storage device 103, and the like. Other information-processing apparatuses or the like connected to the performance-evaluating apparatus 100 for a genetic testing apparatus via the Internet, a local area network (LAN), a wide area network (WAN), or the like may store the performance evaluation program for a genetic testing apparatus and the various databases. The performance-evaluating apparatus 100 for a genetic testing apparatus may acquire the performance evaluation program for a genetic testing apparatus and various databases from these other information-processing apparatuses and execute the program and the databases.

The main storage device 102 stores various programs and stores data and the like necessary to execute the various programs. The main storage device 102 includes a read-only memory (ROM) and a random-access memory (RAM), which are not shown.

The ROM stores various programs such as a basic input/output system (BIOS). The RAM functions as a work range developed when the various programs stored in the ROM are executed by the CPU 101. The RAM is not particularly limited and can be appropriately selected in accordance with purposes. Examples of the RAM include a dynamic random-access memory (DRAM), a static random-access memory (SRAM), and the like.

The auxiliary storage device 103 is not particularly limited as long as it can store various kinds of information and can be appropriately selected in accordance with purposes, and examples thereof include a solid-state drive, a hard disk drive, and the like. Moreover, the auxiliary storage device 103 may be a portable storage device such as a compact disc (CD) drive, a digital versatile disc (DVD) drive, or a Blu-ray (registered trademark) disc (BD), for example.

As the output device 104, it is possible to use a display, a speaker, and the like. The display is not particularly limited, a known display can be appropriately used, and examples thereof include a liquid crystal display and an organic EL display.

The input device 105 is not particularly limited as long as it is possible to receive various requests for the performance-evaluating apparatus 100 for the testing apparatus, a known input device can be appropriately used, and examples thereof include a keyboard, a mouse, a touch panel, and the like.

The communication interface (communication I/F) 106 is not particularly limited, a known communication interface can be appropriately used, and examples thereof include a wireless or wired communication device and the like.

The hardware configuration as described above can realize processing functions of the performance-evaluating apparatus 100 for a genetic testing apparatus.

(Functional configuration of performance-evaluating apparatus)

Fig. 24 is a diagram showing an example of a functional configuration of the performance-evaluating apparatus 100 for a genetic testing apparatus. As shown in Fig. 24, the performance-evaluating apparatus 100 includes an input unit 110, an output unit 120, a control unit 130, and a storage unit 140.

The control unit 130 includes an information acquisition unit 131 and an evaluation unit 132. The control unit 130 controls the entire performance-evaluating apparatus 100. The storage unit 140 includes an information database 141 and an evaluation result database 142. Hereinafter, the "database" may be referred to as a "DB". The information acquisition unit 131 uses the data stored in the information DB 141 of the storage unit 140 to acquire information regarding analysis of a nucleic acid sequence. The information DB 141 stores data such as a PM value obtained in advance by an experiment as described above, for example.

Note that information linked to the device may be stored in the information DB 141. An input to the DB may be performed by another information-processing apparatus connected to the performance-evaluating apparatus 100 or may be performed by an operator.

The evaluation unit 132 evaluates performance of a genetic testing apparatus based on information regarding analysis of a nucleic acid sequence. A specific method for evaluating the performance of the genetic testing apparatus is as described above. The result of the evaluation on the performance of the genetic testing apparatus obtained by the evaluation unit 132 is stored in the evaluation result DB 142 in the storage unit 140.

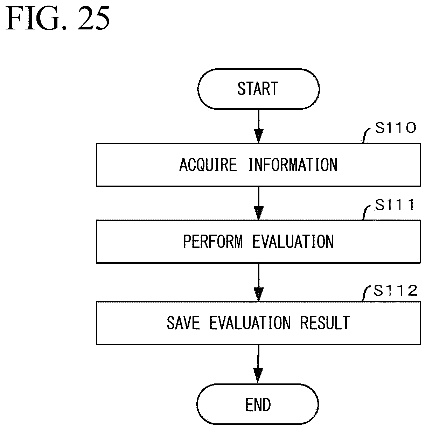

Next, a processing procedure of the performance evaluation program according to the present embodiment will be described. Fig. 25 is a flowchart showing a processing procedure of the performance evaluation program in the control unit 130 in the performance-evaluating apparatus 100 for a genetic testing apparatus.

In Step S110, the information acquisition unit 131 of the control unit 130 in the performance-evaluating apparatus 100 acquires the information data regarding analysis of a nucleic acid sequence stored in the information DB 141 in the storage unit 140 and shifts to the processing in S111.

In Step Sill, the evaluation unit 132 of the control unit 130 in the performance-evaluating apparatus 100 evaluates the performance of the genetic testing apparatus based on the acquired information and shifts to the processing in S112.

In Step S112, the control unit 130 in the performance evaluation apparatus 100 saves the obtained result of evaluating the performance of the genetic testing apparatus in the evaluation result DB 142 of the storage unit 140 and ends the processing.

EXAMPLES

Although the present invention will be described based on examples below, the present invention is not limited to the following examples.

[Example 1]

(Filling with low number of copies of nucleic acid and evaluation of filling accuracy)

1. Preparation of low number of copies of nucleic acids

(1) Design of artificial nucleic acid

An artificial nucleic acid for incorporation into yeast was produced in (2) described later. The artificial nucleic acid was designed to have a homologous sequence the same as those in regions of exons 18, 19, 20, and 21 of an EGFR gene and to be able to be amplified with the same primer at the time of mutation analysis for the EGFR gene. A restriction enzyme site was inserted into exon sequences. The artificial nucleic acid contained five types of mutations as targets of detection. The five incorporated mutations were e19_deletion, T790M, C797S, L858R, and L861Q, which are well-known mutations of the EGFR gene. The produced artificial nucleic acid included a base sequence represented by a sequence number 1.

(2) Production of yeast suspension for low number of copies of nucleic acid series

(2-1) Preparation of recombinant yeast

A budding yeast YIL015W/BY4741 (manufactured by ATCC; ATCC4001408) was used as a carrier cell of one copy of a specific nucleic acid sequence thereby to produce a recombinant. As a specific nucleic acid sequence, plasmid into which the artificial nucleic acid designed in above (1) and a URA 3 gene as a selectable marker were introduced in a tandem manner was created in advance. One copy of artificial nucleic acid was introduced into the Bar1 gene region of a genomic DNA of a carrier cell through homologous recombination using the plasmid thereby to produce a recombinant yeast. One type of recombinant having a mutant EGFR gene was produced.

(2-2) Culture of recombinant yeast and control of cell cycle

In a triangular flask containing 90 mL of recombinant yeast cultured in 50 g/L YPD medium (manufactured by Takara Bio Co., Ltd.; CLN-630409), 900 $\mu$L of Dulbecco's phosphate buffered saline (manufactured by Thermo Fisher Scientific Co., Ltd.; 14190-144; hereinafter may also be referred to as "DPBS") containing 500 $\mu$g/mL $\alpha$1-Mating Factor acetate salt (manufactured by Sigma-Aldrich; T6901-5MG, hereinafter referred to as "a-factor" in some cases) was added, and incubation was performed using a bioshaker (manufactured by Taitec Co., Ltd.; BR-23FH) at a shaking rate of 250 rpm and a temperature of 28°C for 2 hours, and yeast suspension in which yeast was in the G0/G1 phase was thereby obtained.

(2-3) Immobilization of recombinant yeast

45 mL of yeast suspension confirmed to be in the G0/G1 phase was transferred to a centrifuge tube (manufactured by Azuwan Co., Ltd.; VIO-50R), the mixture was centrifuged using a centrifuge (manufactured by Hitachi, Ltd.; F16RN) at a rotation speed of 3000 rpm for 5 minutes, and the supernatant was removed therefrom to obtain yeast pellets. 4 mL of formalin (manufactured by Wako Pure Chemical Industries, Ltd.; 062-01661) was added to the obtained yeast pellets, and the mixture was allowed to stand for 5 minutes and centrifuged to remove the supernatant liquid, thereby obtaining yeast pellets. Then, 10 mL of ethanol was added to the obtained yeast pellets and suspended to thereby obtain an immobilized yeast suspension.

(2-4) Nuclear staining of recombinant yeast

200 μL of the immobilized yeast suspension was dispensed, washed once with DPBS, and resuspended in 480 μL DPBS. After adding 20 μL of 20 mg/mL RNase A (manufactured by Nippon Gene Co. Ltd.;318-06391), the mixture was incubated at 37°C for 2 hours using a bioshaker. Then, 25 μL of 20 mg/mL of proteinase K (manufactured by Takara Bio Inc.; TKR-9034) was added thereto, and the mixture was incubated at 50°C for 2 hours using Petit Cool (manufactured by Wakenbee Tech Co., Ltd.; Petit Cool MiniT-C). Finally, 6 μL of 5 mM SYTOX Green Nucleic Acid Stain (manufactured by Thermo Fisher Scientific Co., Ltd.; S7020) was added thereto to stain the mixture for 30 minutes under shade.

(2-5) Dispersion of recombinant yeast

The yeast suspension subjected to nuclear staining was dispersed at an output of 30% for 10 seconds using an ultrasonic homogenizer (manufactured by Yamato Scientific Co., Ltd.; LUH150) to obtain a yeast suspension ink.

2. Filling with low number of copies of nucleic acids

(1) Counting number of yeast suspensions and dispensing yeast suspension

The number of yeast fungi in the liquid droplet was counted, one cell was ejected to each well, and a plate with a known number of cells was produced by the following method. Specifically, a liquid droplet-forming device (manufactured by Ricoh Japan Corporation) was used to sequentially eject yeast suspension ink at 10 Hz to each well in a 96-well plate ("MicroAmp 96-well Reaction plate" (product name) manufactured by Thermo Fisher Scientific Co., Ltd.) using an ejection head (manufactured by Ricoh Japan Corporation) based on a piezoelectric application method as a liquid droplet ejection means.

A high-sensitivity camera (manufactured by Tokyo Instruments, Inc.; sCMOS pco.edge) was used as a light-receiving means for imaging the yeast in the ejected liquid droplet. A YAG laser (manufactured by Spectra Physics, Inc.; Explorer ONE-532-200-KE) was used as a light source, image processing was performed using Image J image-processing software as particle-counting means for the captured image to count the number of cells, and a known plate with one cell was produced.

(2) Nucleic acid extraction

1 mg/mL of zymolyase (R) 100T (manufactured by NACALAI TESQUE, INC.; 07665-55) was added to Tris-EDTA (TE) Buffer (hereinafter, referred to as "ColE1/TE" in some cases) containing 5 ng/μL of ColE1 DNA (manufactured by Wako Pure Chemical Industries; 312-00434), thereby preparing a zymolyase solution.

4 μL of zymolyase solution was added to each well of the produced cell number known plate, incubation was performed at 37.2°C for 30 minutes to dissolve the cell walls (extract the nucleic acids), and heat treatment was then performed thereon at 95°C for 2 minutes, thereby producing a reference device.

(3) Calculation of uncertainty

Next, in order to consider reliability of results obtained from the known plate with one cell (reference device), uncertainty of the one cell was calculated. Note that it is possible to calculate uncertainty for various numbers of copies by using the method described below for each specific number of copies.

As the number of cells in the liquid droplet, the number of cells in the liquid droplet counted through image analysis of the liquid droplet ejected by the ejection means and the number of cells obtained by causing the liquid droplet ejected by the ejection means to land on a slide glass and observing each landing liquid droplet with a microscope were used.

As the number of copies of the nucleic acid (cell cycle) in the cells, a proportion of cells corresponding to the G1 phase of the cell cycle (99.5%) and a proportion of cells corresponding to the G2 phase (0.5%) were used for the calculation.

Although the number of ejected liquid droplets landing inside the well was counted as the number of cells in the well, all the liquid droplets landed inside the well in the counting of the 96 samples, and a factor of the number of cells in the well was excluded from calculation of uncertainty.

In regard to contamination, trials were performed three times to check whether nucleic acids other than the nucleic acids introduced into the nucleus of the cell in the real-time PCR of 4 μL ink filtrate had not been included in the ink liquid. As a result, detection lower limit values were obtained in all three trials, and a factor of contamination was thus also excluded from the calculation of uncertainty.

As uncertainty, synthetic standard uncertainty was obtained by a square sum method from standard uncertainty (hereinafter, also referred to as "standard uncertainty (units of measurement amounts)") acquired by obtaining a standard deviation from measured values of each factor and multiplying a sensitivity coefficient to unify the unit of measurement amounts. Since the synthetic standard uncertainty includes only values within a range of about 68% of normal distribution, it is possible to obtain uncertainty in consideration of about 95% of normal distribution by employing extended uncertainty by doubling the synthetic standard uncertainty. The results are shown in Table 2.

[Table 2]

| Symbol | Factor of uncertainty | Value (±) | Probability distribution | Divisor | Standard uncertainty | Sensitivity coefficient | Standard uncertainty (unit of measured amount) |
|---|---|---|---|---|---|---|---|
| U1 | Number of cells in liquid droplet | 0.1037 cells | - | 1 | 0.1037 cells | 1.0290 copies/cell | 0.1067 copies |
| U2 | Number of molecules in nucleic acid in cell (cell cycle) | 0.0709 copies | - | 1 | 0.0709 copies | - | 0.0709 copies |
| U3 | Number of cells in well | - | - | - | - | - | - |
| U4 | Contamination | - | - | - | - | - | - |
| UC | Synthetic standard uncertainty | | Normal distribution | | | | 0.1281 copies |
| U | Extended uncertainty | | Normal distribution | | | | 0.2562 copies |

In Table 2, "symbol" means any selected symbol associated with a factor of uncertainty. "Value (±) is an experimental standard deviation of an average value and is obtained by dividing the calculated experimental standard deviation by a square value of the number of data. "Probability distribution" is probability distribution that each factor of uncertainty has, the section is left blank in a case of A-type uncertainty evaluation, and either normal distribution or rectangular distribution is input for B-type uncertainty evaluation. "Divisor" means a number that normalizes uncertainty obtained from each factor. "Standard uncertainty" is a value obtained by dividing "value (±)" by "divisor". "Sensitivity coefficient" means a value used to unify units into units of measurement amounts.

Next, an average specific number of copies and uncertainty of the nucleic acid samples with which the wells were filled were calculated. The results are shown in Table 3. The variation coefficient CV value was calculated by dividing the value of uncertainty by the average specific number of copies.

[Table 3]

| Specific number of copies | | Variation coefficient CV value |
|---|---|---|
| Average | Uncertainty | |
| Copy | Copy | % |
| 1.02E+00 | 1.28E-01 | 12.6 |
| 2.03E+00 | 1.81E-01 | 8.91 |
| 4.07E+00 | 2.56E-01 | 6.3 |
| 8.13E+00 | 3.62E-01 | 4.46 |
| 1.63E+01 | 5.12E-01 | 3.15 |
| 2.13E+01 | 5.87E-01 | 2.75 |

(continued)

| Specific number of copies | | Variation coefficient CV value |
|---|---|---|
| Average | Uncertainty | |
| 6.50E+01 | 1.02E+00 | 1.58 |
| 1.30E+02 | 1.45E+00 | 1.11 |

It was confirmed that accuracy of dispensing the nucleic acid (one yeast) with a specific number of copies of 1, that is, 1 copy into a well was ±0.1281 copies. In a case in which the well was filled with one or more copies, the accuracy at which filling with the specific number of copies of nucleic acid was achieved was considered to be determined by accumulation of the accuracy.

Based on the aforementioned results, it is possible for a user in an experiment to use the indicator of uncertainty as a determination criterion for reliability of a measurement result for each well by causing the obtained extended uncertainty to be stored as an indicator of variations in measurement and as data of the device. Moreover, it is possible to highly accurately perform performance evaluation for analytical testing by using the aforementioned determination criterion for reliability.

(4) Uncertainty value application to well

Then, the value of the uncertainty (or the variation coefficient) calculated as in Table 2 was applied to each well.

In this manner, it was possible to calculate the average nucleic acid count value of the low-concentration nucleic acid series, the uncertainty thereof, and the variation coefficient and to apply the values to each well.

[Example 2]

(Mutation analysis of EGFR gene)

1. Preparation of samples with ultra-low allele frequencies

The sample filling well was filled with one copy of yeast containing the artificial nucleic acid (mutant EGFR gene) designed in Example 1 using a similar procedure to that in Example 1 so as to have a prescribed number of copies. The prescribed numbers of copies were 53 copies for the wells used for mutation analysis of e19_deletion, 64 copies each for the wells used for mutation analysis of T790M and C797S, and 7 copies each for the wells used for mutation analysis of L858R and L861Q.

Next, each sample filling well filled with yeast was filled with 4.0 $\mu$L (7.5 ng/$\mu$L) of Human genomic DNA (manufactured by Promega). This filling amount corresponds to the amount by which about ten thousand copies of normal EGFR genes are contained. After the filling, mixing was performed to prepare samples with ultra-low allele frequencies of 0.07%, 0.53%, and 0.64% in the sample filling wells. In addition, samples added to the wells by manually diluting the yeast genomic DNA into which the artificial nucleic acid (mutant EGFR gene) designed in Example 1 was introduced so as to have each number of copies (7, 53, and 64 copies) were prepared as controls. Human genomic DNA (manufactured by Promega) was added thereto in a manner similar to that described above to prepare manually diluted samples (control group). Note that the yeast genomic DNA used in the control group was extracted using Gen Toru Kun (registered trademark) for yeast High Recovery (manufactured by Takara Biomedical Inc.)

2. Nucleic acid amplification by PCR method

Next, samples with ultra-low allele frequency and manually diluted samples prepared in "1." were subjected to nucleic acid extraction using a method similar to that in "1.". Next, each of the extracted nucleic acids was subjected to an amplification reaction by the PCR method in the sample filling well. The composition of the reaction solution was a total of 50 $\mu$L including 25.0 $\mu$L of nuclease-free water, 5.0 $\mu$L of 10 $\times$ KOD-Plus-Buffer, 5.0 $\mu$L of 2mM dNTPs, 2.0 $\mu$L of 25mM MgSO$_4$, 2.0 $\mu$L of PCR primer F (10 $\mu$M), 2.0 $\mu$L of PCR primer R (10 $\mu$m), 1.0 $\mu$L of KOD-Plus (1 U/$\mu$L), 4.0 $\mu$L of Human genomic DNA (7.5 ng/$\mu$L), and 4.0 $\mu$L of mutant EGFR gene (including Zymolyase 0.4 U). As the PCR primer, 24 types of indexes were applied to each exon, and a primer with the indexes was used. Specifically, a primer including the following base sequence was used. Note that the index sequence (INDEX) was a sequence including any selected five bases.

[Table 4]

| Exon | | Base sequence (5' → 3') | Sequence number |
|---|---|---|---|
| 19 | Forward primer | CCATCTCATCCCTGCGTGTCTCCGACTCAG[INDEX]CCAG AAGGTGAGAAAGTTAAAA | 2 |
| | Reverse primer | CCTCTCTATGGGCAGTCGGTGATGATagcaaagcagaaactcacAT | 3 |
| 20 | Forward primer | CCATCTCATCCCTGCGTGTCTCCGACTCAG[INDEX]CATCT GCCTCACCTCCA | 4 |
| | Reverse primer | CCTCTCTATGGGCAGTCGGTGATGTCTTTGTGTTCCCGGA CAT | 5 |
| 21 | Forward primer | CCATCTCATCCCTGCGTGTCTCCGACTCAG[INDEX]GCAG CATGTCAAGATCACAG | 6 |
| | Reverse primer | CCTCTCTATGGGCAGTCGGTGATaaagccacctccttacTTTG | 7 |

The amplification reaction of DNA by the PCR method was performed using T100TM Thermal Cycler (manufactured by Bio-rad). As specific reaction conditions, incubation was performed at 94°C for 2 minutes first. Thereafter, 40 cycles including three steps, namely 94°C for 15 seconds, 50°C for 30 seconds, and 68°C for 15 seconds were performed. Finally, the product was cooled to 10°C, and the reaction was ended.

3. Purification of PCR product by QIAquick

The PCR product was purified using QIAquick (manufactured by QIAGEN). The operation was performed in accordance with a protocol. At this time, the product was transferred to a PCR reaction container for the following emulsion PCR using the ION chef system.

4. Concentration measurement

Qubit3.0 (manufactured by Thermo Fisher Scientific Co., Ltd.) was used for measuring the concentration of the PCR product. Based on the measured concentration, all the samples were dispensed into a 1.5 mL tube so as to have the same concentration and diluted to 12 pM using nuclease-free Water (NFW).

5. Emulsion PCR and chip loading using the ION chef system

Emulsion PCR reaction and chip loading were performed in accordance with the protocol of the ION chef system.

6. Sequence by ION PGM

Sequence analysis was performed in accordance with the ION PGM protocol. Hereinafter, three runs (each n = 24, 5 regions) of samples with ultra-low allele frequencies (hereinafter, also collectively referred to as "IJ group" as a whole) were performed, and one run of a manually diluted sample (each n = 24, 5 regions) was performed.

7. Data acquisition and analysis

In accordance with the method of the EGFR liquid (Reference literature 2: "Kukita Y, et. al.," Quantitative Identification of Mutant Alleles Derived from Lung Cancer in Plasma Cell-Free DNA via Anomaly Detection Using Deep Sequencing Data", PLoS ONE, Vol. 8, Issue 11, e81468, 2013.), data acquisition and analysis were performed.

8. Consideration

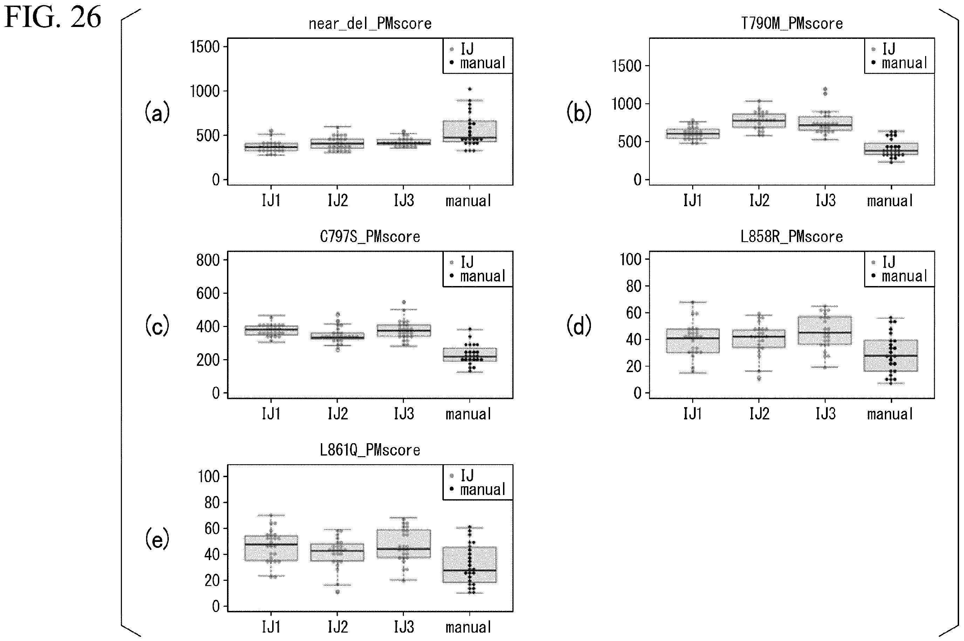

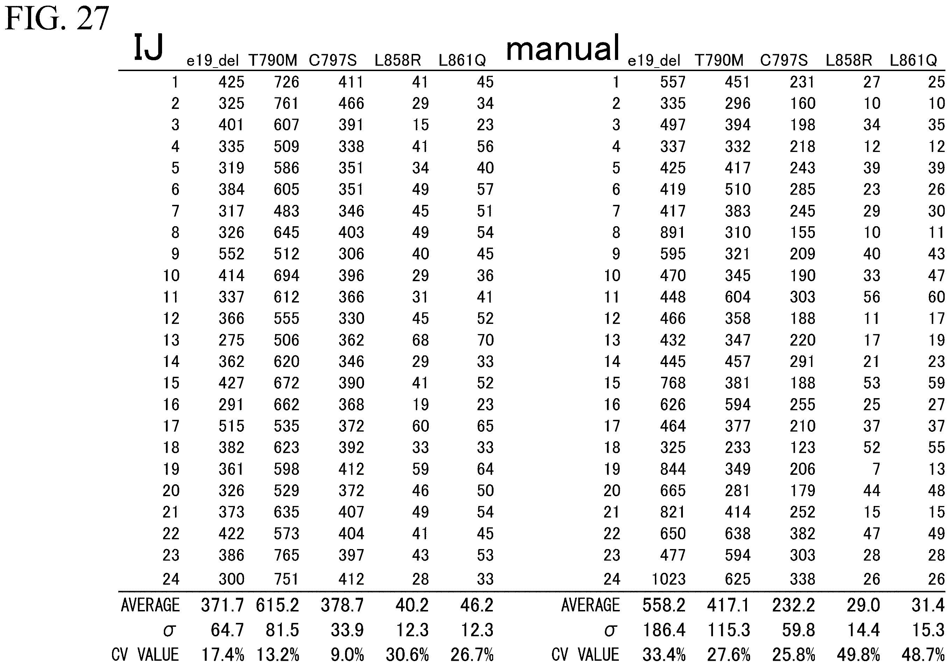

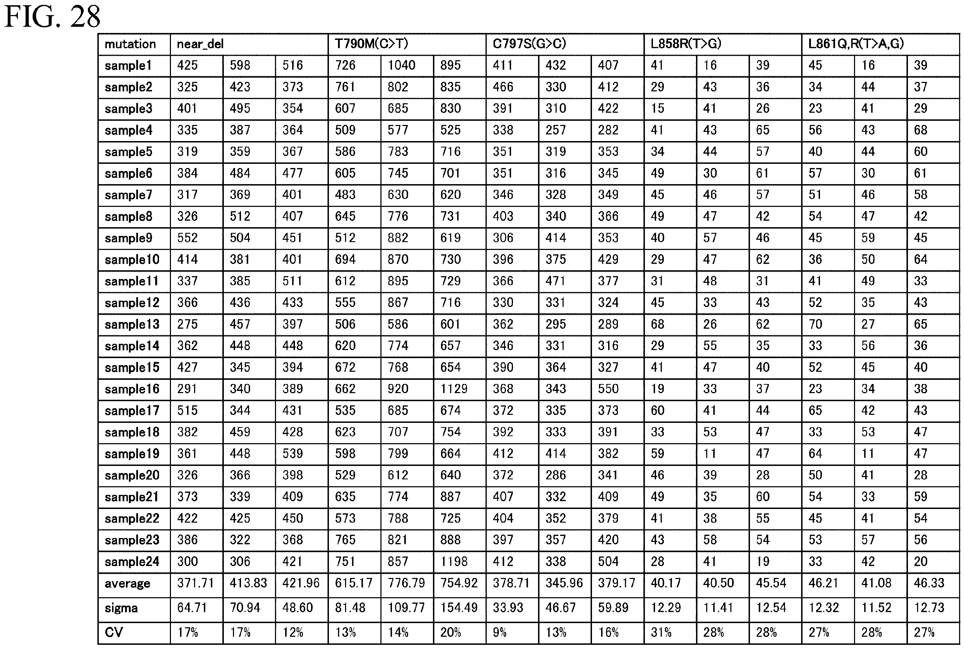

Fig. 26 is a diagram showing PM scores calculated based on the read count obtained from three runs of the IJ group and one run of manually diluted samples (also referred to as a control group or "manual group"). Note that in Fig. 26, IJ 1 to IJ3 mean analysis results of the first run to the third run. It was clarified that variations in all the samples in the IJ group were smaller. Also, Figs. 27 and 28 are tables showing the aforementioned PM scores, and it was also clarified that variations in the IJ group were smaller under all conditions in comparison of CV values as well. Note that the "CV values" are percentages obtained by dividing the standard deviation by an average value.

These results indicated that it was possible to accurately produce samples with low allele frequencies, which were not able to be produced through manual dilution, by the ink jet method and indicated appropriateness and reproducibility as a standard sample in liquid biopsy.

[Example 3]

(Examination of linearity of mutation analysis of EGFR gene)

1. Preparation of samples with ultra-low allele frequencies

The sample filling well was filled with one copy of yeast containing the artificial nucleic acid (mutant EGFR gene) designed in Example 1 using a similar procedure to that in Example 1 so as to have a prescribed number of copies. As the prescribed number of copies, five levels, namely 0, 7, 53, 64, and 100 copies were prepared.

Next, each sample filling well filled with yeast was filled with 4.0 $\mu$L (7.5 ng/$\mu$L) of Human genomic DNA (manufactured by Promega). This filling amount corresponds to the amount by which about ten thousand copies of normal EGFR genes are contained. After the filling, mixing was performed to prepare samples with ultra-low allele frequencies of 0.07%, 0.53%, 0.64%, and 1.00% in the sample filling wells.

2. Nucleic acid amplification by PCR method

Nucleic acid amplification was performed using a method similar to that described in Example 2.

3. Purification of PCR product by QIAquick

The PCR product was purified using a method similar to that described in Example 2.

4. Concentration measurement

The concentration was measured using a method similar to that described in Example 2.

5. Emulsion PCR and chip loading using the ION chef system

Emulsion PCR and chip loading using the ION chef system were performed using a method similar to that described in Example 2.

6. Sequence by ION PGM

Sequence analysis was performed in accordance with the ION PGM protocol. The sequence was performed for one run (each level n = 4, 5 levels (0, 7, 53, 64, 100), and two regions (T790M, L858R)) on samples with ultra-low allele frequencies (hereinafter, collectively referred to as "IJ group" in some cases).

7. Data acquisition and analysis

Data acquisition and analysis were performed using a method similar to that described in Example 2.

8. Consideration

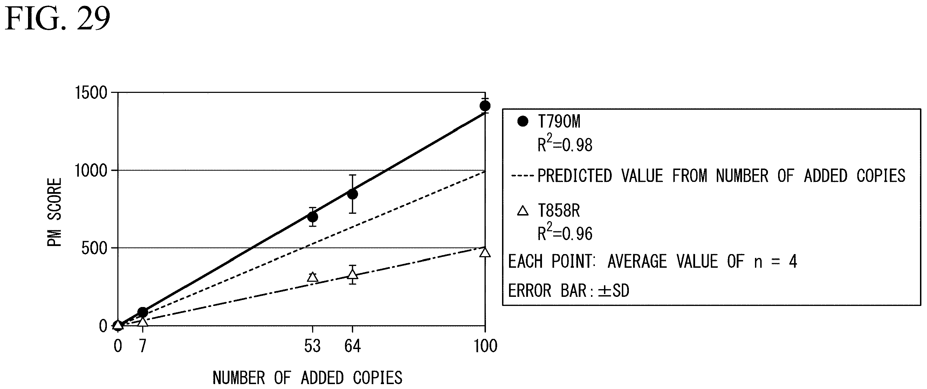

Fig. 29 is a diagram showing PM scores calculated based on the read count obtained from the one run. The vertical axis represents PM scores, and the horizontal axis represents the number of copies introduced into each well. It was clarified that both T790M and L858R showed high linearity. The black dashed line represents PM scores expected from the introduced number of copies, and the reason that the two mutations do not conform to this line is because amplification efficiency or detection efficiency differs between mutant and wild sequences. In this example, the mutant-type T790M has higher amplification efficiency or detection efficiency than the wild type and has higher PM scores as compared with the number of actually introduced copies. On the contrary, L858R has a lower amplification efficiency or detection efficiency and has lower scores as a whole as compared with the expected PM scores.

These results mean that the output PM scores differ depending on mutation. This is an example in which it was possible to clarify properties of the testing based on the sample with a low allele frequency, which cannot be produced by manual dilution, being able to be accurately produced by an ink jet method.

[Example 4]

(Mutation analysis 2 of EGFR gene)

Operations similar to those in Example 2 were performed using three different operators and two different types of equipment, and the differences were visualized. The specific procedure was as follows.

1. Preparation of samples with ultra-low allele frequencies

The sample filling well was filled with one copy of yeast containing the artificial nucleic acid (mutant EGFR gene) designed in Example 1 using a similar procedure to that in Example 1 so as to have a prescribed number of copies. As the prescribed number of copies, wells in four levels, namely 10 copies, 30 copies, 50 copies, and 100 copies were prepared. Next, each sample filling well filled with yeast was filled with 4.0 $\mu$L (7.5 ng/$\mu$L) of Human genomic DNA (manufactured by Promega). This filling amount corresponds to the amount by which about ten thousand copies of normal EGFR genes are contained. After the filling, mixing was performed to prepare samples with ultra-low allele frequencies of 0.1%, 0.3%, 0.5%, and 1.0% in the sample filling wells.

2. Distribution of samples

The required amount of the sample with an ultra-low allele frequency adjusted in "1." was distributed to each of the operators A and B. At this time, the necessary amount for the operator A was 72 wells, and the necessary amount for the operator B was 36 wells in each copy number level.

3. Nucleic acid amplification by PCR method

Each sample with an ultra-low allele concentration distributed in "2." was subjected to an amplification reaction by the PCR method inside a sample filling well. The composition of the reaction solution was a total of 50 $\mu$L including 25.0 $\mu$L of nuclease-free water, 5.0 $\mu$L of 10 $\times$ KOD-Plus-Buffer, 5.0 $\mu$L of 2mM dNTPs, 2.0 $\mu$L of 25 mM MgSO4, 2.0 $\mu$L of PCR primer F (10 $\mu$M), 2.0 $\mu$L of PCR primer R (10 $\mu$M), 1.0 $\mu$l of KOD-Plus (1U/$\mu$L), 4.0 $\mu$L of Human genomic DNA (7.5 ng/$\mu$L), and 4.0 $\mu$L of mutant EGFR gene (including Zymolyase 0.4U). As the PCR primer, 24 types of indexes were applied to each exon, and a primer with the indexes was used. Specifically, a primer including the base sequence shown in Table 4 above was used. Note that the index sequence (INDEX) was a sequence including any selected five bases.

The amplification reaction of DNA by the PCR method was performed using T100TM Thermal Cycler (manufactured by Bio-rad). As specific reaction conditions, incubation was performed at 94°C for 2 minutes first. Thereafter, 40 cycles including three steps, namely 94°C for 15 seconds, 50°C for 30 seconds, and 68°C for 15 seconds were performed. Finally, the product was cooled to 10°C, and the reaction was ended.

4. Purification of PCR product by QIAquick

The PCR product was purified using QIAquick (manufactured by QIAGEN). The operation was performed in accordance with a protocol. At this time, the product was transferred to a PCR reaction container for the following emulsion PCR using the ION chef system.

5. Concentration measurement

Qubit3.0 (manufactured by Thermo Fisher Scientific Co., Ltd.) was used for measuring the concentration of the PCR product. Based on the measured concentration, all the samples were dispensed into 1.5 mL tubes so as to have the same concentration, and were then diluted to 15 nM using nuclease-free Water (NFW). Hereinafter, this may be referred to as a library.

6. Sending of library

The operator A sends the library performed before "5." by the amount corresponding to two runs to the operator C. The operator C measures the concentration in "5." again for the received library.

7. Emulsion PCR and chip loading using ION chef system

Operators A and B performed the emulsion PCR reaction and the chip loading in accordance with a protocol using the ION chef system of the equipment 1.
The operator C performed the emulsion PCR reaction and the chip loading in accordance with a protocol using the ION chef system of the equipment 2.

8. Sequence using ION PGM

The operators A and B performed sequence analysis in accordance with a protocol using ION PGM of the equipment 1. The operator A performed four runs while the operator B performed two runs.
The operator C performed sequence analysis in accordance with a protocol using ION PGM of the equipment 2. The operator C performed two runs. Thereafter, the operator C performed a run again for the library used in the first run and performed a total of three runs.

9. Data acquisition and analysis

Data acquisition and analysis were performed in accordance with a method for the EGFR liquid (see the reference literature 2 listed above).

10. Consideration

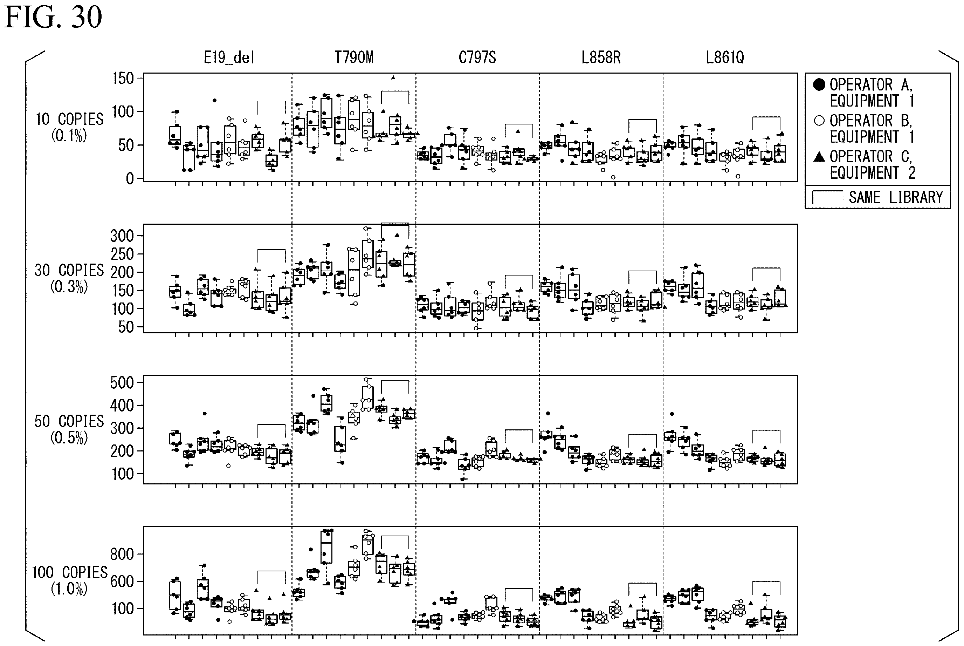

Fig. 30 is a diagram showing PM scores calculated based on the read count obtained from four runs of an operator A/equipment 1, two runs of an operator B/equipment 1, and three runs of an operator C/equipment 2. Note that since the same library was used for the first run and the third run of the operator C, this is shown by connection with a solid line in the drawing. An average value and CV in each level are shown in Tables 5 to 9 below. Asterisks are added to the levels in which CV exceeds 30%.

[Table 5]

| | | E19_del | | | | | | | | |
|---|---|---|---|---|---|---|---|---|---|---|
| | | A | A | A | A | B | B | C | C | C |
| 10 copies 0.1% | Average | 64.5 | 34.7 | 48.3 | 47 | 55 | 48.8 | 57.2 | 25.5 | 54 |
| | CV | 31.7% * | 53.0% * | 49.1% * | 78.5% * | 46.5% * | 42.0% * | 22.90% | 43.2% * | 31.8% * |
| 30 copies 0.3% | Average | 147.3 | 101.2 | 158.5 | 139.3 | 152.8 | 159.2 | 136.7 | 125.8 | 131.3 |
| | CV | 20.20% | 23.50% | 15.70% | 20.30% | 9.30% | 14.80% | 27.90% | 29.20% | 32.6% * |
| 50 copies 0.5% | Average | 244.7 | 187.3 | 251.7 | 228 | 210.7 | 207.5 | 197.2 | 177.5 | 184 |
| | CV | 13.00% | 16.00% | 24.20% | 14.10% | 20.90% | 10.70% | 11.10% | 21.30% | 18.30% |
| 100 copies 1.0% | Average | 488.7 | 377.8 | 559.3 | 428.2 | 386.2 | 415.3 | 378 | 332 | 361.5 |
| | CV | 21.00% | 13.10% | 16.60% | 14.00% | 12.70% | 12.30% | 21.60% | 21.40% | 18.80% |

[Table 6]

| | | T790M | | | | | | | | |
|---|---|---|---|---|---|---|---|---|---|---|
| | | A | A | A | A | B | B | C | C | C |
| 10 copies 0.1% | Average | 77.7 | 76.8 | 93.7 | 73 | 87.7 | 79.3 | 67.8 | 86.5 | 64.3 |
| | CV | 26.30% | 40.6% * | 25.80% | 46.2% * | 33.9% | 36.0% * | 24.70% | 40.7% * | 10.90% |
| 30 copies 0.3% | Average | 190.8 | 205.2 | 208 | 173.2 | 198.2 | 248.3 | 223.5 | 236.7 | 221 |
| | CV | 12.70% | 9.70% | 20.90% | 12.90% | 32.4% * | 19.20% | 21.00% | 12.60% | 17.80% |
| 50 copies 0.5% | Average | 325.3 | 332 | 412.5 | 244.7 | 342.5 | 438.7 | 383.5 | 339.2 | 364.5 |
| | CV | 10.40% | 18.10% | 10.80% | 29.70% | 15.10% | 11.90% | 8.10% | 8.30% | 4.90% |
| 100 copies 1.0% | Average | 518.7 | 678.5 | 830.8 | 584.3 | 706.8 | 875.2 | 718.3 | 668.5 | 676.5 |
| | CV | 10.60% | 11.60% | 19.30% | 8.80% | 11.90% | 9.30% | 11.40% | 12.40% | 10.10% |

[Table 7]

| | | C797S | | | | | | | | |
|---|---|---|---|---|---|---|---|---|---|---|
| | | A | A | A | A | B | B | C | C | C |
| 10 copies 0.1% | Average | 34.3 | 31 | 53.3 | 41.2 | 40.2 | 34.2 | 31.7 | 40.3 | 28 |
| | CV | 18.30% | 40.2% * | 27.90% | 49.2% * | 33.7% * | 44.9% * | 30.9% * | 40.1% * | 10.60% |
| 30 copies 0.3% | Average | 110 | 104.3 | 108.8 | 102.3 | 94.7 | 121.8 | 104.5 | 111 | 95.7 |
| | CV | 20.60% | 25.10% | 32.9% * | 16.40% | 36.3% * | 22.40% | 27.70% | 18.90% | 21.20% |
| 50 copies 0.5% | Average | 174.5 | 157.5 | 216.8 | 137 | 155.8 | 209.8 | 179.2 | 165.7 | 162 |
| | CV | 13.30% | 18.50% | 13.30% | 27.70% | 13.30% | 16.20% | 8.30% | 7.70% | 5.50% |
| 100 copies 1.0% | Average | 296 | 318.7 | 438.3 | 329 | 339.7 | 413.8 | 337.3 | 320.2 | 297.8 |
| | CV | 9.90% | 19.20% | 15.50% | 9.20% | 6.50% | 13.10% | 14.20% | 13.10% | 11.20% |

[Table 8]

| | | L858R | | | | | | | | |
|---|---|---|---|---|---|---|---|---|---|---|
| | | A | A | A | A | B | B | C | C | C |
| 10 copies 0.1% | Average | 46.7 | 53.3 | 45.7 | 41.2 | 28.7 | 31 | 40 | 32.2 | 39.2 |
| | CV | 13.00% | 35.2% * | 45.5% * | 43.6% * | 31.9% | 55.3% * | 27.20% | 43.1% * | 36.3% * |
| 30 copies 0.3% | Average | 160.7 | 155.3 | 155.2 | 104.8 | 113.3 | 113.2 | 118 | 104.8 | 121.3 |
| | CV | 9.80% | 21.80% | 27.30% | 23.10% | 16.10% | 23.90% | 14.40% | 21.30% | 17.80% |
| 50 copies 0.5% | Average | 272 | 249.2 | 200 | 162.7 | 151.2 | 185.5 | 165 | 158 | 158.5 |
| | CV | 19.80% | 14.10% | 19.90% | 15.70% | 14.70% | 13.80% | 9.10% | 16.70% | 17.30% |
| 100 copies 1.0% | Average | 467.7 | 489 | 485.7 | 331.5 | 319.8 | 385.3 | 303.3 | 347.7 | 296 |
| | CV | 5.40% | 9.80% | 12.60% | 15.20% | 8.60% | 10.50% | 19.30% | 20.60% | 17.60% |

[Table 9]

L861Q

| | | A | A | A | A | B | B | C | C | C |
|---|---|---|---|---|---|---|---|---|---|---|
| 10 copies 0.1% | Average | 46.8 | 52 | 46.8 | 41.5 | 29.2 | 31.2 | 40.7 | 33.5 | 39.8 |
| | CV | 15.10% | 36.3% * | 43.3% * | 44.9% * | 32.4% | 55.4% * | 26.50% | 43.4% * | 39.5%* |
| 30 copies 0.3% | Average | 160.7 | 154.2 | 162.3 | 107.7 | 117.2 | 116.7 | 120.3 | 108.2 | 124.3 |
| | CV | 9.50% | 21.20% | 25.10% | 20.50% | 17.40% | 23.00% | 15.60% | 21.70% | 18.40% |
| 50 copies 0.5% | Average | 270 | 247.2 | 208.5 | 165.5 | 154.7 | 188.7 | 168.7 | 163.2 | 163.2 |
| | CV | 20.40% | 16.20% | 18.70% | 15.40% | 15.70% | 14.70% | 8.60% | 16.30% | 16.90% |
| 100 copies 1.0% | Average | 460.7 | 483.5 | 499.3 | 336.3 | 327.8 | 393.2 | 311.3 | 355.5 | 302.2 |
| | CV | 6.00% | 8.90% | 12.20% | 15.70% | 8.70% | 9.90% | 18.60% | 21.20% | 17.10% |

As shown in Figs. 30 and Tables 5 to 9, it was possible to show differences among operators and among equipment as a result of accurately producing a sample having a low allele frequency that cannot be produced through manual dilution by the ink jet method.

[Example 5]

(Examination 2 of linearity of mutation analysis of EGFR gene)

1. Preparation of samples with ultra-low allele frequencies

The sample filling well was filled with one copy of yeast containing the artificial nucleic acid (mutant EGFR gene) designed in Example 1 using a similar procedure to that in Example 1 so as to have a prescribed number of copies. As the prescribed number of copies, four levels, namely 10, 30, 50, and 100 copies were prepared.

Next, each sample filling well filled with yeast was filled with 4.0 $\mu$L (7.5 ng/$\mu$L) of Human genomic DNA (manufactured by Promega). This filling amount corresponds to the amount by which about ten thousand copies of normal EGFR genes are contained. After the filling, mixing was performed to prepare samples with ultra-low allele frequencies of 0.1%, 0.3%, 0.5%, and 1.0% in the sample filling wells.

2. Nucleic acid amplification by PCR method

Nucleic acid amplification was performed using a method similar to that described in Example 2.

3. Purification of PCR product by QIAquick

The PCR product was purified using a method similar to that described in Example 2.

4. Concentration measurement

The concentration was measured using a method similar to that described in Example 2.

5. Emulsion PCR and chip loading using the ION chef system

Emulsion PCR and chip loading using the ION chef system were performed using a method similar to that described in Example 2.

6. Sequence by ION PGM

Sequence analysis was performed in accordance with the ION PGM protocol. The sequence was performed on samples with ultra-low allele frequencies for 1 run (each level n = 6, 4 levels (10, 30, 50, 100), 5 regions (T790M, L861Q, C797S, E19_del, and L858R)).

7. Data acquisition and analysis

Data acquisition and analysis were performed using a method similar to that described in Example 2.

8. Consideration

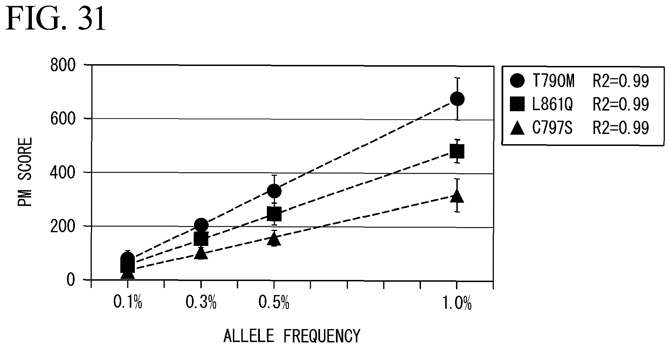

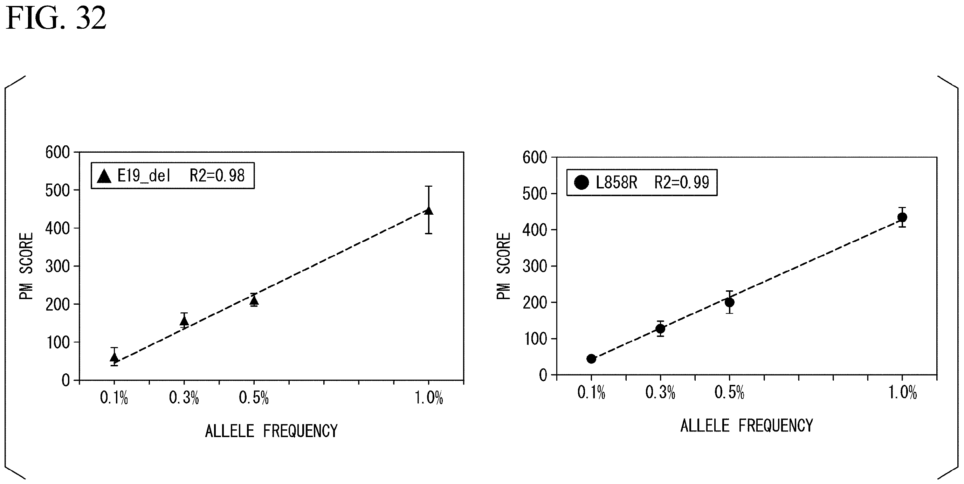

Graphs showing a relationship between PM scores calculated based on the read count obtained from one run of the samples with ultra-low allele frequencies and the allele frequencies are shown in Fig. 31 (T790M, L861Q, C797S) and Fig. 32 (E19_del, L858R). In Figs. 31 and 32, the vertical axis represents the PM scores while the horizontal axis represents the allele frequencies. It was clarified that all of T790M, L861Q, C797S, E19_del, and L858R showed high linearity.

[Example 6]

(Method for determining number of copies of nucleic acid B using measured value of nucleic acid A)

A method for accurately defining the number of copies of nucleic acid B, which has a larger number of copies than that of nucleic acid A, was examined. Nucleic acids A in multiple levels were subjected to qPCR, regression analysis was performed based on the measured values thereof, and a calibration curve was plotted. A numerical value that is the number of copies of the nucleic acid B was substituted into the calibration curve, and this was used as an extrapolation value for the calibration curve of the nucleic acid A. The nucleic acid B was also subjected to qPCR in a similar manner, and this measured value was compared with the extrapolation value of the calibration curve of the nucleic acid A to confirm that the measured value of the nucleic acid B was included within a certain acceptance criterion to thereby define the number of copies of the nucleic acid B. The specific procedure is shown below.

1. Preparation of samples with ultra-low allele frequencies

The sample-filled well to be the nucleic acid A was filled with one copy of yeast containing the artificial nucleic acid (mutant EGFR gene) designed in Example 1 using a similar procedure to that in Example 1 to obtain a prescribed number of copies. As the prescribed number of copies, four levels, namely 10, 30, 50, and 100 copies were prepared. For each level, 12 wells were prepared.

Next, the sample-filled well to be the nucleic acid B was filled with 4.0 μL (7.5 ng/μL) of Human genomic DNA (manufactured by Promega). This filling amount corresponds to the amount by which about ten thousand copies of normal EGFR genes are contained. This was prepared for 48 wells.

2. qPCR

The samples with ultra-low allele frequencies prepared in "1." were subjected to an amplification reaction by the qPCR method in each well. The composition of the reaction solution was a total of 20 μL including 3.6 μL of nuclease-free water, 10.0 μL of TaqMan (registered trademark) Universal PCR Master Mix, 1.0 μL of qPCR primer F (10 μM), 1.0 μL of qPCR primer R (10 μM), 0.4 μL of TaqMan (registered trademark) Probe, and 4.0 μL of Human genomic DNA (7.5 ng/μL) or a mutant EGFR gene

(containing Zymolyase 0.4U). As the qPCR primer, TaqMan (registered trademark) Probe, a primer including the following base sequence was used.

[Table 10]

| Name | Base sequence (5' → 3') | Sequence number |
|---|---|---|
| qPCR primer F | AGGTGACCCTTGTCTCTGTG | 8 |
| qPCR primer R | CCTCAAGAGAGCTTGGTTGG | 9 |
| TaqMan Probe | AGCTTGTGGAGCCTCTTACACCCAGT | 10 |

qPCR was performed using QuantStudio™ 12K Flex Real-Time PCR System (manufactured by Thermo Fisher Scientific Co., Ltd.). As specific reaction conditions, incubation at 50°C for two minutes was performed. Thereafter, incubation at 95°C for 10 minutes was performed. Then, 50 cycles including two steps, namely 95°C for 30 seconds and 61°C for 1 minute were performed. Finally, the product was cooled to 10°C, and the reaction was ended.

3. Regression analysis and extrapolation value calculation

A regression line was obtained from the measurement results (Cq value: quantification cycle) of qPCR of 10 to 100 copies in the reaction wells containing nucleic acid A. The common logarithm of the number of copies (base was 10) was taken, and the regression line was obtained by a linear function. The common logarithm 4 of 10000 copies, which is the number of copies of the nucleic acid B, was substituted into the equation of this regression line and was used as an extrapolation value.

Next, regression lines were obtained for 5 levels including the Cq values of 10000 copies of the nucleic acid B in addition to 10 to 100 copies in the reaction wells containing the nucleic acid. Similarly to the regression line of only the nucleic acid A, the common logarithm of the number of copies was taken, and the regression line was obtained by a linear function.

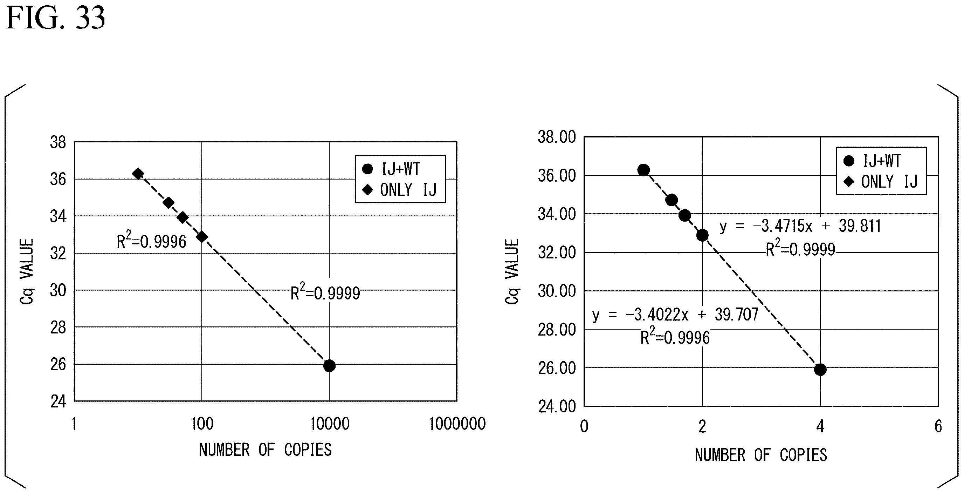

The left diagram in Fig. 33 shows the number of copies by the horizontal axis, and the right diagram in Fig. 33 shows the common logarithm of the number of copies by the horizontal axis.

4. Consideration (comparison between extrapolation value and average value of nucleic acid B)

The extrapolation value was 26.10, and the average value of Cq values of the nucleic acid B was 25.91. This difference was 0.19, and the difference when converted into the number of copies was $2^{0.19}$, which was about 14%.

From the above results, it was possible to define the number of copies of the nucleic acid B by using the measured value obtained by using the nucleic acid A whose number of copies was accurately defined by the ink jet method.

The present invention includes the following aspects.

(1) A sample for evaluating performance of a genetic testing apparatus, the sample including:

a nucleic acid A; and a nucleic acid B,
in which the nucleic acid A and the nucleic acid B have mutually different sequences,
the nucleic acid A with a specific number of molecules is contained,
more nucleic acid B than the number of molecules of the nucleic acid A is contained, and
a ratio A/B of the number of molecules of the nucleic acid A with respect to the number of molecules of the nucleic acid B is specified.

(2) The sample according to (1), in which the number of molecules of the nucleic acid A is 1 or more and 200 or less.
(3) The sample according to (1) or (2), in which a total number of molecules of the nucleic acid A and the nucleic acid B is 30000 or less.
(4) The sample according to any one of (1) to (3), in which the ratio A/B of the number of molecules of the nucleic acid A with respect to the number of molecules of the nucleic acid B is 10% or less.
(5) The sample according to any one of (1) to (4), in which the ratio A/B of the number of molecules of the nucleic acid A with respect to the number of molecules of the nucleic acid B is 1% or less.
(6) The sample according to any one of (1) to (5), in which the nucleic acid A and the nucleic acid B are DNAs of a vertebrate.
(7) The sample according to any one of (1) to (6), in which the nucleic acid A and the nucleic acid B are human genomic DNAs.
(8) The sample according to any one of (1) to (7), in which the ratio A/B of the number of molecules of the nucleic acid A with respect to the number of molecules of the nucleic acid B corresponds to an occurrence frequency of a specific genetic disease.
(9) The sample according to (8), in which the nucleic acid A is an EGFR gene with mutation, and the nucleic acid B is an EGFR gene with no mutation.
(10) The sample according to (9), in which the nucleic acid A includes a base sequence containing a sequence in which exons 18, 19, 20, and 21 of the EGFR gene are tandemly linked.
(11) The sample according to any one of (1) to (10), in which the genetic testing apparatus is a next generation sequencer.
(12) A method for preparing a sample of any one of (1) to (11), the method including the steps of:

an incorporating step of incorporating the nucleic acid A in a nucleic acid in a nucleus of a cell;
a nucleic acid A filling step of producing liquid droplets including one cell in which the nucleic acid A is incorporated in the nucleic acid of the nucleus in a container and performing filling with a specific number of cells by controlling the number of liquid droplets; and
a nucleic acid B filling step of filling the container with the nucleic acid B such that a ratio A/B of the number of molecules of the nucleic acid A with respect to the number of molecules of the nucleic acid B is a specific ratio.

(13) The method according to (12), in which the cell is a yeast or mammalian cell.
(14) The method according to (12) or (13), in which in the nucleic acid B filling step, the number of molecules of the nucleic acid B is measured by an absorption spectrometry or a real-time PCR method.
(15) The method according to (14), in which in the nucleic acid B filling step, the nucleic acid B and a plurality of nucleic acids A of mutually different numbers of molecules are measured by the real-time PCR method, and the number of the molecules of the nucleic acid B is determined using a calibration curve created by the plurality of nucleic acids A of mutually different numbers of molecules.
(16) A device for evaluating performance of a genetic testing apparatus, including: a plurality of reaction spaces, in which a sample of any one of (1) to (11) is contained in at least some of the reaction spaces.
(17) A method for evaluating performance of a genetic testing apparatus, including using the device for evaluating performance of a genetic testing apparatus of (16): a PM value information acquisition process of acquiring PM value information in the device for evaluating performance; and

a performance evaluation process of evaluating a performance of the genetic testing apparatus based on the PM value information.

(18) A program for evaluating performance of a genetic testing apparatus causing a computer to execute:

a PM value information acquisition step of acquiring PM value information in the device for evaluating performance using the device for evaluating performance of a genetic testing apparatus of (16); and
a performance evaluation step of evaluating performance of the genetic testing apparatus based on the PM value information.

(19) An apparatus for evaluating performance of a genetic testing apparatus, including: a PM value information acquisition unit configured to, by using the device for evaluating performance of a genetic testing apparatus of (16), acquire PM value information in the device for evaluating performance; and
a performance evaluation unit configured to evaluate performance of the genetic testing apparatus based on the PM value information.

Reference Signs List

1: Device
2: Nucleic acid A
3: Nucleic acid B
4: Sample for evaluating performance
5: Priority area of sample for evaluating performance
6: Base material
7: Reaction space (well)
10, 10', 10C: Ejection head (liquid droplet ejection means)
11,11a, 11b, 11c, 11C, 11': Liquid chamber
12, 12C: Membrane
13, 13C: Drive element
13a: Motor
13b, 13c: Piezoelectric element
20: Drive means
30, 260: Light source
40: Mirror
60, 61: Light-receiving element
70: Control means
71, 101: CPU
72: ROM
73: RAM
74, 106: I/F
75: Bus line
100: Performance evaluation apparatus
102: Main storage device
103: Auxiliary storage device
104: Output device
105: Input device
107: Bus
111, 111a, 111b, 111c, 121: Nozzle
112: Solenoid valve
115: Atmosphere opening unit
200: Coil
250: Microchannel
255: Detector
255': Image acquisition unit
265, 265': Lens
300, 300a, 300b, 300c: Cell suspension
310, 310': Liquid droplet

350, 350a, 350b, 350', 350": Cell
400: Dispensing device
401, 401A, 401B, 401C: Liquid droplet-forming device
700, 700': Plate
710: Well
800: Stage
900: Control device
L: Light
Lf, $Lf_1$, $Lf_2$: Fluorescence

PRIOR ART LITERATURE

Patent Document

Patent Document 1: Japanese Unexamined Patent Application, First Publication No. 2019-80501

**Claims**

1. A sample for evaluating performance of a genetic testing apparatus, comprising:

   a nucleic acid A; and
   a nucleic acid B,
   wherein the nucleic acid A and the nucleic acid B have mutually different sequences,
   the nucleic acid A with a specific number of molecules is contained,
   more nucleic acid B than the number of molecules of the nucleic acid A is contained,
   and a ratio A/B of the number of molecules of the nucleic acid A with respect to the number of molecules of the nucleic acid B is specified.

2. The sample according to Claim 1, wherein the number of molecules of the nucleic acid A is 1 or more and 200 or less.

3. The sample according to Claim 1 or 2, wherein a total number of molecules of the nucleic acid A and the nucleic acid B is 30000 or less.

4. The sample according to any one of Claims 1 to 3, wherein the ratio A/B of the number of molecules of the nucleic acid A with respect to the number of molecules of the nucleic acid B is 10% or less.

5. The sample according to any one of Claims 1 to 4, wherein the ratio A/B of the number of molecules of the nucleic acid A with respect to the number of molecules of the nucleic acid B is 1% or less.

6. The sample according to any one of Claims 1 to 5, wherein the nucleic acid A and the nucleic acid B are DNAs of a vertebrate.

7. The sample according to any one of Claims 1 to 6, wherein the nucleic acid A and the nucleic acid B are human genomic DNAs.

8. The sample according to any one of Claims 1 to 7, wherein the ratio A/B of the number of molecules of the nucleic acid A with respect to the number of molecules of the nucleic acid B corresponds to an occurrence frequency of a specific genetic disease.

9. The sample according to Claim 8, wherein the nucleic acid A is an EGFR gene with mutation, and the nucleic acid B is an EGFR gene with no mutation.

10. The sample according to Claim 9, wherein the nucleic acid A includes a base sequence containing a sequence in which exons 18, 19, 20, and 21 of the EGFR gene are tandemly linked.

11. The sample according to any one of Claims 1 to 10, wherein the genetic testing apparatus is a next generation sequencer.

**12.** A method for preparing a sample of any one of Claims 1 to 11, the method comprising:

an incorporating step of incorporating the nucleic acid A in a nucleic acid in the nucleus of cell;

a nucleic acid A filling step of producing a liquid droplet containing one cell in which the nucleic acid A is incorporated in the nucleic acid in the nucleus in a container and performing filling with a specific number of cells through control of the number of the liquid droplets; and

a nucleic acid B filling step of performing filling with the nucleic acid B in the container such that the ratio A/B of the number of molecules of the nucleic acid A with respect to the number of molecules of the nucleic acid B is a specific ratio.

**13.** The method according to Claim 12, wherein the cell is a yeast or mammalian cell.

**14.** The method according to Claim 12 or 13, wherein in the nucleic acid B filling step, the number of molecules of the nucleic acid B is measured by an absorption spectrometry or a real-time PCR method.

**15.** The method according to Claim 14, wherein in the nucleic acid B filling step, the nucleic acid B and a plurality of nucleic acids A of mutually different numbers of molecules are measured by the real-time PCR method, and the number of the molecules of the nucleic acid B is determined using a calibration curve created by the plurality of nucleic acids A of mutually different numbers of molecules.

**16.** A device for evaluating performance of a genetic testing apparatus, comprising:

a plurality of reaction spaces,

wherein a sample of any one of Claims 1 to 11 is contained in at least some of the reaction spaces.

**17.** A method for evaluating performance of a genetic testing apparatus, comprising, using the device for evaluating performance of a genetic testing apparatus of Claim 16:

a PM value information acquisition step of acquiring PM value information in the device for evaluating performance; and

a performance evaluation process of evaluating a performance of the genetic testing apparatus based on the PM value information.

**18.** A program for evaluating performance of a genetic testing apparatus, the program causing a computer to execute:

a PM value information acquisition step of acquiring PM value information in the device for evaluating performance using the device for evaluating performance of a genetic testing apparatus of Claim 16; and

a performance evaluation step of evaluating performance of the genetic testing apparatus based on the PM value information.

**19.** An apparatus for evaluating performance of a genetic testing apparatus, comprising:

a PM value information acquisition unit configured to, by using the device for evaluating performance of a genetic testing apparatus of Claim 16, acquire PM value information in the device for evaluating performance; and

a performance evaluation unit configured to evaluate performance of the genetic testing apparatus based on the PM value information.

FIG. 1

FIG. 2

## FIG. 3

## FIG. 4

FIG. 5

11c

300c

12C

13c

111c

FIG. 6

(a)

(b)

# FIG. 7

(a)

11c

12c

13c

(b)

11c

12c

13c

(c)

11c

12c

13c

310'

FIG. 8

# FIG. 9

# FIG. 10

## FIG. 11

```
                                        70
┌──────────────────────────────────────────┐
│ CONTROL MEANS                              │
│                                    701     │
│   ┌────────────────────────────────┐       │
│   │      EJECTION CONTROL          │       │
│   │          MEANS                 │       │
│   └────────────────────────────────┘       │
│                                    702     │
│   ┌────────────────────────────────┐       │
│   │    LIGHT SOURCE CONTROL        │       │
│   │          MEANS                 │       │
│   └────────────────────────────────┘       │
│                                    703     │
│   ┌────────────────────────────────┐       │
│   │    CELL NUMBER-COUNTING        │       │
│   │          MEANS                 │       │
│   └────────────────────────────────┘       │
│                                            │
└──────────────────────────────────────────┘
```

## FIG. 12

```
              ┌─────────────┐
              │    START    │
              └─────────────┘
                     │
                     ▼                  S11
      ┌──────────────────────────────┐
      │     EJECT LIQUID DROPLET      │
      └──────────────────────────────┘
                     │
                     ▼                  S12
      ┌──────────────────────────────┐
      │     TURN ON LIGHT SOURCE      │
      └──────────────────────────────┘
                     │
                     ▼                  S13
      ┌──────────────────────────────┐
      │    COUNT NUMBER OF CELLS      │
      └──────────────────────────────┘
                     │
                     ▼
              ┌─────────────┐
              │     END     │
              └─────────────┘
```

FIG. 13

# FIG. 14

FIG. 15

(a)

$350_1$       $350_2$

(b)

$350_1$       $350_2$

# FIG. 16

FIG. 17

FIG. 18

FIG. 19

FIG. 20

FIG. 21

FIG. 22

(a)

(b)

## FIG. 23

## FIG. 24

# FIG. 25

```
                    ┌─────────────┐
                    │    START    │
                    └──────┬──────┘
                           │              ┌ S110
                           ▼
          ┌────────────────────────────────┐
          │      ACQUIRE  INFORMATION       │
          └────────────────┬───────────────┘
                           │              ┌ S111
                           ▼
          ┌────────────────────────────────┐
          │       PERFORM  EVALUATION       │
          └────────────────┬───────────────┘
                           │              ┌ S112
                           ▼
          ┌────────────────────────────────┐
          │     SAVE  EVALUATION  RESULT    │
          └────────────────┬───────────────┘
                           │
                           ▼
                    ┌─────────────┐
                    │     END     │
                    └─────────────┘
```

FIG. 26

# FIG. 27

| IJ | e19_del | T790M | C797S | L858R | L861Q | manual | e19_del | T790M | C797S | L858R | L861Q |
|---|---|---|---|---|---|---|---|---|---|---|---|
| 1 | 425 | 726 | 411 | 41 | 45 | 1 | 557 | 451 | 231 | 27 | 25 |
| 2 | 325 | 761 | 466 | 29 | 34 | 2 | 335 | 296 | 160 | 10 | 10 |
| 3 | 401 | 607 | 391 | 15 | 23 | 3 | 497 | 394 | 198 | 34 | 35 |
| 4 | 335 | 509 | 338 | 41 | 56 | 4 | 337 | 332 | 218 | 12 | 12 |
| 5 | 319 | 586 | 351 | 34 | 40 | 5 | 425 | 417 | 243 | 39 | 39 |
| 6 | 384 | 605 | 351 | 49 | 57 | 6 | 419 | 510 | 285 | 23 | 26 |
| 7 | 317 | 483 | 346 | 45 | 51 | 7 | 417 | 383 | 245 | 29 | 30 |
| 8 | 326 | 645 | 403 | 49 | 54 | 8 | 891 | 310 | 155 | 10 | 11 |
| 9 | 552 | 512 | 306 | 40 | 45 | 9 | 595 | 321 | 209 | 40 | 43 |
| 10 | 414 | 694 | 396 | 29 | 36 | 10 | 470 | 345 | 190 | 33 | 47 |
| 11 | 337 | 612 | 366 | 31 | 41 | 11 | 448 | 604 | 303 | 56 | 60 |
| 12 | 366 | 555 | 330 | 45 | 52 | 12 | 466 | 358 | 188 | 11 | 17 |
| 13 | 275 | 506 | 362 | 68 | 70 | 13 | 432 | 347 | 220 | 17 | 19 |
| 14 | 362 | 620 | 346 | 29 | 33 | 14 | 445 | 457 | 291 | 21 | 23 |
| 15 | 427 | 672 | 390 | 41 | 52 | 15 | 768 | 381 | 188 | 53 | 59 |
| 16 | 291 | 662 | 368 | 19 | 23 | 16 | 626 | 594 | 255 | 25 | 27 |
| 17 | 515 | 535 | 372 | 60 | 65 | 17 | 464 | 377 | 210 | 37 | 37 |
| 18 | 382 | 623 | 392 | 33 | 33 | 18 | 325 | 233 | 123 | 52 | 55 |
| 19 | 361 | 598 | 412 | 59 | 64 | 19 | 844 | 349 | 206 | 7 | 13 |
| 20 | 326 | 529 | 372 | 46 | 50 | 20 | 665 | 281 | 179 | 44 | 48 |
| 21 | 373 | 635 | 407 | 49 | 54 | 21 | 821 | 414 | 252 | 15 | 15 |
| 22 | 422 | 573 | 404 | 41 | 45 | 22 | 650 | 638 | 382 | 47 | 49 |
| 23 | 386 | 765 | 397 | 43 | 53 | 23 | 477 | 594 | 303 | 28 | 28 |
| 24 | 300 | 751 | 412 | 28 | 33 | 24 | 1023 | 625 | 338 | 26 | 26 |
| AVERAGE | 371.7 | 615.2 | 378.7 | 40.2 | 46.2 | AVERAGE | 558.2 | 417.1 | 232.2 | 29.0 | 31.4 |
| $\sigma$ | 64.7 | 81.5 | 33.9 | 12.3 | 12.3 | $\sigma$ | 186.4 | 115.3 | 59.8 | 14.4 | 15.3 |
| CV VALUE | 17.4% | 13.2% | 9.0% | 30.6% | 26.7% | CV VALUE | 33.4% | 27.6% | 25.8% | 49.8% | 48.7% |

EP 4 092 132 A1

# FIG. 28

| mutation | near_del | | | T790M(C>T) | | | C797S(G>C) | | | L858R(T>G) | | | L861Q,R(T>A,G) | | |
|---|---|---|---|---|---|---|---|---|---|---|---|---|---|---|---|
| sample1 | 425 | 598 | 516 | 726 | 1040 | 895 | 411 | 432 | 407 | 41 | 16 | 39 | 45 | 16 | 39 |
| sample2 | 325 | 423 | 373 | 761 | 802 | 835 | 466 | 330 | 412 | 29 | 43 | 36 | 34 | 44 | 37 |
| sample3 | 401 | 495 | 354 | 607 | 685 | 830 | 391 | 310 | 422 | 15 | 41 | 26 | 23 | 41 | 29 |
| sample4 | 335 | 387 | 364 | 509 | 577 | 525 | 338 | 257 | 282 | 41 | 43 | 65 | 56 | 43 | 68 |
| sample5 | 319 | 359 | 367 | 586 | 783 | 716 | 351 | 319 | 353 | 34 | 44 | 57 | 40 | 44 | 60 |
| sample6 | 384 | 484 | 477 | 605 | 745 | 701 | 351 | 316 | 345 | 49 | 30 | 61 | 57 | 30 | 61 |
| sample7 | 317 | 369 | 401 | 483 | 630 | 620 | 346 | 328 | 349 | 45 | 46 | 57 | 51 | 46 | 58 |
| sample8 | 326 | 512 | 407 | 645 | 776 | 731 | 403 | 340 | 366 | 49 | 47 | 42 | 54 | 47 | 42 |
| sample9 | 552 | 504 | 451 | 512 | 882 | 619 | 306 | 414 | 353 | 40 | 57 | 46 | 45 | 59 | 45 |
| sample10 | 414 | 381 | 401 | 694 | 870 | 730 | 396 | 375 | 429 | 29 | 47 | 62 | 36 | 50 | 64 |
| sample11 | 337 | 385 | 511 | 612 | 895 | 729 | 366 | 471 | 377 | 31 | 48 | 31 | 41 | 49 | 33 |
| sample12 | 366 | 436 | 433 | 555 | 867 | 716 | 330 | 331 | 324 | 45 | 33 | 43 | 52 | 35 | 43 |
| sample13 | 275 | 457 | 397 | 506 | 586 | 601 | 362 | 295 | 289 | 68 | 26 | 62 | 70 | 27 | 65 |
| sample14 | 362 | 448 | 448 | 620 | 774 | 657 | 346 | 331 | 316 | 29 | 55 | 35 | 33 | 56 | 36 |
| sample15 | 427 | 345 | 394 | 672 | 768 | 654 | 390 | 364 | 327 | 41 | 47 | 40 | 52 | 45 | 40 |
| sample16 | 291 | 340 | 389 | 662 | 920 | 1129 | 368 | 343 | 550 | 19 | 33 | 37 | 23 | 34 | 38 |
| sample17 | 515 | 344 | 431 | 535 | 685 | 674 | 372 | 335 | 373 | 60 | 41 | 44 | 65 | 42 | 43 |
| sample18 | 382 | 459 | 428 | 623 | 707 | 754 | 392 | 333 | 391 | 33 | 53 | 47 | 33 | 53 | 47 |
| sample19 | 361 | 448 | 539 | 598 | 799 | 664 | 412 | 414 | 382 | 59 | 11 | 47 | 64 | 11 | 47 |
| sample20 | 326 | 366 | 398 | 529 | 612 | 640 | 372 | 286 | 341 | 46 | 39 | 28 | 50 | 41 | 28 |
| sample21 | 373 | 339 | 409 | 635 | 774 | 887 | 407 | 332 | 409 | 49 | 35 | 60 | 54 | 33 | 59 |
| sample22 | 422 | 425 | 450 | 573 | 788 | 725 | 404 | 352 | 379 | 41 | 38 | 55 | 45 | 41 | 54 |
| sample23 | 386 | 322 | 368 | 765 | 821 | 888 | 397 | 357 | 420 | 43 | 58 | 54 | 53 | 57 | 56 |
| sample24 | 300 | 306 | 421 | 751 | 857 | 1198 | 412 | 338 | 504 | 28 | 41 | 19 | 33 | 42 | 20 |
| average | 371.71 | 413.83 | 421.96 | 615.17 | 776.79 | 754.92 | 378.71 | 345.96 | 379.17 | 40.17 | 40.50 | 45.54 | 46.21 | 41.08 | 46.33 |
| sigma | 64.71 | 70.94 | 48.60 | 81.48 | 109.77 | 154.49 | 33.93 | 46.67 | 59.89 | 12.29 | 11.41 | 12.54 | 12.32 | 11.52 | 12.73 |
| CV | 17% | 17% | 12% | 13% | 14% | 20% | 9% | 13% | 16% | 31% | 28% | 28% | 27% | 28% | 27% |

# FIG. 29

FIG. 30

EP 4 092 132 A1

FIG. 31

FIG. 32

## FIG. 33

## INTERNATIONAL SEARCH REPORT

| | International application No. |
|---|---|
| | PCT/JP2020/047471 |

A. CLASSIFICATION OF SUBJECT MATTER
C12Q 1/6806(2018.01)i; C12M 1/00(2006.01)i; C12M 1/34(2006.01)i; C12N
15/10(2006.01)i; C12N 15/12(2006.01)i; C12Q 1/6851(2018.01)i; C12Q
1/686(2018.01)i; C12Q 1/6869(2018.01)i
FI:    C12Q1/6806 Z ZNA; C12Q1/6851 Z; C12Q1/686 Z; C12Q1/6869 Z;
       C12N15/12; C12M1/34 B; C12N15/10 Z ZNA; C12M1/34 Z; C12M1/00 A
According to International Patent Classification (IPC) or to both national classification and IPC

B. FIELDS SEARCHED

Minimum documentation searched (classification system followed by classification symbols)
C12Q1/6806; C12M1/00; C12M1/34; C12N15/10; C12N15/12; C12Q1/6851;
C12Q1/686; C12Q1/6869

| Documentation searched other than minimum documentation to the extent that such documents are included in the fields searched | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS
(STN)

C. DOCUMENTS CONSIDERED TO BE RELEVANT

| Category* | Citation of document, with indication, where appropriate, of the relevant passages | Relevant to claim No. |
|---|---|---|
| X | JP 2019-520800 A (REGENTS OF THE UNIVERSITY OF MINNESOTA) 25 July 2019 (2019-07-25) claims 1, 3, 19, 20, paragraphs [0004], [0005], [0014], [0041], [0051]-[0060], fig. 23, etc. | 1-7, 11, 16 |
| Y | claims 1, 3, 19, 20, paragraphs [0004], [0005], [0014], [0041], [0051]-[0060], fig. 23, etc. | 12-15 |
| X | JP 2013-542712 A (F. HOFFMANN-LA ROCHE AG) 28 November 2013 (2013-11-28) claims 3, 4, 7, 8, 9, paragraphs [0019]-[0021], [0046], [0047], etc. | 1-5, 16 |
| Y | claims 3, 4, 7, 8, 9, paragraphs [0019]-[0021], [0046], [0047], etc. | 12-15 |

☒ Further documents are listed in the continuation of Box C.    ☒ See patent family annex.

| | | |
|---|---|---|
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| 15 February 2021 (15.02.2021) | 22 February 2021 (22.02.2021) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
|---|
| PCT/JP2020/047471 |

C (Continuation).   DOCUMENTS CONSIDERED TO BE RELEVANT

| Category* | Citation of document, with indication, where appropriate, of the relevant passages | Relevant to claim No. |
|---|---|---|
| X | JP 2019-080501 A (SYSMEX CORPORATION) 30 May 2019 (2019-05-30) claims 1, 6, 9, 22, paragraphs [0001], [0009], [0017], [0063], [0067], [0081], [0120]-[0126], [0131], [0218]-[0233], fig. 6, 8, etc. | 1-11, 16-19 |
| Y | claims 1, 6, 9, 22, paragraphs [0001], [0009], [0017], [0063], [0067], [0081], [0120]-[0126], [0131], [0218]-[0233], fig. 6, 8, etc. | 12-15 |
| Y | JP 2019-216704 A (RICOH CO., LTD.) 26 December 2019 (2019-12-26) claims 1, 9-11, examples, etc. | 12-15 |

Form PCT/ISA/210 (continuation of second sheet) (January 2015)

## INTERNATIONAL SEARCH REPORT
Information on patent family members

International application No.

PCT/JP2020/047471

| Patent Documents referred in the Report | Publication Date | Patent Family | Publication Date |
|---|---|---|---|
| JP 2019-520800 A | 25 Jul. 2019 | US 2019/0177781 A1 claims 1, 3, 19, 20, fig. 23, paragraphs [0004], [0005], [0052]-[0060] WO 2017/192974 A1 CN 109715827 A | |
| JP 2013-542712 A | 28 Nov. 2013 | US 2012/0196283 A1 US 2015/0017645 A1 US 2017/0292145 A1 WO 2012/013731 A1 claims 3, 4, 7, 8, 9, page 11, lines 3-27, etc. CN 103069007 A CN 107190056 A | |
| JP 2019-080501 A | 30 May 2019 | US 2019/0127807 A1 claims 1, 6, 9, paragraphs [0001], [0008], [0073], [0091], [0130]-[0136], [0141], fig. 6, 8, etc. EP 3476946 A1 CN 109722470 A | |
| JP 2019-216704 A | 26 Dec. 2019 | JP 2019-92494 A JP 6447765 B US 2019/0151843 A1 claims 1, 9-11, examples, etc. WO 2019/093530 A1 EP 3486330 A1 CN 109810890 A | |

Form PCT/ISA/210 (patent family annex) (January 2015)

## REFERENCES CITED IN THE DESCRIPTION

**Patent documents cited in the description**

- JP 2020005315 A
- JP 2020137596 A

- JP 2019080501 A

**Non-patent literature cited in the description**

- **MOON S. et al.** Drop-on-demand single cell isolation and total RNA analysis. *PLoS One,* 2011, vol. 6 (3), e17455

- **KUKITA Y.** Quantitative Identification of Mutant Alleles Derived from Lung Cancer in Plasma Cell-Free DNA via Anomaly Detection Using Deep Sequencing Data. *PLoS ONE,* 2013, vol. 8 (11), e81468